# EUROPEAN PATENT APPLICATION

(11) **EP 4 382 621 A2**
(43) Date of publication of application: **12.06.2024**
(21) Application number: 24164868.2
(22) Date of filing: 26.07.2022
(51) Int. Cl.: C12Q 1/689

(54) **COMPOSITIONS AND METHODS FOR DETECTING C. COLI AND AT LEAST ONE OF SALMONELLA, C. JEJUNI, SHIGELLA, AND STEC**

(30) Priority: 27.07.2021 US 202163226079 P
(62) Divisional of application: 22755052.2
(71) Applicant: Gen-Probe Incorporated, San Diego, CA 92121 (US)
(72) Inventor: PANUGANTI, Sree Divya, San Diego, CA 92121 (US)
(74) Representative: Script IP Limited

(57) **Abstract**

There is described a set of oligonucleotides for determining the presence or absence of at least two enteric pathogens, said set of oligonucleotides comprising (a) and at least one of (b)-(e): (a) a C. coli-specific amplification oligomer set comprising first and second oligomers respectively comprising target-hybridizing sequences consisting of the nucleotide sequences of: (i) SEQ ID NO:8 and SEQ ID NO:10; (b) a *Salmonella-specific* amplification oligomer set comprising first and second oligomers respectively comprising target-hybridizing sequences consisting of the nucleotide sequences of: (i) SEQ ID NO:12 and SEQ ID NO:14; (ii) SEQ ID NO:21 and SEQ ID NO:47; (iii) SEQ ID NO:38 and SEQ ID NO:36; (iv) SEQ ID NO:35 and SEQ ID NO:40; (v) SEQ ID NO:12 and SEQ ID NO:28; (vi) SEQ ID NO:42 and SEQ ID NO:31; or (vii) SEQ ID NO:41 and SEQ ID NO:27; (c)a C. *jejuni*-specific amplification oligomer set comprising first and second oligomers respectively comprising target-hybridizing sequences consisting of the nucleotide sequences of: SEQ ID NO:11 and SEQ ID NO:19; (d) a *Shigella-specific* amplification oligomer set comprising first and second oligomers respectively comprising target-hybridizing sequences consisting of the nucleotide sequences of: (i) SEQ ID NO:15 and SEQ ID NO:17; (ii) SEQ ID NO:34 and SEQ ID NO:22; or (iii) SEQ ID NO:32 and SEQ ID NO:33; and (e) a Shigatoxigenic *E. coli* (STEC)-specific amplification oligomer set comprising first and second oligomers respectively comprising target-hybridizing sequences consisting of the nucleotide sequences of: (i) SEQ ID NO:20 and SEQ ID NO:3; (ii) SEQ ID NO:49 and SEQ ID NO:3; or (iii) SEQ ID NO:4 and SEQ ID NO:7.

## Description

### CROSS-REFERENCE TO RELATED APPLICATIONS

This application paragraphs the benefit under priority of United States Provisional Application No. 63/226,079, filed July 27, 2021, the disclosure of which is hereby incorporated by reference in its entirety.

### INTRODUCTION AND SUMMARY

Bacterial gastroenteritis is inflammation of the stomach and intestines that results in acute diarrhea (3 or more episodes per day) lasting less than 14 days and may also include symptoms such as nausea, vomiting, and abdominal cramping. See Thielman and Guerrant, The New England Journal of Medicine, 350:38-47, 2004. In the United States, it is estimated that there are more than 200 million cases of diarrheal illness per year, resulting in 73 million physician consultations, 1.8 million hospitalizations, and up to 6,000 deaths. *See* Guerrant et al., Clinical Infectious Diseases, 32:331-350, 2001. According to the Centers for Disease Control Food Net data (data compilation from 10 state health departments), in 2010, the number of reported infections and incidence per 100,000 population included the following: *Salmonella* (8256; 17.6), *Campylobacter* (6365; 13.6), and *Shigella* (1780; 3.8). *See* Centers for Disease Control and Prevention, "Vital Signs: Incidence and Trends of Infection with Pathogens Transmitted Commonly Through Food - Foodborne Diseases Active Surveillance Network, 10 U.S. Sites, 1996-2010," MMWR 60(22):749-755, 2011. These three bacteria, along with Shigatoxigenic *Escherichia coli* (STEC), are common causes of bacterial gastroenteritis. The populations most at risk due to bacterial gastroenteritis infection are children (≤5), the elderly, and the immunocompromised. Infection, however, can occur in all age groups. The mode of infection is via the fecal-oral route typically from ingesting contaminated food or water or as a result of poor hygiene (hand-washing).

*Salmonella* are gram-negative, aerobic, rod-shaped bacilli. There are two species of *Salmonella,* including *enterica* and *bongori. Salmonella enterica* is further divided into six subspecies with only a fraction of *Salmonella enterica* subspecies I being responsible for human illness. *See* Sabbagh et al., FEMS Microbiol Lett 305:1-13, 2010. *Salmonella* serotypes Typhimurium, Enteritidis, and Newport account for about half of the culture-confirmed *Salmonella* isolates in the US. *Salmonella* serotype Typhi, the strain that causes typhoid fever, is uncommon in the US, while *Salmonella* serotypes Mississippi and Javiana have been increasingly identified as a source of illness. See Centers for Disease Control and Prevention, "Summary of Notifiable Diseases-United States, 2008," MMWR 57(54):15-16, 2008.

*Campylobacter* are curved, motile, microaerophilic, gram-negative rods. They exhibit rapid, darting motility in a corkscrew fashion using one or two flagella and also have a lipopolysaccharide endotoxin. Two species of *Campylobacter, C. jejuni* and *C*. *coli,* are responsible for the vast majority of human infections. *See* Klena et al., Journal of Clinical Microbiology, 42:5549-5557, 2004; Poly and Guerry, Current Opinion in Gastroenterology 24:27-31, 2008; Granato et al., Journal of Clinical Microbiology, 48:4022-4027, 2010.

*E*. *coli* are gram-negative, rod-shaped bacteria that are commonly found in the lower intestines of warm-blooded organisms. Most strains of *E*. *coli* are non-pathogenic and are part of the normal gut flora. However, some strains, such as STEC, can cause life-threatening infections in humans. There are two main types of Shiga toxin produced by STEC, Shiga toxin type 1 and Shiga toxin type 2, which are carried on two different genes, *stx1* and *stx2*, respectively. Some strains of STEC contain the *stx1* gene while other strains contain the *stx2* gene. There are also certain strains of STEC that contain both the *stx1* and *stx2* genes.

*Shigella* are gram-negative, aerobic, rod-shaped bacteria that are closely related to *E. coli.* See Liu et al., FEMS Microbiol. Rev. 32:627-653, 2008. There are four species of *Shigella,* all of which can cause disease in humans and include *S*. *sonnei* (subgroup D), *S. flexneri* (subgroup B), *S*. *boydii* (subgroup B), and *S*. *dysenteriae* (subgroup A). According to the 2006 *Shigella* annual summary published by the CDC, *S*. *sonnei* is the most prevalent cause of infections at 76%, followed by *S. flexneri* (14%), *A. boydii* (1.1%), and *S. dysenteriae* (0.5%). See Centers for Disease Control and Prevention, "Shigella Surveillance: Annual Summary, 2006," Atlanta, GA: US Department of Health and Human Services, November 2008.

There is a need to efficiently and sensitively detect the presence of *Salmonella, Shigella, Campylobacter*, and STEC in samples, including biological specimens to provide diagnostic and prognostic information to physicians treating patients suffering from, or suspected of suffering from, bacterial gastroenteritis or related disorders.

Accordingly, provided herein is a set of oligonucleotides for determining the presence or absence of at least one enteric pathogen, said set of oligonucleotides comprising at least one of (a)-(e):
(a) a *Salmonella*-specific amplification oligomer set comprising first and second oligomers respectively comprising target-hybridizing sequences substantially corresponding to the nucleotide sequences of: (i) SEQ ID NO:12 and SEQ ID NO:14; (ii) SEQ ID NO:21 and SEQ ID NO:47; (iii) SEQ ID NO:38 and SEQ ID NO:36; (iv) SEQ ID NO:35 and SEQ ID NO:40; (v) SEQ ID NO:12 and SEQ ID NO:28; (vi) SEQ ID NO:42 and SEQ ID NO:31; or (vii) SEQ ID NO:41 and SEQ ID NO:27;
(b) a *C. jejuni-*specific amplification oligomer set comprising first and second oligomers respectively comprising target-hybridizing sequences substantially corresponding to the nucleotide sequences of: (i) SEQ ID NO:11 and SEQ ID NO:19;
(c) a *C*. *coli-*specific amplification oligomer set comprising first and second oligomers respectively comprising target-hybridizing sequences substantially corresponding to the nucleotide sequences of: (i) SEQ ID NO:8 and SEQ ID NO:10;
(d) a *Shigella*-specific amplification oligomer set comprising first and second oligomers respectively comprising target-hybridizing sequences substantially corresponding to the nucleotide sequences of: (i) SEQ ID NO:15 and SEQ ID NO:17; (ii) SEQ ID NO:34 and SEQ ID NO:22; or (iii) SEQ ID NO:32 and SEQ ID NO:33; and
(e) a Shigatoxigenic *E. coli* (STEC)-specific amplification oligomer set comprising first and second oligomers respectively comprising target-hybridizing sequences substantially corresponding to the nucleotide sequences of: (i) SEQ ID NO:20 and SEQ ID NO:3; (ii) SEQ ID NO:49 and SEQ ID NO:3; or (iii) SEQ ID NO:4 and SEQ ID NO:7.

In some embodiments, the set of oligonucleotides comprises the *Salmonella-*specific amplification oligomer set. In some embodiments, the *Salmonella-*specific amplification oligomer set comprises first and second oligomers respectively comprising target-hybridizing sequences substantially corresponding to the nucleotide sequences of SEQ ID NO:21 and SEQ ID NO:47. In some embodiments, the *Salmonella-*specific amplification oligomer set comprises first and second oligomers respectively comprising target-hybridizing sequences substantially corresponding to the nucleotide sequences of SEQ ID NO:38 and SEQ ID NO:36. In some embodiments, the *Salmonella-*specific amplification oligomer set comprises first and second oligomers respectively comprising target-hybridizing sequences substantially corresponding to the nucleotide sequences of SEQ ID NO:35 and SEQ ID NO:40. In some embodiments, the *Salmonella-*specific amplification oligomer set comprises first and second oligomers respectively comprising target-hybridizing sequences substantially corresponding to the nucleotide sequences of SEQ ID NO:12 and SEQ ID NO:28. In some embodiments, the *Salmonella-*specific amplification oligomer set comprises first and second oligomers respectively comprising target-hybridizing sequences substantially corresponding to the nucleotide sequences of SEQ ID NO:42 and SEQ ID NO:31. In some embodiments, the *Salmonella-*specific amplification oligomer set comprises first and second oligomers respectively comprising target-hybridizing sequences substantially corresponding to the nucleotide sequences of SEQ ID NO:41 and SEQ ID NO:27.

In some embodiments, the set of oligonucleotides comprises the *C. jejuni*-specific amplification oligomer set.

In some embodiments, the set of oligonucleotides comprises the *C*. *coli*-specific amplification oligomer set.

In some embodiments, the set of oligonucleotides comprises the *Shigella-specific* amplification oligomer set. In some embodiments, the *Shigella-*specific amplification oligomer set comprises first and second oligomers respectively comprising target-hybridizing sequences substantially corresponding to the nucleotide sequences of SEQ ID NO:15 and SEQ ID NO:17. In some embodiments, the *Shigella-*specific amplification oligomer set comprises first and second oligomers respectively comprising target-hybridizing sequences substantially corresponding to the nucleotide sequences of SEQ ID NO:34 and SEQ ID NO:22. In some embodiments, the *Shigella*-specific amplification oligomer set comprises first and second oligomers respectively comprising target-hybridizing sequences substantially corresponding to the nucleotide sequences of SEQ ID NO:32 and SEQ ID NO:33.

In some embodiments, the set of oligonucleotides comprises the STEC-specific amplification oligomer set.

In some embodiments, the set of oligonucleotides comprises at least two of the *Salmonella-*specific amplification oligomer set, the *C. jejuni*-specific amplification oligomer set, the *C*. *coli-specific* amplification oligomer set, the *Shigella-specific* amplification oligomer set, and the STEC-specific amplification oligomer set.

In some embodiments, the set of oligonucleotides comprises at least three of the *Salmonella-*specific amplification oligomer set, the *C. jejuni*-specific amplification oligomer set, the *C*. *coli-*specific amplification oligomer set, the *Shigella-*specific amplification oligomer set, and the STEC-specific amplification oligomer set.

In some embodiments, the set of oligonucleotides comprises at least four of the *Salmonella-*specific amplification oligomer set, the *C. jejuni*-specific amplification oligomer set, the *C*. *coli-*specific amplification oligomer set, the *Shigella-*specific amplification oligomer set, and the STEC-specific amplification oligomer set.

In some embodiments, the set of oligonucleotides comprises the *Salmonella-*specific amplification oligomer set, the *C. jejuni*-specific amplification oligomer set, the *C*. *coli*-specific amplification oligomer set, the *Shigella*-specific amplification oligomer set, and the STEC-specific amplification oligomer set.

In some embodiments, the *Salmonella-*specific amplification oligomer set comprises first and second oligomers respectively comprising target-hybridizing sequences substantially corresponding to the nucleotide sequences of SEQ ID NO:12 and SEQ ID NO:14;
the *C. jejuni-*specific amplification oligomer set comprises first and second oligomers respectively comprising target-hybridizing sequences substantially corresponding to the nucleotide sequences of SEQ ID NO:11 and SEQ ID NO:19;
the *C*. *coli-*specific amplification oligomer set comprises first and second oligomers respectively comprising target-hybridizing sequences substantially corresponding to the nucleotide sequences of SEQ ID NO:8 and SEQ ID NO:10;
the *Shigella-specific* amplification oligomer set comprises first and second oligomers respectively comprising target-hybridizing sequences substantially corresponding to the nucleotide sequences of SEQ ID NO:15 and SEQ ID NO:17; and
the STEC-specific amplification oligomer set comprises first and second oligomers respectively comprising target-hybridizing sequences substantially corresponding to the nucleotide sequences of (i) SEQ ID NO:20 and SEQ ID NO:3; and (ii) SEQ ID NO:4 and SEQ ID NO:7.

In some embodiments, the set of oligonucleotides further comprises
a *Salmonella* detection probe, comprising a target-hybridizing sequence substantially corresponding to the nucleotide sequence of:
SEQ ID NO:13 if the *Salmonella-*specific amplification oligomer set comprises first and second oligomers respectively comprising target-hybridizing sequences substantially corresponding to the nucleotide sequences of SEQ ID NO:12 and SEQ ID NO:14;
SEQ ID NO:13 if the *Salmonella-*specific amplification oligomer set comprises first and second oligomers respectively comprising target-hybridizing sequences substantially corresponding to the nucleotide sequences of SEQ ID NO:12 and SEQ ID NO:28;
SEQ ID NO:45 if the *Salmonella-*specific amplification oligomer set comprises first and second oligomers respectively comprising target-hybridizing sequences substantially corresponding to the nucleotide sequences of SEQ ID NO:21 and SEQ ID NO:47;
SEQ ID NO:44, SEQ ID NO:26, or SEQ ID NO:25 if the *Salmonella-*specific amplification oligomer set comprises first and second oligomers respectively comprising target-hybridizing sequences substantially corresponding to the nucleotide sequences of SEQ ID NO:38 and SEQ ID NO:36;
SEQ ID NO:30 if the *Salmonella-*specific amplification oligomer set comprises first and second oligomers respectively comprising target-hybridizing sequences substantially corresponding to the nucleotide sequences of SEQ ID NO:35 and SEQ ID NO:40;
SEQ ID NO:23 if the *Salmonella-*specific amplification oligomer set comprises first and second oligomers respectively comprising target-hybridizing sequences substantially corresponding to the nucleotide sequences of SEQ ID NO:42 and SEQ ID NO:31; or
SEQ ID NO:43 if the *Salmonella-*specific amplification oligomer set comprises first and second oligomers respectively comprising target-hybridizing sequences substantially corresponding to the nucleotide sequences of SEQ ID NO:41 and SEQ ID NO:27.

In some embodiments, in SEQ ID NO:23, one or more, or each of nucleotides 2, 5, 7, and 14 is 5-methylcytosine. In some embodiments, in SEQ ID NO:25, one or more, or each of nucleotides 7, 9, 13, and 15 is 5-methylcytosine. In some embodiments, in SEQ ID NO:26, one or more, or each of nucleotides 9, 18, and 23 is 5-methylcytosine. In some embodiments, in SEQ ID NO:27, one or more, or each of nucleotides 6, 10, and 18 is 5-methylcytosine. In some embodiments, in SEQ ID NO:30, one or more, or each of nucleotides 2, 4, 18, 19, and 20 is 5-methylcytosine. In some embodiments, in SEQ ID NO:43, one or more, or each of nucleotides 2, 9, 12, 16, 17, 22, 23, and 26 is 5-methylcytosine. In some embodiments, in SEQ ID NO:44, one or more, or each of nucleotides 7, 9, 13, and 15 is 5-methylcytosine. In some embodiments, in SEQ ID NO:45, one or more, or each of nucleotides 3, 6, 8, and 17 is 5-methylcytosine.

In some embodiments, the set of oligonucleotides further comprises a *C. jejuni* detection probe, the *C. jejuni* detection probe comprising a target-hybridizing sequence substantially corresponding to the nucleotide sequence of SEQ ID NO:18.

In some embodiments, in SEQ ID NO:18, one or more of nucleotides 7, 12, and 25, or each of nucleotides 7 and 12, or each of nucleotides 7, 12, and 25 is 5-methylcytosine.

In some embodiments, the set of oligonucleotides further comprises a *C*. *coli* detection probe, the *C*. *coli* detection probe comprising a target-hybridizing sequence substantially corresponding to the nucleotide sequence of SEQ ID NO:9, SEQ ID NO:37, or SEQ ID NO:39.

In some embodiments, in SEQ ID NO:9, one or more, or each of nucleotides 2, 6, 13, 16, 23, 26, 28, and 29 is 5-methylcytosine. In some embodiments, in SEQ ID NO:37, one or more, or each of nucleotides 17, 22, 23, and 27 is 5-methylcytosine. In some embodiments, in SEQ ID NO:39, one or more, or each of nucleotides 2, 9, 12, 19, 22, 24, and 25 is 5-methylcytosine.

In some embodiments, the set of oligonucleotides further comprises
a *Shigella* detection probe, the *Shigella* detection probe comprising a target-hybridizing sequence substantially corresponding to the nucleotide sequence of
SEQ ID NO:16 if the *Shigella*-specific amplification oligomer set comprises first and second oligomers respectively comprising target-hybridizing sequences substantially corresponding to the nucleotide sequences of SEQ ID NO:15 and SEQ ID NO:17;
SEQ ID NO:46 or SEQ ID NO:29 if the *Shigella*-specific amplification oligomer set comprises first and second oligomers respectively comprising target-hybridizing sequences substantially corresponding to the nucleotide sequences of SEQ ID NO:34 and SEQ ID NO:22; or
SEQ ID NO:24 if the *Shigella*-specific amplification oligomer set comprises first and second oligomers respectively comprising target-hybridizing sequences substantially corresponding to the nucleotide sequences of SEQ ID NO:32 and SEQ ID NO:33.

In some embodiments, in SEQ ID NO:46, each of nucleotides 5, 11, and 12 is 5-methylcytosine.

In some embodiments, the set of oligonucleotides further comprises
a STEC detection probe, the STEC detection probe comprising a target-hybridizing sequence substantially corresponding to the nucleotide sequence of
SEQ ID NO:1, SEQ ID NO:2, or SEQ ID NO:48 if the STEC-specific amplification oligomer set comprises first and second oligomers respectively comprising target-hybridizing sequences substantially corresponding to the nucleotide sequences of SEQ ID NO:20 and SEQ ID NO:3;
SEQ ID NO:1, SEQ ID NO:2, or SEQ ID NO:48 if the STEC-specific amplification oligomer set comprises first and second oligomers respectively comprising target-hybridizing sequences substantially corresponding to the nucleotide sequences of SEQ ID NO:49 and SEQ ID NO:3; or
SEQ ID NO:5 or SEQ ID NO:6 if the STEC-specific amplification oligomer set comprises first and second oligomers respectively comprising target-hybridizing sequences substantially corresponding to the nucleotide sequences of SEQ ID NO:4 and SEQ ID NO:7.

In some embodiments, in SEQ ID NO:1, one or more, or each of nucleotides 6, 7, 9, 14, 17, and 24 is 5-methylcytosine. In some embodiments, in SEQ ID NO:2, one or more, or each of nucleotides 5, 8, 11, 13, 18, and 21 is 5-methylcytosine. In some embodiments, in SEQ ID NO:6, one or more, or each of nucleotides 3, 7, 8, 15, 20, 24, and 27 is 5-methylcytosine. In some embodiments, in SEQ ID NO:15, one or more, or each of nucleotides 7 and 15 is 5-methylcytosine. In some embodiments, in SEQ ID NO:19, one or more, or each of nucleotides 1, 2, 7, 14, 15, 19, and 20 is 5-methylcytosine. In some embodiments, in SEQ ID NO:21, one or more, or each of nucleotides 13, 17, and 24 is 5-methylcytosine. In some embodiments, in SEQ ID NO:28, one or more, or each of nucleotides 2, 4, 12, 14, 15, and 17 is 5-methylcytosine. In some embodiments, in SEQ ID NO:31, one or more, or each of nucleotides 6, 7, 8, 13, 23, 24, and 26 is 5-methylcytosine. In some embodiments, in SEQ ID NO:32, one or more, or each of nucleotides 5, 11, 12, and 15 is 5-methylcytosine. In some embodiments, in SEQ ID NO:36, one or more, or each of nucleotides 2, 3, 4, 16, 18, 19, and 22 is 5-methylcytosine. In some embodiments, in SEQ ID NO:38, one or more, or each of nucleotides 4, 6, 7, and 8 is 5-methylcytosine. In some embodiments, in SEQ ID NO:40, one or more, or each of nucleotides 4, 13, 18, and 22 is 5-methylcytosine. In some embodiments, in SEQ ID NO:47, one or more, or each of nucleotides 11, 16, and 17 is 5-methylcytosine. In some embodiments, in SEQ ID NO:7, one or more, or each of nucleotides 3, 4, 18, and 19 is 5-methylcytosine. In some embodiments, in SEQ ID NO:12, one or more, or each of nucleotides 4, 11, 12, and 16 is 5-methylcytosine. In some embodiments, in SEQ ID NO:22, each of nucleotides 5 and 16 is 5-methylcytosine.

In some embodiments, one or more, or each, of the detection probes comprises a fluorescent dye compound. In some embodiments, each of the detection probes further comprises a non-fluorescent quenching dye compound.

Also provided herein is an oligonucleotide for determining the presence or absence of at least one enteric pathogen, wherein the oligonucleotide comprises the sequence of any one of:
SEQ ID NO:15, wherein each of nucleotides 7 and 15 is 5-methylcytosine;
SEQ ID NO:16;
SEQ ID NO:19, wherein each of nucleotides 1, 2, 7, 14, 15, 19, and 20 is 5-methylcytosine;
SEQ ID NO:21, wherein each of nucleotides 13, 17, and 24 is 5-methylcytosine;
SEQ ID NO:23, wherein each of nucleotides 2, 5, 7, and 14 is 5-methylcytosine;
SEQ ID NO:25, wherein each of nucleotides 7, 9, 13, and 15 is 5-methylcytosine;
SEQ ID NO:26, wherein each of nucleotides 9, 18, and 23 is 5-methylcytosine;
SEQ ID NO:27, wherein each of nucleotides 6, 10, and 18 comprises 5-methylcytosine;
SEQ ID NO:28, wherein each of nucleotides 2, 4, 12, 14, 15, and 17 is 5-methylcytosine;
SEQ ID NO:30, wherein each of nucleotides 2, 4, 18, 19, and 20 is 5-methylcytosine;
SEQ ID NO:31, wherein each of nucleotides 6, 7, 8, 13, 23, 24, and 26 is 5-methylcytosine;
SEQ ID NO:32, wherein each of nucleotides 5, 11, 12, and 15 is 5-methylcytosine;
SEQ ID NO:36, wherein each of nucleotides 2, 3, 4, 16, 18, 19, and 22 is 5-methylcytosine;
SEQ ID NO:38, wherein each of nucleotides 4, 6, 7, and 8 is 5-methylcytosine;
SEQ ID NO:39, wherein each of nucleotides 2, 9, 12, 19, 22, 24, and 25 is 5-methylcytosine;
SEQ ID NO:40, wherein each of nucleotides 4, 13, 18, and 22 is 5-methylcytosine; SEQ ID NO:42;
SEQ ID NO:43, wherein each of nucleotides 2, 9, 12, 16, 17, 22, 23, and 26 is 5-methylcytosine;
SEQ ID NO:44, wherein each of nucleotides 7, 9, 13, and 15 is 5-methylcytosine;
SEQ ID NO:45, wherein each of nucleotides 3, 6, 8, and 17 is 5-methylcytosine;
SEQ ID NO:47, wherein each of nucleotides 11, 16, and 17 is 5-methylcytosine;
SEQ ID NO:1, wherein each of nucleotides 6, 7, 9, 14, 17, and 24 is 5-methylcytosine;
SEQ ID NO:2, wherein each of nucleotides 5, 8, 11, 13, 18, and 21 is 5-methylcytosine;
SEQ ID NO:6, wherein each of nucleotides 3, 7, 8, 15, 20, 24, and 27 is 5-methylcytosine;
SEQ ID NO:7, wherein each of nucleotides 3, 4, 18, and 19 is 5-methylcytosine;
SEQ ID NO:9, wherein each of nucleotides 2, 6, 13, 16, 23, 26, 28, and 29 is 5-methylcytosine;
SEQ ID NO:12, wherein each of nucleotides 4, 11, 12, and 16 is 5-methylcytosine;
SEQ ID NO:18, wherein each of nucleotides 7 and 12 or each of nucleotides 7, 12, and 25 is 5-methylcytosine;
SEQ ID NO:22, wherein each of nucleotides 5 and 16 is 5-methylcytosine;
SEQ ID NO:37, wherein each of nucleotides 17, 22, 23, and 27 is 5-methylcytosine; and
SEQ ID NO:46, wherein each of nucleotides 5, 11, and 12 is 5-methylcytosine.

In some embodiments, the oligonucleotide comprises the sequence of any one of:
SEQ ID NO:15, wherein each of nucleotides 7 and 15 is 5-methylcytosine;
SEQ ID NO:16;
SEQ ID NO:19, wherein each of nucleotides 1, 2, 7, 14, 15, 19, and 20 is 5-methylcytosine;
SEQ ID NO:21, wherein each of nucleotides 13, 17, and 24 is 5-methylcytosine;
SEQ ID NO:23, wherein each of nucleotides 2, 5, 7, and 14 is 5-methylcytosine;
SEQ ID NO:25, wherein each of nucleotides 7, 9, 13, and 15 is 5-methylcytosine;
SEQ ID NO:26, wherein each of nucleotides 9, 18, and 23 is 5-methylcytosine;
SEQ ID NO:27, wherein each of nucleotides 6, 10, and 18 comprises 5-methylcytosine;
SEQ ID NO:28, wherein each of nucleotides 2, 4, 12, 14, 15, and 17 is 5-methylcytosine;
SEQ ID NO:30, wherein each of nucleotides 2, 4, 18, 19, and 20 is 5-methylcytosine;
SEQ ID NO:31, wherein each of nucleotides 6, 7, 8, 13, 23, 24, and 26 is 5-methylcytosine;
SEQ ID NO:32, wherein each of nucleotides 5, 11, 12, and 15 is 5-methylcytosine;
SEQ ID NO:36, wherein each of nucleotides 2, 3, 4, 16, 18, 19, and 22 is 5-methylcytosine;
SEQ ID NO:38, wherein each of nucleotides 4, 6, 7, and 8 is 5-methylcytosine;
SEQ ID NO:39, wherein each of nucleotides 2, 9, 12, 19, 22, 24, and 25 is 5-methylcytosine;
SEQ ID NO:40, wherein each of nucleotides 4, 13, 18, and 22 is 5-methylcytosine;
SEQ ID NO:42;
SEQ ID NO:43, wherein each of nucleotides 2, 9, 12, 16, 17, 22, 23, and 26 is 5-methylcytosine;
SEQ ID NO:44, wherein each of nucleotides 7, 9, 13, and 15 is 5-methylcytosine;
SEQ ID NO:45, wherein each of nucleotides 3, 6, 8, and 17 is 5-methylcytosine; and
SEQ ID NO:47, wherein each of nucleotides 11, 16, and 17 is 5-methylcytosine.

Also provided herein is a kit comprising the set of oligonucleotides of any one of the above embodiments or the oligonucleotide of any one of the above embodiments.

Also provided herein is a reaction mixture comprising the set of oligonucleotides of any one of the above embodiments or the oligonucleotide of any one of the above embodiments.

Also provided herein is a multiplex method, comprising:
(1) contacting a sample, said sample suspected of containing at least one enteric pathogen, with the set of oligonucleotides of any one of the preceding embodiments;
(2) performing an *in vitro* nucleic acid amplification reaction, wherein any *Salmonella*, *C. jejuni*, *C. coli*, *Shigella,* and STEC target nucleic acid, if present in said sample, is used as a template for generating one or more amplification products corresponding to the *Salmonella, C. jejuni*, *C. coli*, *Shigella*, and STEC target regions; and
(3) detecting the presence or absence of the one or more amplification products, thereby determining the presence or absence of at least one of *Salmonella*, *C. jejuni*, *C. coli*, *Shigella,* and STEC in said sample.

In some embodiments, step (3) of the multiplex method comprises:
contacting the sample with at least one of the *Salmonella* detection probe, the *C. j juni* detection probe, the *C*. *coli* detection probe, the *Shigella* detection probe, and the STEC detection probe;
performing electrophoresis on the sample; or
determining the sequence of the one or more amplification product, if present.

Also provided herein is a method for synthesizing an oligonucleotide, comprising the steps of:
(a) obtaining a solid support comprising at least one nucleobase residue, wherein at least one nucleobase residue is covalently bound at a 3' position to the solid support;
(b) coupling a 5' position of the nucleobase residue furtherest from the solid support to a 3' position of another nucleobase residue;
(c) repeating step (b) at least 13 additional times, thereby generating at least 15 contiguous nucleobase residues coupled to the solid support; and
(d) cleaving the at least 15 contiguous nucleobase residues generated in step (c), thereby obtaining the oligonucleotide,
wherein the oligonucleotide comprises the sequence of any one of:
SEQ ID NO:15, wherein each of nucleotides 7 and 15 is 5-methylcytosine;
SEQ ID NO:16;
SEQ ID NO:19, wherein each of nucleotides 1, 2, 7, 14, 15, 19, and 20 is 5-methylcytosine;
SEQ ID NO:21, wherein each of nucleotides 13, 17, and 24 is 5-methylcytosine;
SEQ ID NO:23, wherein each of nucleotides 2, 5, 7, and 14 is 5-methylcytosine;
SEQ ID NO:25, wherein each of nucleotides 7, 9, 13, and 15 is 5-methylcytosine;
SEQ ID NO:26, wherein each of nucleotides 9, 18, and 23 is 5-methylcytosine;
SEQ ID NO:27, wherein each of nucleotides 6, 10, and 18 comprises 5-methylcytosine;
SEQ ID NO:28, wherein each of nucleotides 2, 4, 12, 14, 15, and 17 is 5-methylcytosine;
SEQ ID NO:30, wherein each of nucleotides 2, 4, 18, 19, and 20 is 5-methylcytosine;
SEQ ID NO:31, wherein each of nucleotides 6, 7, 8, 13, 23, 24, and 26 is 5-methylcytosine;
SEQ ID NO:32, wherein each of nucleotides 5, 11, 12, and 15 is 5-methylcytosine;
SEQ ID NO:36, wherein each of nucleotides 2, 3, 4, 16, 18, 19, and 22 is 5-methylcytosine;
SEQ ID NO:38, wherein each of nucleotides 4, 6, 7, and 8 is 5-methylcytosine;
SEQ ID NO:39, wherein each of nucleotides 2, 9, 12, 19, 22, 24, and 25 is 5-methylcytosine;
SEQ ID NO:40, wherein each of nucleotides 4, 13, 18, and 22 is 5-methylcytosine;
SEQ ID NO:42;
SEQ ID NO:43, wherein each of nucleotides 2, 9, 12, 16, 17, 22, 23, and 26 is 5-methylcytosine;
SEQ ID NO:44, wherein each of nucleotides 7, 9, 13, and 15 is 5-methylcytosine;
SEQ ID NO:45, wherein each of nucleotides 3, 6, 8, and 17 is 5-methylcytosine;
SEQ ID NO:47, wherein each of nucleotides 11, 16, and 17 is 5-methylcytosine;
SEQ ID NO:1, wherein each of nucleotides 6, 7, 9, 14, 17, and 24 is 5-methylcytosine;
SEQ ID NO:2, wherein each of nucleotides 5, 8, 11, 13, 18, and 21 is 5-methylcytosine;
SEQ ID NO:6, wherein each of nucleotides 3, 7, 8, 15, 20, 24, and 27 is 5-methylcytosine;
SEQ ID NO:7, wherein each of nucleotides 3, 4, 18, and 19 is 5-methylcytosine;
SEQ ID NO:9, wherein each of nucleotides 2, 6, 13, 16, 23, 26, 28, and 29 is 5-methylcytosine;
SEQ ID NO:12, wherein each of nucleotides 4, 11, 12, and 16 is 5-methylcytosine;
SEQ ID NO:18, wherein each of nucleotides 7 and 12 or each of nucleotides 7, 12, and 25 is 5-methylcytosine;
SEQ ID NO:22, wherein each of nucleotides 5 and 16 is 5-methylcytosine;
SEQ ID NO:37, wherein each of nucleotides 17, 22, 23, and 27 is 5-methylcytosine; and
SEQ ID NO:46, wherein each of nucleotides 5, 11, and 12 is 5-methylcytosine.

In some embodiments, the oligonucleotide has a length of from 18 to 32 contiguous nucleobase residues. In some embodiments, the oligonucleotide has a length of from 20 to 30 contiguous nucleobase residues.

Also provided herein is a method for synthesizing a pair of oligonucleotides, comprising of synthesizing a first oligonucleotide and synthesizing a second oligonucleotide, wherein each of the synthesizing the first oligonucleotide and the synthesizing the second oligonucleotide comprises the steps of:
(a) obtaining a solid support comprising at least one nucleobase residue, wherein at least one nucleobase residue is covalently bound at a 3' position to the solid support;
(b) coupling a 5' position of the nucleobase residue furtherest from the solid support to a 3' position of another nucleobase residue;
(c) repeating step (b) at least 13 additional times, thereby generating at least 15 contiguous nucleobase residues coupled to the solid support; and
(d) cleaving at least 15 contiguous nucleobase residues generated in step (c), thereby obtaining the oligonucleotide, and
wherein the first oligonucleotide and the second oligonucleotide respectively comprise the sequence of any one of:
SEQ ID NO:12 and SEQ ID NO:14;
SEQ ID NO:21 and SEQ ID NO:47;
SEQ ID NO:38 and SEQ ID NO:36;
SEQ ID NO:35 and SEQ ID NO:40;
SEQ ID NO:12 and SEQ ID NO:28;
SEQ ID NO:42 and SEQ ID NO:31;
SEQ ID NO:41 and SEQ ID NO:27;
SEQ ID NO:11 and SEQ ID NO:19;
SEQ ID NO:8 and SEQ ID NO:10;
SEQ ID NO:15 and SEQ ID NO:17;
SEQ ID NO:34 and SEQ ID NO:22;
SEQ ID NO:32 and SEQ ID NO:33;
SEQ ID NO:49 and SEQ ID NO:3;
SEQ ID NO:20 and SEQ ID NO:3; and
SEQ ID NO:4 and SEQ ID NO:7.

In some embodiments, each of the first oligonucleotide and the second oligonucleotide have lengths of from 18 to 32 contiguous nucleobase residues. In some embodiments, each of the first oligonucleotide and the second oligonucleotide have lengths of from 20 to 30 contiguous nucleobase residues.

Accordingly, the following embodiments are among those provided by the disclosure.

Embodiment 1 is a set of oligonucleotides for determining the presence or absence of at least one enteric pathogen, said set of oligonucleotides comprising at least one of (a)-(e):
(a) a *Salmonella*-specific amplification oligomer set comprising first and second oligomers respectively comprising target-hybridizing sequences substantially corresponding to the nucleotide sequences of: (i) SEQ ID NO:12 and SEQ ID NO:14; (ii) SEQ ID NO:21 and SEQ ID NO:47; (iii) SEQ ID NO:38 and SEQ ID NO:36; (iv) SEQ ID NO:35 and SEQ ID NO:40; (v) SEQ ID NO:12 and SEQ ID NO:28; (vi) SEQ ID NO:42 and SEQ ID NO:31; or (vii) SEQ ID NO:41 and SEQ ID NO:27;
(b) a *C. jejuni-*specific amplification oligomer set comprising first and second oligomers respectively comprising target-hybridizing sequences substantially corresponding to the nucleotide sequences of: (i) SEQ ID NO:11 and SEQ ID NO:19;
(c) a *C*. *coli-*specific amplification oligomer set comprising first and second oligomers respectively comprising target-hybridizing sequences substantially corresponding to the nucleotide sequences of: (i) SEQ ID NO:8 and SEQ ID NO:10;
(d) a *Shigella-*specific amplification oligomer set comprising first and second oligomers respectively comprising target-hybridizing sequences substantially corresponding to the nucleotide sequences of: (i) SEQ ID NO:15 and SEQ ID NO:17; (ii) SEQ ID NO:34 and SEQ ID NO:22; or (iii) SEQ ID NO:32 and SEQ ID NO:33; and
(e) a Shigatoxigenic *E. coli* (STEC)-specific amplification oligomer set comprising first and second oligomers respectively comprising target-hybridizing sequences substantially corresponding to the nucleotide sequences of: (i) SEQ ID NO:20 and SEQ ID NO:3; (ii) SEQ ID NO:49 and SEQ ID NO:3; or (iii) SEQ ID NO:4 and SEQ ID NO:7.

Embodiment 2 is the set of oligonucleotides of embodiment 1, comprising the *Salmonella-*specific amplification oligomer set.

Embodiment 3 is the set of oligonucleotides of embodiment 2, wherein the *Salmonella-*specific amplification oligomer set comprises first and second oligomers respectively comprising target-hybridizing sequences substantially corresponding to the nucleotide sequences of SEQ ID NO:21 and SEQ ID NO:47.

Embodiment 4 is the set of oligonucleotides of embodiment 2, wherein the *Salmonella-*specific amplification oligomer set comprises first and second oligomers respectively comprising target-hybridizing sequences substantially corresponding to the nucleotide sequences of SEQ ID NO:38 and SEQ ID NO:36.

Embodiment 5 is the set of oligonucleotides of embodiment 2, wherein the *Salmonella-*specific amplification oligomer set comprises first and second oligomers respectively comprising target-hybridizing sequences substantially corresponding to the nucleotide sequences of SEQ ID NO:35 and SEQ ID NO:40.

Embodiment 6 is the set of oligonucleotides of embodiment 2, wherein the *Salmonella-*specific amplification oligomer set comprises first and second oligomers respectively comprising target-hybridizing sequences substantially corresponding to the nucleotide sequences of SEQ ID NO:12 and SEQ ID NO:28.

Embodiment 7 is the set of oligonucleotides of embodiment 2, wherein the *Salmonella-*specific amplification oligomer set comprises first and second oligomers respectively comprising target-hybridizing sequences substantially corresponding to the nucleotide sequences of SEQ ID NO:42 and SEQ ID NO:31.

Embodiment 8 is the set of oligonucleotides of embodiment 2, wherein the *Salmonella-*specific amplification oligomer set comprises first and second oligomers respectively comprising target-hybridizing sequences substantially corresponding to the nucleotide sequences of SEQ ID NO:41 and SEQ ID NO:27.

Embodiment 9 is the set of oligonucleotides of any one of the preceding embodiments, comprising the *C. jejuni-*specific amplification oligomer set.

Embodiment 10 is the set of oligonucleotides of any one of the preceding embodiments, comprising the *C*. *coli-*specific amplification oligomer set.

Embodiment 11 is the set of oligonucleotides of any one of the preceding embodiments, comprising the *Shigella-*specific amplification oligomer set.

Embodiment 12 is the set of oligonucleotides of embodiment 11, wherein the *Shigella-*specific amplification oligomer set comprises first and second oligomers respectively comprising target-hybridizing sequences substantially corresponding to the nucleotide sequences of SEQ ID NO:15 and SEQ ID NO:17.

Embodiment 13 is the set of oligonucleotides of embodiment 11, wherein the *Shigella-*specific amplification oligomer set comprises first and second oligomers respectively comprising target-hybridizing sequences substantially corresponding to the nucleotide sequences of SEQ ID NO:34 and SEQ ID NO:22.

Embodiment 14 is the set of oligonucleotides of embodiment 11, wherein the *Shigella-*specific amplification oligomer set comprises first and second oligomers respectively comprising target-hybridizing sequences substantially corresponding to the nucleotide sequences of SEQ ID NO:32 and SEQ ID NO:33.

Embodiment 15 is the set of oligonucleotides of any one of the preceding embodiments, comprising the STEC-specific amplification oligomer set.

Embodiment 16 is the set of oligonucleotides of any one of the preceding embodiments, comprising at least two of the *Salmonella-*specific amplification oligomer set, the *C. jejuni-*specific amplification oligomer set, the *C*. *coli-*specific amplification oligomer set, the *Shigella-*specific amplification oligomer set, and the STEC-specific amplification oligomer set.

Embodiment 17 is the set of oligonucleotides of any one of the preceding
embodiments, comprising at least three of the *Salmonella-*specific amplification oligomer set, the *C. jejuni*-specific amplification oligomer set, the *C*. *coli-*specific amplification oligomer set, the *Shigella-*specific amplification oligomer set, and the STEC-specific amplification oligomer set.

Embodiment 18 is the set of oligonucleotides of any one of the preceding embodiments, comprising at least four of the *Salmonella-*specific amplification oligomer set, the *C. jejuni*-specific amplification oligomer set, the *C*. *coli-*specific amplification oligomer set, the *Shigella-*specific amplification oligomer set, and the STEC-specific amplification oligomer set.

Embodiment 19 is the set of oligonucleotides of any one of the preceding embodiments, comprising the *Salmonella-*specific amplification oligomer set, the *C. jejuni-*specific amplification oligomer set, the *C*. *coli-*specific amplification oligomer set, the *Shigella-*specific amplification oligomer set, and the STEC-specific amplification oligomer set.

Embodiment 20 is the set of oligonucleotides of the immediately preceding embodiment, wherein
the *Salmonella-*specific amplification oligomer set comprises first and second oligomers respectively comprising target-hybridizing sequences substantially corresponding to the nucleotide sequences of SEQ ID NO:12 and SEQ ID NO:14;
the *C. jejuni*-specific amplification oligomer set comprises first and second oligomers respectively comprising target-hybridizing sequences substantially corresponding to the nucleotide sequences of SEQ ID NO:11 and SEQ ID NO:19;
the *C*. *coli-*specific amplification oligomer set comprises first and second oligomers respectively comprising target-hybridizing sequences substantially corresponding to the nucleotide sequences of SEQ ID NO:8 and SEQ ID NO:10;
the *Shigella*-specific amplification oligomer set comprises first and second oligomers respectively comprising target-hybridizing sequences substantially corresponding to the nucleotide sequences of SEQ ID NO:15 and SEQ ID NO:17; and
the STEC-specific amplification oligomer set comprises first and second oligomers respectively comprising target-hybridizing sequences substantially corresponding to the nucleotide sequences of (i) SEQ ID NO:20 and SEQ ID NO:3; and (ii) SEQ ID NO:4 and SEQ ID NO:7.

Embodiment 21 is the set of oligonucleotides of any one of embodiments 2-20, further comprising:
a *Salmonella* detection probe, comprising a target-hybridizing sequence substantially corresponding to the nucleotide sequence of:
SEQ ID NO:13 if the *Salmonella-*specific amplification oligomer set comprises first and second oligomers respectively comprising target-hybridizing sequences substantially corresponding to the nucleotide sequences of SEQ ID NO:12 and SEQ ID NO:14;
SEQ ID NO:13 if the *Salmonella-*specific amplification oligomer set comprises first and second oligomers respectively comprising target-hybridizing sequences substantially corresponding to the nucleotide sequences of SEQ ID NO:12 and SEQ ID NO:28;
SEQ ID NO:45 if the *Salmonella-*specific amplification oligomer set comprises first and second oligomers respectively comprising target-hybridizing sequences substantially corresponding to the nucleotide sequences of SEQ ID NO:21 and SEQ ID NO:47;
SEQ ID NO:44, SEQ ID NO:26, or SEQ ID NO:25 if the *Salmonella-*specific amplification oligomer set comprises first and second oligomers respectively comprising target-hybridizing sequences substantially corresponding to the nucleotide sequences of SEQ ID NO:38 and SEQ ID NO:36;
SEQ ID NO:30 if the *Salmonella-*specific amplification oligomer set comprises first and second oligomers respectively comprising target-hybridizing sequences substantially corresponding to the nucleotide sequences of SEQ ID NO:35 and SEQ ID NO:40;
SEQ ID NO:23 if the *Salmonella-*specific amplification oligomer set comprises first and second oligomers respectively comprising target-hybridizing sequences substantially corresponding to the nucleotide sequences of SEQ ID NO:42 and SEQ ID NO:31; or
SEQ ID NO:43 if the *Salmonella-*specific amplification oligomer set comprises first and second oligomers respectively comprising target-hybridizing sequences substantially corresponding to the nucleotide sequences of SEQ ID NO:41 and SEQ ID NO:27.

Embodiment 22 is the set of oligonucleotides of embodiment 21, wherein in SEQ ID NO:23, one or more, or each of nucleotides 2, 5, 7, and 14 comprises 5-methylcytosine.

Embodiment 23 is the set of oligonucleotides of embodiment 21, wherein in SEQ ID NO:25, one or more, or each of nucleotides 7, 9, 13, and 15 comprises 5-methylcytosine.

Embodiment 24 is the set of oligonucleotides of embodiment 21, wherein in SEQ ID NO:26, one or more, or each of nucleotides 9, 18, and 23 comprises 5-methylcytosine.

Embodiment 25 is the set of oligonucleotides of embodiment 21, wherein in SEQ ID NO:27, one or more, or each of nucleotides 6, 10, and 18 comprises 5-methylcytosine.

Embodiment 26 is the set of oligonucleotides of embodiment 21, wherein in SEQ ID NO:30, one or more, or each of nucleotides 2, 4, 18, 19, and 20 comprises 5-methylcytosine.

Embodiment 27 is the set of oligonucleotides of embodiment 21, wherein in SEQ ID NO:43, one or more, or each of nucleotides 2, 9, 12, 16, 17, 22, 23, and 26 comprises 5-methylcytosine.

Embodiment 28 is the set of oligonucleotides of embodiment 21, wherein in SEQ ID NO:44, one or more, or each of nucleotides 7, 9, 13, and 15 comprises 5-methylcytosine.

Embodiment 29 is the set of oligonucleotides of embodiment 21, wherein in SEQ ID NO:45, one or more, or each of nucleotides 3, 6, 8, and 17 comprises 5-methylcytosine.

Embodiment 30 is the set of oligonucleotides of any one of embodiments 9-27, further comprising a *C. jejuni* detection probe, the *C. jejuni* detection probe comprising a target-hybridizing sequence substantially corresponding to the nucleotide sequence of SEQ ID NO:18.

Embodiment 31 is the set of oligonucleotides of embodiment 29, wherein in SEQ ID NO:18, one or more of nucleotides 7, 12, and 25, or each of nucleotides 7 and 12, or each of nucleotides 7, 12, and 25 comprises 5-methylcytosine.

Embodiment 32 is the set of oligonucleotides of any one of embodiments 10-30, further comprising a *C*. *coli* detection probe, the *C*. *coli* detection probe comprising a target-hybridizing sequence substantially corresponding to the nucleotide sequence of SEQ ID NO:9, SEQ ID NO:37, or SEQ ID NO:39.

Embodiment 33 is the set of oligonucleotides of embodiment 31, wherein in SEQ ID NO:9, one or more, or each of nucleotides 2, 6, 13, 16, 23, 26, 28, and 29 comprises 5-methylcytosine.

Embodiment 34 is the set of oligonucleotides of embodiment 31, wherein in SEQ ID NO:37, one or more, or each of nucleotides 17, 22, 23, and 27 comprises 5-methylcytosine.

Embodiment 35 is the set of oligonucleotides of embodiment 31, wherein in SEQ ID NO:39, one or more, or each of nucleotides 2, 9, 12, 19, 22, 24, and 25 comprises 5-methylcytosine.

Embodiment 36 is the set of oligonucleotides of any one of embodiments 11-35, further comprising:
a *Shigella* detection probe, the *Shigella* detection probe comprising a target-hybridizing sequence substantially corresponding to the nucleotide sequence of
SEQ ID NO:16 if the *Shigella*-specific amplification oligomer set comprises first and second oligomers respectively comprising target-hybridizing sequences substantially corresponding to the nucleotide sequences of SEQ ID NO:15 and SEQ ID NO:17;
SEQ ID NO:46 or SEQ ID NO:29 if the *Shigella*-specific amplification oligomer set comprises first and second oligomers respectively comprising target-hybridizing sequences substantially corresponding to the nucleotide sequences of SEQ ID NO:34 and SEQ ID NO:22; or
SEQ ID NO:24 if the *Shigella*-specific amplification oligomer set comprises first and second oligomers respectively comprising target-hybridizing sequences substantially corresponding to the nucleotide sequences of SEQ ID NO:32 and SEQ ID NO:33.

Embodiment 37 is the set of oligonucleotides of embodiment 36, wherein in SEQ ID NO:46, each of nucleotides 5, 11, and 12 comprises 5-methylcytosine.

Embodiment 38 is the set of oligonucleotides of any one of embodiments 15-37, further comprising:
a STEC detection probe, the STEC detection probe comprising a target-hybridizing sequence substantially corresponding to the nucleotide sequence of
SEQ ID NO:1, SEQ ID NO:2, or SEQ ID NO:48 if the STEC-specific amplification oligomer set comprises first and second oligomers respectively comprising target-hybridizing sequences substantially corresponding to the nucleotide sequences of SEQ ID NO:20 and SEQ ID NO:3;
SEQ ID NO:1, SEQ ID NO:2, or SEQ ID NO:48 if the STEC-specific amplification oligomer set comprises first and second oligomers respectively comprising target-hybridizing sequences substantially corresponding to the nucleotide sequences of SEQ ID NO:49 and SEQ ID NO:3; or
SEQ ID NO:5 or SEQ ID NO:6 if the STEC-specific amplification oligomer set comprises first and second oligomers respectively comprising target-hybridizing sequences substantially corresponding to the nucleotide sequences of SEQ ID NO:4 and SEQ ID NO:7.

Embodiment 39 is the set of oligonucleotides of embodiment 38, wherein in SEQ ID NO:1, one or more, or each of nucleotides 6, 7, 9, 14, 17, and 24 comprises 5-methylcytosine.

Embodiment 40 is the set of oligonucleotides of embodiment 38, wherein in SEQ ID NO:2, one or more, or each of nucleotides 5, 8, 11, 13, 18, and 21 comprises 5-methylcytosine.

Embodiment 41 is the set of oligonucleotides of embodiment 38, wherein in SEQ ID NO:6, one or more, or each of nucleotides 3, 7, 8, 15, 20, 24, and 27 comprises 5-methylcytosine.

Embodiment 42 is the set of oligonucleotides of any one of the preceding embodiments, wherein in SEQ ID NO:15, one or more, or each of nucleotides 7 and 15 comprises 5-methylcytosine.

Embodiment 43 is the set of oligonucleotides of any one of the preceding embodiments, wherein in SEQ ID NO:19, one or more, or each of nucleotides 1, 2, 7, 14, 15, 19, and 20 comprises 5-methylcytosine.

Embodiment 44 is the set of oligonucleotides of any one of the preceding embodiments, wherein in SEQ ID NO:21, one or more, or each of nucleotides 13, 17, and 24 comprises 5-methylcytosine.

Embodiment 45 is the set of oligonucleotides of any one of the preceding embodiments, wherein in SEQ ID NO:28, one or more, or each of nucleotides 2, 4, 12, 14, 15, and 17 comprises 5-methylcytosine.

Embodiment 46 is the set of oligonucleotides of any one of the preceding embodiments, wherein in SEQ ID NO:31, one or more, or each of nucleotides 6, 7, 8, 13, 23, 24, and 26 comprises 5-methylcytosine.

Embodiment 47 is the set of oligonucleotides of any one of the preceding embodiments, wherein in SEQ ID NO:32, one or more, or each of nucleotides 5, 11, 12, and 15 comprises 5-methylcytosine.

Embodiment 48 is the set of oligonucleotides of any one of the preceding embodiments, wherein in SEQ ID NO:36, one or more, or each of nucleotides 2, 3, 4, 16, 18, 19, and 22 comprises 5-methylcytosine.

Embodiment 49 is the set of oligonucleotides of any one of the preceding embodiments, wherein in SEQ ID NO:38, one or more, or each of nucleotides 4, 6, 7, and 8 comprises 5-methylcytosine.

Embodiment 50 is the set of oligonucleotides of any one of the preceding embodiments, wherein in SEQ ID NO:40, one or more, or each of nucleotides 4, 13, 18, and 22 comprises 5-methylcytosine.

Embodiment 51 is the set of oligonucleotides of any one of the preceding embodiments, wherein in SEQ ID NO:47, one or more, or each of nucleotides 11, 16, and 17 comprises 5-methylcytosine.

Embodiment 52 is the set of oligonucleotides of any one of the preceding embodiments, wherein in SEQ ID NO:7, one or more, or each of nucleotides 3, 4, 18, and 19 comprises 5-methylcytosine.

Embodiment 53 is the set of oligonucleotides of any one of the preceding embodiments, wherein in SEQ ID NO:12, one or more, or each of nucleotides 4, 11, 12, and 16 comprises 5-methylcytosine.

Embodiment 54 is the set of oligonucleotides of any one of the preceding embodiments, wherein in SEQ ID NO:22, each of nucleotides 5 and 16 comprises 5-methylcytosine.

Embodiment 55 is the set of oligonucleotides of any one of embodiments 21-54, wherein one or more, or each, of the detection probes comprises a fluorescent dye compound.

Embodiment 56 is the set of oligonucleotides of the immediately preceding embodiment, wherein each of the detection probes further comprises a non-fluorescent quenching dye compound.

Embodiment 57 is an oligonucleotide for determining the presence or absence of at least one enteric pathogen, wherein the oligonucleotide comprises the sequence of any one of:
SEQ ID NO:15, wherein each of nucleotides 7 and 15 comprises 5-methylcytosine;
SEQ ID NO:16;
SEQ ID NO:19, wherein each of nucleotides 1, 2, 7, 14, 15, 19, and 20 comprises 5-methylcytosine;
SEQ ID NO:21, wherein each of nucleotides 13, 17, and 24 comprises 5-methylcytosine;
SEQ ID NO:23, wherein each of nucleotides 2, 5, 7, and 14 comprises 5-methylcytosine;
SEQ ID NO:25, wherein each of nucleotides 7, 9, 13, and 15 comprises 5-methylcytosine;
SEQ ID NO:26, wherein each of nucleotides 9, 18, and 23 comprises 5-methylcytosine;
SEQ ID NO:27, wherein each of nucleotides 6, 10, and 18 comprises 5-methylcytosine;
SEQ ID NO:28, wherein each of nucleotides 2, 4, 12, 14, 15, and 17 comprises 5-methylcytosine;
SEQ ID NO:30, wherein each of nucleotides 2, 4, 18, 19, and 20 comprises 5-methylcytosine;
SEQ ID NO:31, wherein each of nucleotides 6, 7, 8, 13, 23, 24, and 26 comprises 5-methylcytosine;
SEQ ID NO:32, wherein each of nucleotides 5, 11, 12, and 15 comprises 5-methylcytosine;
SEQ ID NO:36, wherein each of nucleotides 2, 3, 4, 16, 18, 19, and 22 comprises 5-methylcytosine;
SEQ ID NO:38, wherein each of nucleotides 4, 6, 7, and 8 comprises 5-methylcytosine;
SEQ ID NO:39, wherein each of nucleotides 2, 9, 12, 19, 22, 24, and 25 comprises 5-methylcytosine;
SEQ ID NO:40, wherein each of nucleotides 4, 13, 18, and 22 comprises 5-methylcytosine;
SEQ ID NO:42;
SEQ ID NO:43, wherein each of nucleotides 2, 9, 12, 16, 17, 22, 23, and 26 comprises 5-methylcytosine;
SEQ ID NO:44, wherein each of nucleotides 7, 9, 13, and 15 comprises 5-methylcytosine;
SEQ ID NO:45, wherein each of nucleotides 3, 6, 8, and 17 comprises 5-methylcytosine;
SEQ ID NO:47, wherein each of nucleotides 11, 16, and 17 comprises 5-methylcytosine;
SEQ ID NO:1, wherein each of nucleotides 6, 7, 9, 14, 17, and 24 comprises 5-methylcytosine;
SEQ ID NO:2, wherein each of nucleotides 5, 8, 11, 13, 18, and 21 comprises 5-methylcytosine;
SEQ ID NO:6, wherein each of nucleotides 3, 7, 8, 15, 20, 24, and 27 comprises 5-methylcytosine;
SEQ ID NO:7, wherein each of nucleotides 3, 4, 18, and 19 comprises 5-methylcytosine;
SEQ ID NO:9, wherein each of nucleotides 2, 6, 13, 16, 23, 26, 28, and 29 comprises 5-methylcytosine;
SEQ ID NO:12, wherein each of nucleotides 4, 11, 12, and 16 comprises 5-methylcytosine;
SEQ ID NO:18, wherein each of nucleotides 7 and 12 or each of nucleotides 7, 12, and 25 comprises 5-methylcytosine;
SEQ ID NO:22, wherein each of nucleotides 5 and 16 comprises 5-methylcytosine;
SEQ ID NO:37, wherein each of nucleotides 17, 22, 23, and 27 comprises 5-methylcytosine; and
SEQ ID NO:46, wherein each of nucleotides 5, 11, and 12 comprises 5-methylcytosine.

Embodiment 58 is the oligonucleotide of the immediately preceding embodiment, wherein the oligonucleotide comprises the sequence of any one of:
SEQ ID NO:15, wherein each of nucleotides 7 and 15 comprises 5-methylcytosine;
SEQ ID NO:16;
SEQ ID NO:19, wherein each of nucleotides 1, 2, 7, 14, 15, 19, and 20 comprises 5-methylcytosine;
SEQ ID NO:21, wherein each of nucleotides 13, 17, and 24 comprises 5-methylcytosine;
SEQ ID NO:23, wherein each of nucleotides 2, 5, 7, and 14 comprises 5-methylcytosine;
SEQ ID NO:25, wherein each of nucleotides 7, 9, 13, and 15 comprises 5-methylcytosine;
SEQ ID NO:26, wherein each of nucleotides 9, 18, and 23 comprises 5-methylcytosine;
SEQ ID NO:27, wherein each of nucleotides 6, 10, and 18 comprises 5-methylcytosine;
SEQ ID NO:28, wherein each of nucleotides 2, 4, 12, 14, 15, and 17 comprises 5-methylcytosine;
SEQ ID NO:30, wherein each of nucleotides 2, 4, 18, 19, and 20 comprises 5-methylcytosine;
SEQ ID NO:31, wherein each of nucleotides 6, 7, 8, 13, 23, 24, and 26 comprises 5-methylcytosine;
SEQ ID NO:32, wherein each of nucleotides 5, 11, 12, and 15 comprises 5-methylcytosine;
SEQ ID NO:36, wherein each of nucleotides 2, 3, 4, 16, 18, 19, and 22 comprises 5-methylcytosine;
SEQ ID NO:38, wherein each of nucleotides 4, 6, 7, and 8 comprises 5-methylcytosine;
SEQ ID NO:39, wherein each of nucleotides 2, 9, 12, 19, 22, 24, and 25 comprises 5-methylcytosine;
SEQ ID NO:40, wherein each of nucleotides 4, 13, 18, and 22 comprises 5-methylcytosine;
SEQ ID NO:42;
SEQ ID NO:43, wherein each of nucleotides 2, 9, 12, 16, 17, 22, 23, and 26 comprises 5-methylcytosine;
SEQ ID NO:44, wherein each of nucleotides 7, 9, 13, and 15 comprises 5-methylcytosine;
SEQ ID NO:45, wherein each of nucleotides 3, 6, 8, and 17 comprises 5-methylcytosine; and
SEQ ID NO:47, wherein each of nucleotides 11, 16, and 17 comprises 5-methylcytosine.

Embodiment 59 is a kit comprising the set of oligonucleotides of any one of embodiments 1-56 or the oligonucleotide of any one of embodiments 57-58.

Embodiment 60 is a reaction mixture comprising the set of oligonucleotides of any one of embodiments 1-55 or the oligonucleotide of any one of embodiments 57-58.

Embodiment 61 is the reaction mixture of the immediately preceding embodiment, further comprising α-cyclodextrin or polysorbate 20.

Embodiment 62 is the reaction mixture of embodiment 60 or 61, further comprising α-cyclodextrin and polysorbate 20.

Embodiment 63 is the reaction mixture of embodiment 61 or 62, wherein the concentration of α-cyclodextrin in the reaction mixture is from about 10 mg/mL to about 40 mg/mL.

Embodiment 64 is the reaction mixture of any one of embodiments 61-63, wherein the concentration of α-cyclodextrin in the reaction mixture is from about 16 mg/mL to about 30 mg/mL, from about 15 mg/mL to about 20 mg/mL, or from about 10 mg/mL to about 15 mg/mL.

Embodiment 65 is the reaction mixture of any one of embodiments 61-64, wherein the concentration of α-cyclodextrin in the reaction mixture is about 20 mg/mL, about 17.5 mg/mL, or about 12.5 mg/mL.

Embodiment 66 is the reaction mixture of any one of embodiments 61-65, wherein the concentration of polysorbate 20 in the reaction mixture is from about 0.002% to about 0.05% (v/v).

Embodiment 67 is the reaction mixture of any one of embodiments 61-66, wherein the concentration of polysorbate 20 in the reaction mixture is from about 0.003% to about 0.03% (v/v).

Embodiment 68 is the reaction mixture of any one of embodiments 61-67, wherein the concentration of polysorbate 20 in the reaction mixture is about 0.0042% (v/v), about 0.0035% (v/v), about 0.0026% (v/v), or about 0.02% (v/v).

Embodiment 69 is the reaction mixture of any one of embodiments 61-68, further comprising a detergent.

Embodiment 70 is the reaction mixture of the immediately preceding embodiment, wherein the detergent comprises sodium dodecyl sulfate.

Embodiment 71 is the reaction mixture of embodiment 69 or 70, wherein the concentration of the detergent in the reaction mixture is from about 3 mg/mL to 300 mg/mL.

Embodiment 72 is the reaction mixture of any one of embodiments 69-71, wherein the concentration of the detergent in the reaction mixture is from about 10 mg/mL to 100 mg/mL.

Embodiment 73 is the reaction mixture of any one of embodiments 69-72, wherein the concentration of the detergent in the reaction mixture is about 33.3 mg/mL.

Embodiment 74 is a multiplex method, comprising:
(1) contacting a sample, said sample suspected of containing the at least one enteric pathogen, with the set of oligonucleotides of any one of embodiments 1-56;
(2) performing an *in vitro* nucleic acid amplification reaction, wherein any *Salmonella, C. jejuni*, *C. coli*, *Shigella*, and STEC target nucleic acid, if present in said sample, is used as a template for generating one or more amplification products corresponding to the *Salmonella, C. jejuni*, *C. coli*, *Shigella*, and STEC target regions; and
(3) detecting the presence or absence of the one or more amplification products,
thereby determining the presence or absence of at least one of *Salmonella*, *C. jejuni*, *C. coli*, *Shigella*, and STEC in said sample.

Embodiment 74.1 is the multiplex method of the immediately preceding embodiment, wherein the *in vitro* nucleic acid amplification reaction occurs in a reaction mixture of any one of embodiments 60-73.

Embodiment 75 is the multiplex method of embodiment 74, wherein (3) comprises:
contacting the sample with at least one of the *Salmonella* detection probe, the *C. j juni* detection probe, the *C*. *coli* detection probe, the *Shigella* detection probe, and the STEC detection probe;
performing electrophoresis on the sample; or
determining the sequence of the one or more amplification product, if present.

Embodiment 75.1 is the multiplex method of the immediately preceding embodiment, wherein the *in vitro* nucleic acid amplification reaction occurs in a reaction mixture of any one of embodiments 60-73.

Embodiment 76 is a method for synthesizing an oligonucleotide, comprising the steps of:
(a) obtaining a solid support comprising at least one nucleobase residue, wherein the at least one nucleobase residue is covalently bound at a 3' position to the solid support;
(b) coupling a 5' position of the nucleobase residue furtherest from the solid support to a 3' position of another nucleobase residue;
(c) repeating step (b) at least 13 additional times, thereby generating at least 15 contiguous nucleobase residues coupled to the solid support; and
(d) cleaving the at least 15 contiguous nucleobase residues generated in step (c), thereby obtaining the oligonucleotide,
wherein the oligonucleotide comprises the sequence of any one of:
SEQ ID NO:15, wherein each of nucleotides 7 and 15 comprises 5-methylcytosine;
SEQ ID NO:16;
SEQ ID NO:19, wherein each of nucleotides 1, 2, 7, 14, 15, 19, and 20 comprises 5-methylcytosine;
SEQ ID NO:21, wherein each of nucleotides 13, 17, and 24 comprises 5-methylcytosine;
SEQ ID NO:23, wherein each of nucleotides 2, 5, 7, and 14 comprises 5-methylcytosine;
SEQ ID NO:25, wherein each of nucleotides 7, 9, 13, and 15 comprises 5-methylcytosine;
SEQ ID NO:26, wherein each of nucleotides 9, 18, and 23 comprises 5-methylcytosine;
SEQ ID NO:27, wherein each of nucleotides 6, 10, and 18 comprises 5-methylcytosine;
SEQ ID NO:28, wherein each of nucleotides 2, 4, 12, 14, 15, and 17 comprises 5-methylcytosine;
SEQ ID NO:30, wherein each of nucleotides 2, 4, 18, 19, and 20 comprises 5-methylcytosine;
SEQ ID NO:31, wherein each of nucleotides 6, 7, 8, 13, 23, 24, and 26 comprises 5-methylcytosine;
SEQ ID NO:32, wherein each of nucleotides 5, 11, 12, and 15 comprises 5-methylcytosine;
SEQ ID NO:36, wherein each of nucleotides 2, 3, 4, 16, 18, 19, and 22 comprises 5-methylcytosine;
SEQ ID NO:38, wherein each of nucleotides 4, 6, 7, and 8 comprises 5-methylcytosine;
SEQ ID NO:39, wherein each of nucleotides 2, 9, 12, 19, 22, 24, and 25 comprises 5-methylcytosine;
SEQ ID NO:40, wherein each of nucleotides 4, 13, 18, and 22 comprises 5-methylcytosine;
SEQ ID NO:42;
SEQ ID NO:43, wherein each of nucleotides 2, 9, 12, 16, 17, 22, 23, and 26 comprises 5-methylcytosine;
SEQ ID NO:44, wherein each of nucleotides 7, 9, 13, and 15 comprises 5-methylcytosine;
SEQ ID NO:45, wherein each of nucleotides 3, 6, 8, and 17 comprises 5-methylcytosine;
SEQ ID NO:47, wherein each of nucleotides 11, 16, and 17 comprises 5-methylcytosine;
SEQ ID NO:1, wherein each of nucleotides 6, 7, 9, 14, 17, and 24 comprises 5-methylcytosine;
SEQ ID NO:2, wherein each of nucleotides 5, 8, 11, 13, 18, and 21 comprises 5-methylcytosine;
SEQ ID NO:6, wherein each of nucleotides 3, 7, 8, 15, 20, 24, and 27 comprises 5-methylcytosine;
SEQ ID NO:7, wherein each of nucleotides 3, 4, 18, and 19 comprises 5-methylcytosine;
SEQ ID NO:9, wherein each of nucleotides 2, 6, 13, 16, 23, 26, 28, and 29 comprises 5-methylcytosine;
SEQ ID NO:12, wherein each of nucleotides 4, 11, 12, and 16 comprises 5-methylcytosine;
SEQ ID NO:18, wherein each of nucleotides 7 and 12 or each of nucleotides 7, 12, and 25 comprises 5-methylcytosine;
SEQ ID NO:22, wherein each of nucleotides 5 and 16 comprises 5-methylcytosine;
SEQ ID NO:37, wherein each of nucleotides 17, 22, 23, and 27 comprises 5-methylcytosine; and
SEQ ID NO:46, wherein each of nucleotides 5, 11, and 12 comprises 5-methylcytosine.

Embodiment 77 is the method of embodiment 76, wherein the oligonucleotide has a length of from 18 to 32 contiguous nucleobase residues, or from 20 to 30 contiguous nucleobase residues.

Embodiment 78 is a method for synthesizing a pair of oligonucleotides, comprising synthesizing a first oligonucleotide and synthesizing a second oligonucleotide,
wherein each of the synthesizing the first oligonucleotide and the synthesizing the second oligonucleotide comprises the steps of:
   (a) obtaining a solid support comprising at least one nucleobase residue, wherein the at least one nucleobase residue is covalently bound at a 3' position to the solid support;
   (b) coupling a 5' position of the nucleobase residue furtherest from the solid support to a 3' position of another nucleobase residue;
   (c) repeating step (b) at least 13 additional times, thereby generating at least 15 contiguous nucleobase residues coupled to the solid support; and
   (d) cleaving the at least 15 contiguous nucleobase residues generated in step (c), thereby obtaining the oligonucleotide, and
wherein the first oligonucleotide and the the second oligonucleotide respectively comprise the sequence of any one of:
   SEQ ID NO:12 and SEQ ID NO:14;
   SEQ ID NO:21 and SEQ ID NO:47;
   SEQ ID NO:38 and SEQ ID NO:36;
   SEQ ID NO:35 and SEQ ID NO:40;
   SEQ ID NO:12 and SEQ ID NO:28;
   SEQ ID NO:42 and SEQ ID NO:31;
   SEQ ID NO:41 and SEQ ID NO:27;
   SEQ ID NO:11 and SEQ ID NO:19;
   SEQ ID NO:8 and SEQ ID NO:10;
   SEQ ID NO:15 and SEQ ID NO:17;
   SEQ ID NO:34 and SEQ ID NO:22;
   SEQ ID NO:32 and SEQ ID NO:33;
   SEQ ID NO:49 and SEQ ID NO:3;
   SEQ ID NO:20 and SEQ ID NO:3; and
   SEQ ID NO:4 and SEQ ID NO:7.

Embodiment 79 is the method of embodiment 78, wherein each of the first oligonucleotide and the second oligonucleotide have a length of from 18 to 32 contiguous nucleobase residues, or from 20 to 30 contiguous nucleobase residues.

### DETAILED DESCRIPTION OF CERTAIN EMBODIMENTS

### Definitions

Before describing the present teachings in detail, it is to be understood that the disclosure is not limited to specific compositions or process steps, as such may vary. It should be noted that, as used in this specification and the appended claims, the singular form "a," "an," and "the" include plural references unless the context clearly dictates otherwise. Thus, for example, reference to "an oligomer" includes a plurality of oligomers and the like. The conjunction "or" is to be interpreted in the inclusive sense, i.e., as equivalent to "and/or," unless the inclusive sense would be unreasonable in the context.

It will be appreciated that there is an implied "about" prior to the temperatures, concentrations, times, etc. discussed in the present disclosure, such that slight and insubstantial deviations are within the scope of the present teachings herein. In general, the term "about" indicates insubstantial variation in a quantity of a component of a composition not having any significant effect on the activity or stability of the composition. All ranges are to be interpreted as encompassing the endpoints in the absence of express exclusions such as "not including the endpoints"; thus, for example, "within 10-15" includes the values 10 and 15. Also, the use of "comprise," "comprises," "comprising," "contain," "contains," "containing," "include," "includes," and "including" are not intended to be limiting. It is to be understood that both the foregoing general description and detailed description are exemplary and explanatory only and are not restrictive of the teachings. To the extent that any material incorporated by reference is inconsistent with the express content of this disclosure, the express content controls.

Unless specifically noted, embodiments in the specification that recite "comprising" various components are also contemplated as "consisting of" or "consisting essentially of" the recited components; embodiments in the specification that recite "consisting of' various components are also contemplated as "comprising" or "consisting essentially of' the recited components; and embodiments in the specification that recite "consisting essentially of" various components are also contemplated as "consisting of' or "comprising" the recited components (this interchangeability does not apply to the use of these terms in the claims). "Consisting essentially of' means that additional component(s), composition(s) or method step(s) that do not materially change the basic and novel characteristics of the compositions and methods described herein may be included in those compositions or methods. Such characteristics include the ability to detect an enteric pathogen's nucleic acid sequence present in a sample with specificity that distinguishes the nucleic acid from other known pathogens, optionally at a sensitivity that can detect from 75 to 150 CFU/mL of the enteric pathogen, and, optionally from about 90 to about 180 minutes or from about 120 to about 150 minutes, and/or from about 30 cycles to about 60 cycles, from about 40 cycles to about 50 cycles, or about 45 cycles, from the beginning of an amplification reaction when a cycled amplification reaction is used.

A "sample" or "specimen," including "biological" or "clinical" samples, refers to any material that may contain or is suspected of containing one or more of *Salmonella, Shigella*, *Campylobacter jejuni*, *Campylobacter coli*, and STEC, or components thereof, such as nucleic acids or fragments of nucleic acids. A sample may be a complex mixture of components. Samples include "biological samples" which include any tissue or material derived from a living or dead mammal or organism, including, for example, stool, blood, plasma, serum, blood cells, saliva, mucous and cerebrospinal fluid. A sample may include stool from an organism experiencing one or more symptoms of infection by one or more of *Salmonella*, *Shigella*, *Campylobacter jejuni*, *Campylobacter coli*, and STEC. A sample may also be a "spiked" sample, such as stool that is from an organism not experiencing any symptoms of infection but to which one or more of *Salmonella*, *Shigella*, *Campylobacter jejuni*, *Campylobacter coli,* and STEC is artificially added. Samples may also include samples of *in vitro* cell culture constituents including, for example, conditioned media resulting from the growth of cells and tissues in culture medium. The sample may be treated to chemically, physically or mechanically to disrupt tissue or cell structure to release intracellular nucleic acids into a solution which may contain enzymes, buffers, salts, detergents and the like, to prepare the sample for analysis. In one step of the methods described herein, a sample is provided that is suspected of containing target nucleic acid of at least one enteric pathogen, such as *Salmonella*, *Shigella, C. jejuni*, *C. coli*, and STEC. Accordingly, this step excludes the physical step of obtaining the sample from a subject.

"Nucleic acid" and "polynucleotide" refer to a multimeric compound comprising nucleosides or nucleoside analogs which have nitrogenous heterocyclic bases or base analogs linked together to form a polynucleotide, including conventional RNA, DNA, mixed RNA-DNA, and polymers that are analogs thereof. A nucleic acid "backbone" may be made up of a variety of linkages, including one or more of sugar-phosphodiester linkages, peptide-nucleic acid bonds ("peptide nucleic acids" or PNA; PCT No. WO 95/32305), phosphorothioate linkages, methylphosphonate linkages, or combinations thereof. Sugar moieties of a nucleic acid may be ribose, deoxyribose, or similar compounds with substitutions, e.g., 2' methoxy or 2' halide substitutions. Nitrogenous bases may be conventional bases (A, G, C, T, U), analogs thereof (e.g., inosine or others; see The Biochemistry of the Nucleic Acids 5-36, Adams et al., ed., 11th ed., 1992), derivatives of purines or pyrimidines (e.g., N⁴-methyl deoxyguanosine, deaza- or aza-purines, deaza- or azapyrimidines, pyrimidine bases with substituent groups at the 5 or 6 position, purine bases with a substituent at the 2, 6, or 8 positions, 2-amino-6-methylaminopurine, O⁶-methylguanine, 4-thio-pyrimidines, 4-amino-pyrimidines, 4-dimethylhydrazine-pyrimidines, and O⁴-alkyl-pyrimidines; US Pat. No. 5,378,825 and PCT No. WO 93/13121). Nucleic acids may include one or more "abasic" residues where the backbone includes no nitrogenous base for position(s) of the polymer (US Pat. No. 5,585,481). A nucleic acid may comprise only conventional RNA or DNA sugars, bases and linkages, or may include both conventional components and substitutions (e.g., conventional bases with 2' methoxy linkages, or polymers containing both conventional bases and one or more base analogs). Nucleic acid includes "locked nucleic acid" (LNA), an analogue containing one or more LNA nucleotide monomers with a bicyclic furanose unit locked in an RNA mimicking sugar conformation, which enhance hybridization affinity toward complementary RNA and DNA sequences (Vester and Wengel, 2004, Biochemistry 43(42):13233-41). Embodiments of oligomers that may affect stability of a hybridization complex include PNA oligomers, oligomers that include 2'-methoxy or 2'-fluoro substituted RNA, or oligomers that affect the overall charge, charge density, or steric associations of a hybridization complex, including oligomers that contain charged linkages (e.g., phosphorothioates) or neutral groups (e.g., methylphosphonates). 5-methylcytosines may be used in conjunction with any of the foregoing backbones/sugars/linkages including RNA or DNA backbones (or mixtures thereof) unless otherwise indicated. It is understood that when referring to ranges for the length of an oligonucleotide, amplicon, or other nucleic acid, that the range is inclusive of all whole numbers (e.g., 19-25 contiguous nucleotides in length includes 19, 20, 21, 22, 23, 24, and 25).

"C" or "cytosine" residues include methylated and unmethylated cytosines unless the context indicates otherwise.

An "oligonucleotide" or "oligomer" refers to a nucleic acid of generally less than 1,000 nucleotides (nt), including those in a size range having a lower limit of about 2 to 5 nt and an upper limit of about 500 to 900 nt. Some particular embodiments are oligonucleotides in a size range with a lower limit of about 5 to 15, 16, 17, 18, 19, or 20 nt and an upper limit of about 50 to 600 nt, and other particular embodiments are in a size range with a lower limit of about 10 to 20 nt and an upper limit of about 22 to 100 nt. Oligonucleotides may be purified from naturally occurring sources, but may be synthesized by using any well known enzymatic or chemical method. Oligonucleotides may be referred to by a functional name (e.g., capture probe, primer or promoter primer) but those skilled in the art will understand that such terms refer to oligomers.

By "amplicon" or "amplification product" is meant a nucleic acid molecule generated in a nucleic acid amplification reaction and which is derived from a target nucleic acid. An amplicon or amplification product contains a target nucleic acid sequence that may be of the same or opposite sense as the target nucleic acid.

An "amplification oligonucleotide" or "amplification oligomer" refers to an oligonucleotide that hybridizes to a target nucleic acid, or its complement, and participates in a nucleic acid amplification reaction, e.g., serving as a primer or and promoter-primer. Particular amplification oligonucleotides contain at least about 10 contiguous bases, and optionally at least 11, 12, 13, 14, 15, 16, 17, 18, 19, or 20 contiguous bases, that are complementary to a region of the target nucleic acid sequence or its complementary strand. The contiguous bases may be at least about 80%, at least about 90%, or completely complementary to the target sequence to which the amplification oligomer binds. One skilled in the art will understand that the recited ranges include all whole and rational numbers within the range (e.g., 92% or 98.377%). Particular amplification oligonucleotides are about 10 to about 60 bases long and optionally may include modified nucleotides.

A "primer" refers to an oligonucleotide that hybridizes to a template nucleic acid and has a 3' end that is extended by polymerization. A primer may be optionally modified, e.g., by including a 5' region that is non-complementary to the target sequence. Such modification can include functional additions, such as tags, promoters, or other sequences used or useful for manipulating or amplifying the primer or target oligonucleotide.

Within the context of transcription mediated amplification, a primer modified with a 5' promoter sequence may be referred to as a "promoter-primer." A person of ordinary skill in the art of molecular biology or biochemistry will understand that an oligonucleotide that can function as a primer can be modified to include a 5' promoter sequence and then function as a promoter-primer, and, similarly, any promoter-primer can serve as a primer with or without its 5' promoter sequence.

"Nucleic acid amplification" refers to any *in vitro* procedure that produces multiple copies of a target nucleic acid sequence, or its complementary sequence, or fragments thereof (i.e., an amplified sequence containing less than the complete target nucleic acid). Examples of nucleic acid amplification procedures include transcription associated methods, such as transcription-mediated amplification (TMA), nucleic acid sequence-based amplification (NASBA) and others (e.g., US Pat. Nos. 5,399,491, 5,554,516, 5,437,990, 5,130,238, 4,868,105, and 5,124,246), replicase-mediated amplification (e.g., US Pat. No. 4,786,600), the polymerase chain reaction (PCR) (e.g., US Pat. Nos. 4,683,195, 4,683,202, and 4,800,159), ligase chain reaction (LCR) (e.g., EP Pat. App. 0320308), helicase-dependent amplification (e.g., US Pat. No. 7,282,328), and strand-displacement amplification (SDA) (e.g., US Pat. No. 5,422,252). Amplification may be linear or exponential. Replicase-mediated amplification uses self-replicating RNA molecules, and a replicase such as QB-replicase. PCR amplification uses DNA polymerase, primers, and thermal cycling steps to synthesize multiple copies of the two complementary strands of DNA or cDNA. LCR amplification uses at least four separate oligonucleotides to amplify a target and its complementary strand by using multiple cycles of hybridization, ligation, and denaturation. Helicase-dependent amplification uses a helicase to separate the two strands of a DNA duplex generating single-stranded templates, followed by hybridization of sequence-specific primers hybridize to the templates and extension by DNA polymerase to amplify the target sequence. SDA uses a primer that contains a recognition site for a restriction endonuclease that will nick one strand of a hemimodified DNA duplex that includes the target sequence, followed by amplification in a series of primer extension and strand displacement steps. Particular embodiments use PCR or TMA, but it will be apparent to persons of ordinary skill in the art that oligonucleotides disclosed herein may be readily used as primers in other amplification methods.

Transcription associated amplification uses a DNA polymerase, an RNA polymerase, deoxyribonucleoside triphosphates, ribonucleoside triphosphates, a promoter-containing oligonucleotide, and optionally may include other oligonucleotides, to ultimately produce multiple RNA transcripts from a nucleic acid template (described in detail in US Pat. Nos. 5,399,491 and 5,554,516, Kacian et al., US Pat. No. 5,437,990, Burg et al., PCT Nos. WO 88/01302 and WO 88/10315, Gingeras et al., US Pat. No. 5,130,238, Malek et al., US Pat. Nos. 4,868,105 and 5,124,246, Urdea et al., PCT No. WO 94/03472, McDonough et al., PCT No. WO 95/03430, and Ryder et al.). Methods that use TMA are described in detail previously (US Pat. Nos. 5,399,491 and 5,554,516).

In cyclic amplification methods that detect amplicons in real-time, the term "threshold cycle" (Ct) is a measure of the emergence time of a signal associated with amplification of target, and is generally 10x standard deviation of the normalized reporter signal. Once an amplification reaches the "threshold cycle," generally there is considered to be a positive amplification product of a sequence to which the probe binds. The identity of the amplification product can then be determined through methods known to one of skill in the art, such as gel electrophoresis, nucleic acid sequencing, and other such analytical procedures.

As used herein, the term "relative fluorescence unit" ("RFU") is a unit of measurement of fluorescence intensity. RFU varies with the characteristics of the detection means used for the measurement, and can be used as a measurement to compare relative intensities between samples and controls.

"Detection probe" or "probe" refers to an oligonucleotide that hybridizes specifically to a target sequence, including an amplified sequence, under conditions that promote nucleic acid hybridization, for detection of the target nucleic acid. Detection may either be direct (i.e., probe hybridized directly to the target) or indirect (i.e., a probe hybridized to an intermediate structure that links the probe to the target). A probe's target sequence generally refers to the specific sequence within a larger sequence which the probe hybridizes specifically. A detection probe may include target-specific sequences and a non-target-complementary sequence. Such non-target-complementary sequences can include sequences which will confer a desired secondary or tertiary structure, such as a hairpin structure, which can be used to facilitate detection and/or amplification (e.g., US Pat. Nos. 5,118,801, 5,312,728, 6,835,542, and 6,849,412). Probes of a defined sequence may be produced by techniques known to those of ordinary skill in the art, such as by chemical synthesis, and by *in vitro* or in vivo expression from recombinant nucleic acid molecules.$

As used herein, a nucleic acid "substantially corresponding to" a specified nucleic acid sequence, or its complement, means that the oligonucleotide is sufficiently similar to the reference nucleic acid sequence such that the oligonucleotide has similar hybridization properties to the reference nucleic acid sequence in that it would hybridize with the same target nucleic acid sequence under stringent hybridization conditions. Substantially corresponding nucleic acids vary by at least one nucleotide from the specified nucleic acid. This variation may be stated in terms of a percentage of sequence identity or complementarity between the nucleic acid and the specified nucleic acid (e.g., from less than 100% to about 80%). One skilled in the art will understand that the recited ranges include all whole and rational numbers of the range (e.g., 92%, 92.377%, etc).

By "hybridization" or "hybridize" is meant the ability of two completely or partially complementary nucleic acid strands to come together under specified hybridization assay conditions in a parallel or antiparallel orientation to form a stable structure having a double-stranded region. The two constituent strands of this double-stranded structure, sometimes called a hybrid, are held together by hydrogen bonds. Although these hydrogen bonds most commonly form between nucleotides containing the bases adenine and thymine or uracil (A and T or U) or cytosine and guanine (C and G) on single nucleic acid strands, base pairing can also form between bases which are not members of these "canonical" pairs. Non-canonical base pairing is well-known in the art. (*See, e.g.*, Adams et al., The Biochemistry of the Nucleic Acids (11th ed. 1992).)

By "preferentially hybridize" is meant that under stringent hybridization conditions, an amplification or detection probe oligonucleotide can hybridize to its target nucleic acid to form stable oligonucleotide:target hybrid, but not form a sufficient number of stable oligonucleotide:non-target hybrids. Amplification and detection oligonucleotides that preferentially hybridize to a target nucleic acid are useful to amplify and detect target nucleic acids, but not non-targeted organisms, especially phylogenetically closely related organisms. Thus, the oligonucleotide hybridizes to target nucleic acid to a sufficiently greater extent than to non-target nucleic acid to enable one having ordinary skill in the art to accurately amplify and/or detect the presence (or absence) of nucleic acid derived from the specified enteric pathogen as appropriate. In general, reducing the degree of complementarity between an oligonucleotide sequence and its target sequence will decrease the degree or rate of hybridization of the oligonucleotide to its target region. However, the inclusion of one or more non-complementary nucleosides or nucleobases may facilitate the ability of an oligonucleotide to discriminate against non-target organisms.

Preferential hybridization can be measured using techniques known in the art and described herein, such as in the examples provided below. In some embodiments, there is at least a 10-fold difference between target and non-target hybridization signals in a test sample, at least a 100-fold difference, or at least a 1,000-fold difference. In some embodiments, non-target hybridization signals in a test sample are no more than the background signal level.

By "stringent hybridization conditions" or "stringent conditions" is meant conditions permitting an oligomer to preferentially hybridize to a target nucleic acid (such as an enteric pathogen nucleic acid) and not to nucleic acid derived from a closely related non-target nucleic acid. While the definition of stringent hybridization conditions does not vary, the actual reaction environment that can be used for stringent hybridization may vary depending upon factors including the GC content and length of the oligonucleotide, the degree of similarity between the oligonucleotide sequence and sequences of non-target nucleic acids that may be present in the test sample, and the target sequence. Hybridization conditions include the temperature and the composition of the hybridization reagents or solutions. Exemplary hybridization assay conditions for amplifying and/or detecting target nucleic acids derived from one or more the target enteric pathogens with the oligonucleotides of the present disclosure correspond to a temperature of about 60 °C when the salt concentration, such as a monovalent salt, e.g., KCl, is in the range of about 0.06-0.09 M. Specific hybridization assay conditions are set forth *infra* in the Examples section. Other acceptable stringent hybridization conditions could be easily ascertained by those having ordinary skill in the art.

By "assay conditions" is meant conditions permitting stable hybridization of an oligonucleotide to a target nucleic acid. Assay conditions do not require preferential hybridization of the oligonucleotide to the target nucleic acid.

"Label" or "detectable label" refers to a moiety or compound joined directly or indirectly to a probe that is detected or leads to a detectable signal. Direct joining may use covalent bonds or non-covalent interactions (e.g., hydrogen bonding, hydrophobic or ionic interactions, and chelate or coordination complex formation) whereas indirect joining may use a bridging moiety or linker (e.g., via an antibody or additional oligonucleotide(s), which amplify a detectable signal. Any detectable moiety may be used, e.g., radionuclide, ligand such as biotin or avidin, enzyme, enzyme substrate, reactive group, chromophore such as a dye or particle (e.g., latex or metal bead) that imparts a detectable color, luminescent compound (e.g. bioluminescent, phosphorescent, or chemiluminescent compound), and fluorescent compound (i.e., fluorophore). Embodiments of fluorophores include those that absorb light in the range of about 495 to 690 nm and emit light in the range of about 520 to 705 nm, which include those known as FAM^{™}, TET^{™}, CAL FLUOR^{™} (Orange or Red), and QUASAR^{™} compounds. Fluorophores may be used in combination with a quencher molecule that absorbs light when in close proximity to the fluorophore to diminish background fluorescence. Such quenchers are well known in the art and include, e.g., BLACK HOLE QUENCHER^{™} (or BHQ^{™}) or TAMRA^{™} compounds. Particular embodiments include a "homogeneous detectable label" that is detectable in a homogeneous system in which bound labeled probe in a mixture exhibits a detectable change compared to unbound labeled probe, which allows the label to be detected without physically removing hybridized from unhybridized labeled probe (e.g., US Pat. Nos. 5,283,174, 5,656,207, and 5,658,737). Particular homogeneous detectable labels include chemiluminescent compounds, including acridinium ester ("AE") compounds, such as standard AE or AE derivatives, which are well known (US Pat. Nos. 5,656,207, 5,658,737, and 5,639,604). Methods of synthesizing labels, attaching labels to nucleic acid, and detecting signals from labels are well known (e.g., Sambrook et al., Molecular Cloning, A Laboratory Manual, 2nd ed. (Cold Spring Harbor Laboratory Press, Cold Spring Harbor, NY, 1989) at Chapt. 10, and US Pat. Nos. 5,658,737, 5,656,207, 5,547,842, 5,283,174, and 4,581,333, and EP Pat. App. 0 747 706). Particular methods of linking an AE compound to a nucleic acid are known (e.g., US Pat. No. 5,585,481 and US Pat. No. 5,639,604, see column 10, line 6 to column 11, line 3, and Example 8). Particular AE labeling positions are a probe's central region and near a region of A/T base pairs, at a probe's 3' or 5' terminus, or at or near a mismatch site with a known sequence that is the probe should not detect compared to the desired target sequence. Other detectably labeled probes include TaqMan^{™} probes, molecular torches, and molecular beacons. TaqMan^{™} probes include a donor and acceptor label wherein fluorescence is detected upon enzymatically degrading the probe during amplification in order to release the fluorophore from the presence of the quencher. Molecular torches and beacons exist in open and closed configurations wherein the closed configuration quenches the fluorophore and the open position separates the fluorophore from the quencher to allow fluorescence. Hybridization to target opens the otherwise closed probes.

A "non-extendable" oligonucleotide includes a blocking moiety at or near its 3'-terminus to prevent extension. A blocking group near the 3' end is in some embodiments within five residues of the 3' end and is sufficiently large to limit binding of a polymerase to the oligomer, and other embodiments contain a blocking group covalently attached to the 3' terminus. Many different chemical groups may be used to block the 3' end, e.g., alkyl groups, non-nucleotide linkers, alkane-diol dideoxynucleotide residues, and cordycepin. Further examples of blocking moieties include a 3'-deoxy nucleotide (e.g., a 2',3'-dideoxy nucleotide); a 3'-phosphorylated nucleotide; a fluorophore, quencher, or other label that interferes with extension; an inverted nucleotide (e.g., linked to the preceding nucleotide through a 3'-to-3' phosphodiester, optionally with an exposed 5'-OH or phosphate); or a protein or peptide joined to the oligonucleotide so as to prevent further extension of a nascent nucleic acid chain by a polymerase. A non-extendable oligonucleotide of the present disclosure may be at least 10 bases in length, and may be up to 15, 20, 25, 30, 35, 40, 50 or more nucleotides in length. Non-extendable oligonucleotides that comprise a detectable label can be used as probes.

References, particularly in the claims, to "the sequence of SEQ ID NO: X" refer to the base sequence of the corresponding sequence listing entry and do not require identity of the backbone (e.g., RNA, 2'-O-Me RNA, or DNA) or base modifications (e.g., methylation of cytosine residues) unless otherwise specifically indicated.

A "non-Watson Crick" (NWC) position in an oligonucleotide refers to a position where the oligonucleotide is configured to hybridize to at least one target sequence with a non-Watson Crick pairing, including through the use of inosine (e.g., via the inosine's hypoxanthine (I)). Such NWC pairing includes, for example, I-A, I-T, I-C, I-G, I-U, G-U, G-T, and G-A (any of the I/A/T/C/G/U can be the base in the oligonucleotide). In some embodiments, the NWC position is configured to hybridize via an I-A pair. In some embodiments, the NWC position is configured to hybridize via an I-T pair. In some embodiments, the NWC position is configured to hybridize via an I-C pair. In some embodiments, the NWC position is configured to hybridize via an I-G pair. In some embodiments, the NWC position is configured to hybridize via an I-U pair. In some embodiments, the NWC position is configured to hybridize via a wobble (G-U) or purine-purine (G-A) pair. In some embodiments, the NWC position is configured to hybridize via a G-T pair. In some embodiments, the NWC position is configured to hybridize via a G-U pair. In some embodiments, the NWC position is configured to hybridize via a G-A pair.

Unless defined otherwise, all scientific and technical terms used herein have the same meaning as commonly understood by those skilled in the relevant art. General definitions may be found in technical books relevant to the art of molecular biology, e.g., DICTIONARY OF MICROBIOLOGY AND MOLECULAR BIOLOGY, 2nd ed. (Singleton et al., 1994, John Wiley & Sons, New York, NY) or THE HARPER COLLINS DICTIONARY OF BIOLOGY (Hale & Marham, 1991, Harper Perennial, New York, NY).

### Exemplary compositions, kits, methods, and uses

The present disclosure provides oligomers, compositions, and kits, useful for determining the presence or absence of at least one enteric pathogen, such as *Salmonella*, *C. jejuni*, *C. coli*, *Shigella*, and STEC, in a sample.

In some embodiments, oligonucleotides are provided, e.g., in a kit or composition. Oligonucleotides generally comprise a target-hybridizing region, e.g., configured to hybridize specifically to a target nucleic acid of an enteric pathogen. While oligonucleotides of different lengths and base composition may be used for amplifying target nucleic acids, in some embodiments, oligonucleotides in this disclosure have target-hybridizing regions from about 10-60 bases in length, about 14-50 bases in length, about 14-40 bases in length, about 14-35 bases in length, or about 15-30 bases in length. In some embodiments, an oligonucleotide comprises a second region of sequence in addition to the target-hybridizing region, such as a promoter, which can be located 5' of the target-hybridizing region. In some embodiments, an oligonucleotide does not comprise a second region of sequence.

In some embodiments, a set of oligonucleotides is provided wherein one oligonucleotide is configured to hybridize to a sense strand of a target nucleic acid and the other oligonucleotide is configured to hybridize to an anti-sense strand of a target nucleic acid. Such oligonucleotides include amplification oligomer sets (e.g., primer pairs) for PCR or other forms of amplification.

In some embodiments, one or more oligonucleotides, such as a primer pair or a primer pair and a third oligonucleotide that is optionally labeled (e.g., for use as a probe), are configured to hybridize to a target nucleic acid of one or more enteric pathogens, including *Salmonella*, *C. jejuni*, *C. coli*, *Shigella*, and STEC. In some embodiments, a plurality of oligonucleotides, such as a plurality of primer pairs or a plurality of primer pairs and third oligonucleotides that are optionally labeled (e.g., for use as probes), are configured to collectively hybridize to one or more target nucleic acids of one or more enteric pathogens, including *Salmonella*, *C. jejuni*, *C. coli*, *Shigella*, or STEC.

In some embodiments, one or more oligonucleotides comprise a non-Watson Crick (NWC) position. In some embodiments, a *Salmonella* primer, a *Salmonella* primer pair, and/or a *Salmonella* probe comprises a NWC position, such as a position that includes inosine. In some embodiments, a *C. j juni* primer, a *C. j juni* primer pair, and/or a *C. jejuni* probe comprises a NWC position, such as a position that includes inosine. In some embodiments, a *C*. *coli* primer, a *C*. *coli* primer pair, and/or a *C*. *coli* probe comprises a NWC position, such as a position that includes inosine. In some embodiments, a *Shigella* primer, a *Shigella* primer pair, and/or a *Shigella* probe comprises a NWC position, such as a position that includes inosine. In some embodiments, a STEC primer, a STEC primer pair, and/or a STEC probe comprises a NWC position, such as a position that includes inosine.

In some embodiments, one or more oligonucleotides comprise a position comprising 5-methylcytosine. In some embodiments, a *Salmonella* primer, a *Salmonella* primer pair, and/or a *Salmonella* probe comprises a position comprising 5-methylcytosine. In some embodiments, a *C. jejuni* primer, a *C. jejuni* primer pair, and/or a *C. jejuni* probe comprises a position comprising 5-methylcytosine. In some embodiments, a *C*. *coli* primer, a *C*. *coli* primer pair, and/or a *C*. *coli* probe comprises a position comprising 5-methylcytosine. In some embodiments, a *Shigella* primer, a *Shigella* primer pair, and/or a *Shigella* probe comprises a position comprising 5-methylcytosine. In some embodiments, a STEC primer, a STEC primer pair, and/or a STEC probe comprises a position comprising 5-methylcytosine.

In some embodiments, the first and second oligomers of the *Salmonella-*specific amplification oligomer set respectively comprise target-hybridizing sequences comprising SEQ ID NO:12 and SEQ ID NO: 14. In some embodiments, the first and second oligomers of the *Salmonella-*specific amplification oligomer set respectively comprise target-hybridizing sequences comprising SEQ ID NO:21 and SEQ ID NO:47. In some embodiments, the first and second oligomers of the *Salmonella-*specific amplification oligomer set respectively comprise target-hybridizing sequences comprising SEQ ID NO:38 and SEQ ID NO:36. In some embodiments, the first and second oligomers of the *Salmonella-*specific amplification oligomer set respectively comprise target-hybridizing sequences comprising SEQ ID NO:35 and SEQ ID NO:40. In some embodiments, the first and second oligomers of the *Salmonella-*specific amplification oligomer set respectively comprise target-hybridizing sequences comprising SEQ ID NO:12 and SEQ ID NO:28. In some embodiments, the first and second oligomers of the *Salmonella-*specific amplification oligomer set respectively comprise target-hybridizing sequences comprising SEQ ID NO:42 and SEQ ID NO:31. In some embodiments, the first and second oligomers of the *Salmonella-*specific amplification oligomer set respectively comprise target-hybridizing sequences comprising SEQ ID NO:41 and SEQ ID NO:27.

In some embodiments, the first and second oligomers of the *C. jejuni*-specific amplification oligomer set respectively comprise target-hybridizing sequences comprising SEQ ID NO:11 and SEQ ID NO:19.

In some embodiments, the first and second oligomers of the *C. jejuni*-specific amplification oligomer set respectively consist of target-hybridizing sequences comprising SEQ ID NO:11 and SEQ ID NO:19.

In some embodiments, the first and second oligomers of the *C*. *coli-*specific amplification oligomer set respectively comprise target-hybridizing sequences comprising SEQ ID NO:8 and SEQ ID NO:10.

In some embodiments, the first and second oligomers of the *C*. *coli-*specific amplification oligomer set respectively consist of target-hybridizing sequences comprising SEQ ID NO:8 and SEQ ID NO:10.

In some embodiments, the first and second oligomers of the *Shigella-*specific amplification oligomer set respectively comprise target-hybridizing sequences comprising SEQ ID NO:15 and SEQ ID NO:17. In some embodiments, the first and second oligomers of the *Shigella-*specific amplification oligomer set respectively comprise target-hybridizing sequences comprising SEQ ID NO:34 and SEQ ID NO:22. In some embodiments, the first and second oligomers of the *Shigella*-specific amplification oligomer set respectively comprise target-hybridizing sequences comprising SEQ ID NO:32 and SEQ ID NO:33.

In some embodiments, the first and second oligomers of the *Shigella*-specific amplification oligomer set respectively consist of target-hybridizing sequences comprising SEQ ID NO:15 and SEQ ID NO:17. In some embodiments, the first and second oligomers of the *Shigella-*specific amplification oligomer set respectively consist of target-hybridizing sequences comprising SEQ ID NO:34 and SEQ ID NO:22. In some embodiments, the first and second oligomers of the *Shigella*-specific amplification oligomer set respectively consist of target-hybridizing sequences comprising SEQ ID NO:32 and SEQ ID NO:33.

In some embodiments, the first and second oligomers of the STEC-specific amplification oligomer set respectively comprise target-hybridizing sequences comprising SEQ ID NO:20 and SEQ ID NO:3. In some embodiments, the first and second oligomers of the STEC-specific amplification oligomer set respectively comprise target-hybridizing sequences comprising SEQ ID NO:49 and SEQ ID NO:3. In some embodiments, the first and second oligomers of the STEC-specific amplification oligomer set respectively comprise target-hybridizing sequences comprising SEQ ID NO:4 and SEQ ID NO:7.

In some embodiments, the first and second oligomers of the STEC-specific amplification oligomer set respectively consist of target-hybridizing sequences comprising SEQ ID NO:20 and SEQ ID NO:3. In some embodiments, the first and second oligomers of the STEC-specific amplification oligomer set respectively consist of target-hybridizing sequences comprising SEQ ID NO:49 and SEQ ID NO:3. In some embodiments, the first and second oligomers of the STEC-specific amplification oligomer set respectively consist of target-hybridizing sequences comprising SEQ ID NO:4 and SEQ ID NO:7.

Exemplary primer pairs and optional third oligomers (e.g., probe) are set forth in the following **Table A.**

**Table A. Exemplary oligonucleotide sets. Oligonucleotides are referred to by their SEQ ID NO (see the Sequence Table below).**

| **Oligonucleotide 1 (e.g., forward primer)** | **Oligonucleotide 2 (e.g., reverse primer)** | **Oligonucleotide 3 (e.g., probe; optionally labeled)** |
|---|---|---|
| ***Salmonella*** | | |
| 12 | 14 | 13 |
| 21 | 47 | 45 |
| 38 | 36 | 44, 26, or 25 |
| 35 | 40 | 30 |
| 12 | 28 | 13 |
| 42 | 31 | 23 |
| 41 | 27 | 43 |

| ***C. jejuni*** | | |
|---|---|---|
| 11 | 19 | 18 |

| ***C. coli*** | | |
|---|---|---|
| 8 | 10 | 9, 37, or 39 |

| ***Shigella*** | | |
|---|---|---|
| 15 | 17 | 16 |
| 34 | 22 | 46 or 29 |
| 32 | 33 | 24 |

| **STEC** | | |
|---|---|---|
| 20 | 3 | 1, 2, or 48 |
| 49 | 3 | 1, 2, or 48 |
| 4 | 7 | 5 or 6 |

In some embodiments, an oligonucleotide is provided that comprises a label. Such an oligonucleotide can be used as a probe. In some embodiments, the labeled oligonucleotide has a sequence corresponding to a SEQ ID NO listed in the Oligomer 3 column of **Table A.** In some embodiments, the label is a non-nucleotide label. Suitable labels include compounds that emit a detectable light signal, e.g., fluorophores or luminescent (e.g., chemiluminescent) compounds that can be detected in a homogeneous mixture. More than one label, and more than one type of label, may be present on a particular probe, or detection may rely on using a mixture of probes, in which each probe is labeled with a compound that produces a detectable signal (*see. e.g.*, US Pat. Nos. 6,180,340 and 6,350,579, each incorporated by reference herein). Labels may be attached to a probe by various means including covalent linkages, chelation, and ionic interactions, but in some embodiments the label is covalently attached. For example, in some embodiments, a detection probe has an attached chemiluminescent label such as, e.g., an acridinium ester (AE) compound (*see, e.g.*, US Pat. Nos. 5,185,439; 5,639,604; 5,585,481; and 5,656,744). A label, such as a fluorescent or chemiluminescent label, can be attached to the probe by a non-nucleotide linker (c Pat. Nos. 5,585,481; 5,656,744; and 5,639,604). In some embodiments, the label may include one or more of Quasar670, CalRed610, CalOrange560, fluorescein, ROX, FAM, and HEX.

In some embodiments, a probe (e.g., comprising a fluorescent label) further comprises a second label that interacts with the first label. For example, the second label can be a quencher. In some embodiments, the second label may include one or both of BHQ-1 and BHQ-2.Such probes can be used, e.g., in TaqMan^{™} assays, where hybridization of the probe to a target or amplicon followed by nucleolysis by a polymerase comprising 5'-3' exonuclease activity results in liberation of the fluorescent label and thereby increased fluorescence, or fluorescence independent of the interaction with the second label.

In some applications, one or more probes exhibiting at least some degree of self-complementarity are used to facilitate detection of probe:target duplexes in a test sample without first requiring the removal of unhybridized probe prior to detection. Specific embodiments of such detection probes include, for example, probes that form conformations held by intramolecular hybridization, such as conformations generally referred to as hairpins. Suitable hairpin probes include a "molecular torch" (*see, e.g.*, US Pat. Nos. 6,849,412; 6,835,542; 6,534,274; and 6,361,945) and a "molecular beacon" (*see, e.g.*, US Pat. No. 5,118,801 and U.S. Pat. No. 5,312,728). Molecular torches include distinct regions of self-complementarity (coined "the target binding domain" and "the target closing domain") which are connected by a joining region (e.g., a -(CH₂CH₂O)₃- linker) and which hybridize to one another under predetermined hybridization assay conditions. When exposed to an appropriate target or denaturing conditions, the two complementary regions (which may be fully or partially complementary) of the molecular torch melt, leaving the target binding domain available for hybridization to a target sequence when the predetermined hybridization assay conditions are restored. Molecular torches are designed so that the target binding domain favors hybridization to the target sequence over the target closing domain. The target binding domain and the target closing domain of a molecular torch include interacting labels (e.g., fluorescent/ quencher) positioned so that a different signal is produced when the molecular torch is self-hybridized as opposed to when the molecular torch is hybridized to a target nucleic acid, thereby permitting detection of probe:target duplexes in a test sample in the presence of unhybridized probe having a viable label associated therewith.

Examples of interacting donor/acceptor label pairs that may be used in connection with the disclosure, making no attempt to distinguish FRET from non-FRET pairs, include fluorescein/tetramethylrhodamine, IAEDANS / fluororescein, EDANS/DABCYL, coumarin/DABCYL, fluorescein/fluorescein, BODIPY FL/BODIPY FL, fluorescein/DABCYL, lucifer yellow/DABCYL, BODIPY/DABCYL, eosine/DABCYL, erythrosine/DABCYL, tetramethylrhodamine/DABCYL, Texas Red/DABCYL, CY5/BHQ-1, CY5/BHQ-2, CY3/BHQ-1, CY3/BHQ-2 and fluorescein/QSY7 dye. Those having an ordinary level of skill in the art will understand that when donor and acceptor dyes are different, energy transfer can be detected by the appearance of sensitized fluorescence of the acceptor or by quenching of donor fluorescence. Non-fluorescent acceptors such as DABCYL and the QSY7 dyes advantageously eliminate the potential problem of background fluorescence resulting from direct (i.e., non-sensitized) acceptor excitation. Exemplary fluorophore moieties that can be used as one member of a donor-acceptor pair include fluorescein, ROX, and the CY dyes (such as CY5). Exemplary quencher moieties that can be used as another member of a donor-acceptor pair include DABCYL and the Black Hole Quencher moieties which are available from Biosearch Technologies, Inc., (Novato, Calif.).

In some embodiments, a labeled oligonucleotide (e.g., probe) is non-extendable. For example, the labeled oligomer can be rendered non-extendable by 3'-phosphorylation, having a 3'-terminal 3'-deoxynucleotide (e.g., a terminal 2',3'-dideoxynucleotide), having a 3'-terminal inverted nucleotide (e.g., in which the last nucleotide is inverted such that it is joined to the penultimate nucleotide by a 3' to 3' phosphodiester linkage or analog thereof, such as a phosphorothioate), or having an attached fluorophore, quencher, or other label that interferes with extension (possibly but not necessarily attached via the 3' position of the terminal nucleotide). In some embodiments, the 3'-terminal nucleotide is not methylated.

Also provided by the disclosure is a reaction mixture for determining the presence or absence of a target nucleic acid of at least one enteric pathogen, such as *Salmonella, C. jejuni*, *C. coli*, *Shigella*, and STEC, or quantifying the amount thereof in a sample. A reaction mixture in accordance with the present disclosure comprises at least one or more of the following: an oligonucleotide as described herein for amplification of a target nucleic acid; and an oligonucleotide (e.g., probe) as described herein for determining the presence or absence of an amplification product of the target nucleic acid. For a reaction mixture that includes a detection probe together with an amplification oligonucleotide combination, the amplification oligonucleotides and detection probe oligonucleotides for a reaction mixture are linked by a common target region (i.e., the reaction mixture will include a probe that binds to a sequence amplifiable by an amplification oligonucleotides combination of the reaction mixture).

The reaction mixture may further include a number of optional components such as, for example, capture probes, e.g., poly-(k) capture probes as described in US 2013/0209992, which is incorporated herein by reference, and/or poly-(R) capture probes as described in US 2020/0165599, which is incorporated herein by reference. For an amplification reaction mixture, the reaction mixture will typically include other reagents suitable for performing *in vitro* amplification such as, e.g., buffers, salt solutions, appropriate nucleotide triphosphates (e.g., dATP, dCTP, dGTP, and dTTP; and/or ATP, CTP, GTP and UTP), and/or enzymes (e.g., a thermostable DNA polymerase, or reverse transcriptase and/or RNA polymerase), and will typically include test sample components, in which a target nucleic acid may or may not be present. Suitable reagents include, for example, formulations containing lithium lauryl sulfate (LLS), sodium lauryl sulfate (SLS), NaH₂PO₄, Na₂HPO₄, EDTA, EGTA, LiOH, NaCl, KCl, MgCl₂, NaOH, ethanol, methylparaben, propylparaben, trehalose, Tris Buffer, Triton X-100, paramagnetic particles, target capture oligonucleotides, HEPES, succinic acid, polymerases (e.g., DNA polymerases, reverse transcriptases), and/or RNasin.

In some embodiments, the reaction mixture comprises KCl. In some embodiments, the KCl concentration is about 50 mM. In some embodiments, the KCl concentration is greater than about 50 mM, e.g., about 60-150 mM, about 75-125 mM, about 80-120 mM, about 85-115 mM, or about 90-110 mM. In some embodiments, the KCl concentration is 55-65, 65-75, 75-85, 85-95, 95-105, 105-115, 115-125, 125-135, or 135-145, wherein each of the foregoing is in mM and is optionally modified by "about". In some embodiments, a composition according to the disclosure comprises KCl, e.g., at any of the foregoing concentrations. In some embodiments, a method according to the disclosure comprises performing an amplification reaction in the presence of KCl, e.g., at any of the foregoing concentrations.

In some embodiments, the reaction mixture comprises α-cyclodextrin and/or polysorbate 20. In some embodiments, the concentration of α-cyclodextrin in the reaction mixture is from about 10 mg/mL to about 40 mg/mL. In some embodiments, the concentration of α-cyclodextrin in the reaction mixture is from about 16 mg/mL to about 30 mg/mL, from about 15 mg/mL to about 20 mg/mL, or from about 10 mg/mL to about 15 mg/mL. In some embodiments, the concentration of α-cyclodextrin in the reaction mixture is about 20 mg/mL, about 17.5 mg/mL, or about 12.5 mg/mL. In some embodiments, the concentration of polysorbate 20 in the reaction mixture is from about 0.002% to about 0.05% (v/v). In some embodiments, the concentration of polysorbate 20 in the reaction mixture is from about 0.003% to about 0.03% (v/v). In some embodiments, the concentration of polysorbate 20 in the reaction mixture is about 0.0042% (v/v), about 0.0035% (v/v), about 0.0026% (v/v), or about 0.02% (v/v). In some embodiments, the reaction mixture comprises a detergent. In some embodiments, the detergent comprises sodium dodecyl sulfate. In some embodiments, the concentration of the detergent in the reaction mixture is from about 3 mg/mL to 300 mg/mL. In some embodiments, the concentration of the detergent in the reaction mixture is from about 10 mg/mL to 100 mg/mL. In some embodiments, the concentration of the detergent in the reaction mixture is about 33.3 mg/mL.

Also provided by the subject disclosure are kits for practicing the methods as described herein. A kit in accordance with the present disclosure comprises at least one or more of the following: an oligonucleotide as described herein for amplification of a target nucleic acid; and an oligonucleotide (e.g., probe) as described herein for determining the presence or absence of an amplification product of the target nucleic acid. In some embodiments, any oligonucleotide combination described herein is present in the kit. The kits may further include a number of optional components such as, for example, capture probes, e.g., poly-(k) capture probes as described in US 2013/0209992 and/or poly-(R) capture probes as described in US 2020/0165599. Other reagents that may be present in the kits include reagents suitable for performing *in vitro* amplification such as, e.g., buffers, salt solutions, appropriate nucleotide triphosphates (e.g., dATP, dCTP, dGTP, dTTP; and/or ATP, CTP, GTP and UTP), and/or enzymes (e.g., a thermostable DNA polymerase, or a reverse transcriptase and/or RNA polymerase). Oligonucleotides as described herein may be packaged in a variety of different embodiments, and those skilled in the art will appreciate that the disclosure embraces many different kit configurations. For example, a kit may include amplification oligonucleotides for only one, two, three, four, or all of *Salmonella, C. jejuni*, *C. coli*, *Shigella*, and STEC. In addition, for a kit that includes a detection probe together with an amplification oligomer combination, the amplification oligonucleotides and detection probe oligonucleotides for a reaction mixture are linked by a common target region (i.e., the reaction mixture will include a probe that binds to a sequence amplifiable by an amplification oligonucleotides combination of the reaction mixture). In certain embodiments, the kit further includes a set of instructions for practicing methods in accordance with the present disclosure, where the instructions may be associated with a package insert and/or the packaging of the kit or the components thereof.

Also provided by the subject disclosure are methods (e.g., multiplex methods) for determining the presence or absence of at least one enteric pathogen, including *Salmonella, C. jejuni*, *C. coli*, *Shigella*, and STEC, in a sample by, for example, using one or more of the oligonucleotides disclosed herein. Any method disclosed herein is also to be understood as a disclosure of corresponding uses of materials involved in the method directed to the purpose of the method. Any of the oligonucleotides and any combinations (e.g., kits and compositions) comprising such an oligonucleotide are to be understood as also disclosed for use in detecting or quantifying at least one enteric pathogen, and for use in the preparation of a composition for detecting or quantifying at least one enteric pathogen.

Broadly speaking, methods can comprise one or more of the following components: target capture, in which a target nucleic acid (e.g., from a sample, such as a clinical sample) is annealed to a capture oligonucleotide; isolation, e.g., washing, to remove material not associated with a capture oligonucleotide; amplification; and amplicon detection, e.g., amplicon quantification, which may be performed in real time with amplification. Certain embodiments involve each of the foregoing steps. Certain embodiments involve exponential amplification, optionally with a preceding linear amplification step. Certain embodiments involve exponential amplification and amplicon detection. Certain embodiments involve any two of the components listed above. Certain embodiments involve any two components listed adjacently above, e.g., washing and amplification, or amplification and detection.

In some embodiments, amplification comprises (1) contacting the sample with at least two oligonucleotides for amplifying a target nucleic acid target region corresponding to a target nucleic acid, where the oligonucleotides include at least two amplification oligonucleotides as described above (e.g., one or more oriented in the sense direction and one or more oriented in the antisense direction for exponential amplification);
(2) performing an *in vitro* nucleic acid amplification reaction, where any target nucleic acid present in the sample is used as a template for generating an amplification product; and
(3) detecting the presence or absence of the amplification product, thereby determining the presence or absence of at least one enteric pathogen, including *Salmonella*, *C. jejuni*, *C. coli*, *Shigella,* and STEC, in a sample, or quantifying the amount of the target nucleic acid in the sample.

A detection method in accordance with the present disclosure can further include the step of obtaining the sample to be subjected to subsequent steps of the method. In certain embodiments, "obtaining" a sample to be used includes, for example, receiving the sample at a testing facility or other location where one or more steps of the method are performed, and/or retrieving the sample from a location (e.g., from storage or other depository) within a facility where one or more steps of the method are performed.

In certain embodiments, the method further includes purifying the target nucleic acid from other components in the sample, e.g., before an amplification, such as before a capture step. Such purification may include methods of separating and/or concentrating organisms contained in a sample from other sample components, or removing or degrading non-nucleic acid sample components, e.g., protein, carbohydrate, salt, lipid, etc. In some embodiments, nucleic acid in the sample is degraded, e.g., with DNase, and optionally removing or inactivating the DNase or removing degraded nucleic acid.

In particular embodiments, purifying the target nucleic acid includes capturing the target nucleic acid to specifically or non-specifically separate the target nucleic acid from other sample components. Non-specific target capture methods may involve selective precipitation of nucleic acids from a substantially aqueous mixture, adherence of nucleic acids to a support that is washed to remove other sample components, or other means of physically separating nucleic acids from a mixture that contains the target nucleic acid and other sample components.

Target capture typically occurs in a solution phase mixture that contains one or more capture probe oligonucleotide that hybridize to the target nucleic acid sequence under hybridizing conditions. For embodiments comprising a capture probe tail, the target:capture-probe complex is captured by adjusting the hybridization conditions so that the capture probe tail hybridizes to the immobilized probe. Certain embodiments use a particulate solid support, such as paramagnetic beads.

Isolation can follow capture, wherein the complex on the solid support is separated from other sample components. Isolation can be accomplished by any apporpiate technique, e.g., washing a support associated with the target-sequence one or more times (e.g., 2 or 3 times) to remove other sample components and/or unbound oligomer. In embodiments using a particulate solid support, such as paramagnetic beads, particles associated with the target may be suspended in a washing solution and retrieved from the washing solution, In some embodiments by using magnetic attraction. To limit the number of handling steps, the target nucleic acid may be amplified by simply mixing the target sequence in the complex on the support with amplification oligomers and proceeding with amplification steps.

Exponentially amplifying a target sequence utilizes an *in vitro* amplification reaction using at least two amplification oligonucleotides that flank a target region to be amplified. In some embodiments, at least one oligonucleotide as described above is provided. In some embodiments, a plurality of pairs of oligonucleotides is provided, wherein the plurality comprises oligonucleotides pairs configured to hybridize to at least one, two, three, four, or all of *Salmonella, C. jejuni*, *C. coli, Shigella,* and STEC target nucleic acids. The amplification reaction can be cycled or isothermal. Suitable amplification methods include, for example, replicase-mediated amplification, polymerase chain reaction (PCR), ligase chain reaction (LCR), strand-displacement amplification (SDA), and transcription-mediated or transcription-associated amplification (TMA).

A detection step may be performed using any of a variety of known techniques to detect a signal specifically associated with the amplified target sequence, such as, e.g., by hybridizing the amplification product with a labeled detection probe and detecting a signal resulting from the labeled probe (including from label released from the probe following hybridization in some embodiments), performing electrophoresis on the sample and/or the amplification product, determining the sequence of the amplification product. In some embodiments, the labeled probe comprises a second moiety, such as a quencher or other moiety that interacts with the first label, as discussed above. The detection step may also provide additional information on the amplified sequence, such as, e.g., all or a portion of its nucleic acid base sequence. Detection may be performed after the amplification reaction is completed, or may be performed simultaneously with amplifying the target region, e.g., in real time. In one embodiment, the detection step allows homogeneous detection, e.g., detection of the hybridized probe without removal of unhybridized probe from the mixture (*see, e.g.*, US Pat. Nos. 5,639,604 and 5,283,174). In some embodiments, the nucleic acids are associated with a surface that results in a physical change, such as a detectable electrical change. Amplified nucleic acids may be detected by concentrating them in or on a matrix and detecting the nucleic acids or dyes associated with them (e.g., an intercalating agent such as ethidium bromide or cyber green), or detecting an increase in dye associated with nucleic acid in solution phase. Other methods of detection may use nucleic acid detection probes that are configured to specifically hybridize to a sequence in the amplified product and detecting the presence of the probe:product complex, or by using a complex of probes that may amplify the detectable signal associated with the amplified products (*see, e.g.*, US Pat. Nos. 5,424,413; 5,451,503; and 5,849,481; each incorporated by reference herein). Directly or indirectly labeled probes that specifically associate with the amplified product provide a detectable signal that indicates the presence of the target nucleic acid in the sample. In particular, the amplified product will contain a target sequence in or complementary to a target nucleic sequence of at least one enteric pathogen, and a probe will bind directly or indirectly to a sequence contained in the amplified product to indicate the presence or absence of the pathogen in the tested sample.

In embodiments that detect the amplified product near or at the end of the amplification step, a linear detection probe may be used to provide a signal to indicate hybridization of the probe to the amplified product. One example of such detection uses a luminescentally labeled probe that hybridizes to target nucleic acid. Luminescent label is then hydrolyzed from non-hybridized probe. Detection is performed by chemiluminescence using a luminometer (*see, e.g.*, PCT Pub. No. WO 89/002476). In other embodiments that use real-time detection, the detection probe may be a hairpin probe such as, for example, a molecular beacon, molecular torch, or hybridization switch probe that is labeled with a reporter moiety that is detected when the probe binds to amplified product. Such probes may comprise target-hybridizing sequences and non-target-hybridizing sequences. Various forms of such probes are described, e.g., in U.S. Pat. Nos. 5,118,801; 5,312,728; 5,925,517; 6,150,097; 6,849,412; 6,835,542; 6,534,274; and 6,361,945; and US Pub. Nos. 2006/0068417A1 and 2006/0194240A1).

In some embodiments, in an amplication reaction, an amplification oligonucleotide may preferentially hybridize with a target nucleic acid, such as a target sequence of a pathogen. In some embodiments, in a detection step, a probe may preferentially hybridize with a target nucleic acid, such as an amplification product. For example, under stringent hybridization conditions, an amplification or detection probe oligonucleotide can hybridize to its target nucleic acid to form stable oligonucleotide:target hybrids, but not form a sufficient number of stable oligonucleotide:non-target hybrids. In some embodiments, there is at least a 5-fold difference between target and non-target hybridization signals (i.e., the ratio of signal strengths of oligonucleotide:target hybrids and of oligonucleotide:non-target hybrids) in a test sample, at least a 10-fold difference, at least a 20-fold difference, at least a 50-fold difference, at least a 75-fold difference, at least a 100-fold difference, at least a 200-fold difference, at least a 500-fold difference, at least a 1,000-fold difference, or at least a 2,000-fold difference. In some embodiments, non-target hybridization signals in a test sample are no more than the background signal level.

In some embodiments, an amplification reaction and/or a detection step may be performed under stringent hybridization conditions. In some embodiments, such stringent hybridization conditions permit an oligonucleotide (e.g., an amplification oligonucleotide or probe) to preferentially hybridize to a target nucleic acid (such as a target sequence of a pathogen or an amplification product) and not to nucleic acid derived from a closely related non-target nucleic acid. In different embodiments, the actual reaction environment that can be used for stringent hybridization may vary depending upon factors including the GC content and length of the oligonucleotide, the degree of similarity between the oligonucleotide sequence and sequences of non-target nucleic acids that may be present in the test sample, and the target sequence. In some embodiments, hybridization conditions for an amplification reaction and/or a detection step correspond to a temperature of about 55 °C, about 56 °C, about 57 °C, about 58 °C, about 59 °C, about 60 °C, about 61 °C, about 62 °C, about 63 °C, about 64 °C, or about 65 °C when the salt concentration, such as a monovalent salt, e.g., KCl, is about 0.5 M, about 0.6 M, about 0.7 M, about 0.8 M, about 0.9 M, or about 1.0 M.

The methods (e.g., multiplex methods) for determining the presence or absence of at least one enteric pathogen, including *Salmonella, C. jejuni*, *C. coli, Shigella,* and STEC, in a sample by, for example, using one or more of the oligonucleotides disclosed herein may have a detection sensitivity from 25 to 500 CFU/mL, from 50 to 500 CFU/mL, from 75 to 500 CFU/mL, from 100 to 500 CFU/mL, from 25 to 300 CFU/mL, from 50 to 300 CFU/mL, from 75 to 300 CFU/mL, from 100 to 300 CFU/mL, from 25 to 150 CFU/mL, from 50 to 150 CFU/mL, from 75 to 150 CFU/mL, from 100 to 150 CFU/mL, from 25 to 100 CFU/mL, from 50 to 100 CFU/mL, from 75 to 100 CFU/mL, from 25 to 75 CFU/mL, from 50 to 75 CFU/mL, or from 25 to 50 CFU/mL.

Also provided by the subject disclosure are methods for synthesizing one or more (e.g., one or more pairs) of the oligonucleotides disclosed herein, the oligonucleotides useful for determining the presence or absence of at least one enteric pathogen, including *Salmonella*, *C. jejuni*, *C. coli*, *Shigella*, and STEC. The method may, for example, include the steps of (a) obtaining a solid support comprising at least one nucleobase residue, wherein the at least one nucleobase residue is bound (e.g., covalently bound) at a 3' position to the solid support; (b) coupling a 5' position of the nucleobase residue furtherest from the solid support to a 3' position of another nucleobase residue; (c) repeating step (b) at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, or at least 28 additional times, thereby generating at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, or at least 30 contiguous nucleobase residues coupled to the solid support; and (d) cleaving the at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, or at least 30 contiguous nucleobase residues generated in step (c), thereby obtaining the oligonucleotide or oligonucleotides. In some embodiments, the oligonucleotide has a length of from 18 to 32 contiguous nucleobase residues. In some embodiments, the oligonucleotide has a length of from 20 to 30 contiguous nucleobase residues.

A method for synthesizing one or more of the oligonucleotides disclosed herein may be a solid phase method. For example, phosphoramidite solid-phase chemistry for joining nucleotides by phosphodiester linkages is disclosed in Caruthers et al., "Chemical Synthesis of Deoxynucleotides by the Phosphoramidite Method," Methods Enzymol. 154:287 (1987). As another example, automated solid-phase chemical synthesis using cyanoethyl phosphoramidite precursors has been described in Barone et al., "In Situ Activation of bis-dialkylaminephosphines - a New Method for Synthesizing Deoxyoligonucleotides on Polymer Supports," Nucleic Acids Res. 12(10):4051 (1984). As another example, US Patent No. 5,449,769, entitled "Method and Reagent for Sulfurization of Organophosphorous Compounds" discloses a procedure for synthesizing oligonucleotides containing phosphorothioate linkages. In addition, US Patent No. 5,811,538, entitled "Process for the Purification of Oligomers" discloses the synthesis of oligonucleotides having different linkages, including methylphosphonate linkages. Moreover, methods for the organic synthesis of oligonucleotides are described in, for example, Sambrook et al., Molecular Cloning, A Laboratory Manual, 2nd ed. (Cold Spring Harbor Laboratory Press, Cold Spring Harbor, NY, 1989) at Chapt. 10.

Following synthesis and purification of a particular oligonucleotide, several different procedures may be utilized to purify and control the quality of the oligonucleotide. Suitable procedures include electrophoresis (e.g., polyacrylamide gel electrophoresis) or chromatography (e.g, high pressure liquid chromatography).

### EXAMPLES

The following examples are provided to illustrate certain disclosed embodiments and are not to be construed as limiting the scope of this disclosure in any way.

### Example 1 - Real-Time PCR Amplification and Detection of GI-Bacterial Panel Targets Using Different Combinations of Primers and Probes

Several primer and probe combinations for real-time PCR amplification and detection of *Salmonella*, *Campylobacter*, *Shigella*/Enteroinvasive *E*. *coli* (*Shigella*/EIEC), and Shigatoxigenic *E*. *coli* (STEC) targets were tested.

Amplification and detection reactions were performed using a Panther Fusion instrument (Hologic, Inc. San Diego, CA). Typically, 20 µL of an amplification reagent was combined in a reaction well of a multi-well plate with 5 µL of a target nucleic acid. The multi-well plate was placed in the Panther Fusion instrument and subjected to thermal cycling. Real-time amplification and detection reactions were performed by thermal cycling, generally for 45 cycles (denaturation at 95 °C for 8 seconds and annealing and extension at 60 °C for 25 seconds), taking fluorescent emission readings every 30 seconds. Fluorescence curve profiles for the target nucleic acids were evaluated for Ct and RFU signals.

### Example 2 - Analytical Sensitivity-Bacterial Limit of Detection

Analytical sensitivity experiments were performed for determining the limit of detection (LoD) for target nucleic acids of six individual bacterial organisms, using a multiplex primer-probe combination. The primers and probes used in the experiment are shown in **Table 1.** Known stock concentrations of each organism (available from American Type Tissue Culture, Manassas, VA) were serially diluted in a raw stool matrix to provide a series of 5 dilution concentrations for each organism. *Salmonella typhimurium*, *Campylobacter coli*, *Campylobacter jejuni*, *Shigella sonnei*, Shiga Toxin-producing *Escherichia coli* O157:H7 (STEC stxl and STEC stx2) were each serially diluted to provide the following concentrations: 1,000 CFU/mL, 500 CFU/mL, 300 CFU/mL, 150 CFU/mL, and 100 CFU/mL. *Campylobacter jejuni* was serially diluted to provide the following concentrations: 1,000 CFU/mL, 500 CFU/mL, 300 CFU/mL, 100 CFU/mL, and 75 CFU/mL. Each of the dilutions was tested in replicates of 5, and Ct and RFU data are presented in **Table 2.** The lowest concentration that was 100% positive for each organism was then further tested in replicates of 20 (dilutions made in both raw stool matrix and Cary-Blair stool matrix), and the Ct and RFU data are presented in **Table 3.** Sample processing, target capture, amplification and detection reactions were generally performed as is described herein. An internal control nucleic acid was present in each amplification reaction.

**Table 1. Primer-Probe Combinations**

| Oligo Type | SEQ ID NO: | Modifications* |
|---|---|---|
| Primers | SEQ ID NO:3 | |
| | SEQ ID NO:4 | |
| | SEQ ID NO:7 | 5mC at residues 3, 4, 18, and 19 |
| | SEQ ID NO:8 | |
| | SEQ ID NO:10 | |
| | SEQ ID NO:11 | |
| | SEQ ID NO:12 | 5mC at residues 4, 11, 12, and 16 |
| | SEQ ID NO:14 | |
| | SEQ ID NO:15 | Inosine at residue 3 |
| | | 5mC at residues 7 and 15 |
| | SEQ ID NO:17 | |
| | SEQ ID NO:19 | Inosine at residue 23 |
| | | 5mC at residues 1, 2, 7, 14, 15, 19, and 20 |
| | SEQ ID NO:20 | |
| Probes | SEQ ID NO:1 | 5mC at residues 6, 7, 9, 14, 17, and 24 |
| | | Quasar670/BHQ2 |
| | SEQ ID NO:2 | 5mC at residues 5, 8, 11, 13, 18, and 21 |
| | | Quasar670/BHQ2 |
| | SEQ ID NO:5 | Quasar 670/BHQ2 |
| | SEQ ID NO:6 | 5mC at residues 3, 7, 8, 15, 20, 24, and 27 |
| | | Quasar670/BHQ2 |
| | SEQ ID NO:9 | 5mC at residues 2, 6, 13, 16, 23, 26, 28, and 29 |
| | | BHQ1/HEX |
| | SEQ ID NO:13 | BHQ1/FAM |
| | SEQ ID NO:16 | Inosine at residue 16 |
| | | CalRed610/BHQ2 |
| | SEQ ID NO:18 | 5mC at residues 7 and 12 |
| | | HEX/BHQ1 |

| | | |
|---|---|---|
| * 5mC refers to a residue that comprises 5-methylcytosine. BHQ refers to Black Hole Quencher. FAM refers to fluorescein. | | |

**Table 2. Initial LoD Testing Results**

| Target | Conc (CFU/mL) | Reactivity | Avg Ct | Avg Total RFU | Avg Background RFU | Signal to Noise |
|---|---|---|---|---|---|---|
| *Salmonella* | 100 | 5/5 | 36.96 | 15,519 | 8,556 | 1.8 |
| | 150 | 5/5 | 35.49 | 23,955 | 9,263 | 2.6 |
| | 300 | 5/5 | 34.71 | 26,305 | 9,069 | 2.9 |
| | 500 | 5/5 | 33.81 | 30,129 | 8,665 | 3.5 |
| | 1,000 | 5/5 | 32.94 | 35,666 | 9,664 | 3.7 |
| *C. jejuni* | 75 | 5/5 | 36.90 | 15,384 | 10,446 | 1.5 |
| | 100 | 5/5 | 36.68 | 14,652 | 9,504 | 1.5 |
| | 300 | 5/5 | 35.15 | 18,835 | 10,983 | 1.7 |
| | 500 | 5/5 | 34.43 | 19,223 | 10,703 | 1.8 |
| | 1,000 | 5/5 | 33.29 | 19,860 | 10,196 | 1.9 |
| *C. coli* | 100 | 4/5 | 40.36 | 12,455 | 10,017 | 1.2 |
| | 150 | 4/5 | 39.78 | 12,906 | 10,044 | 1.3 |
| | 300 | 5/5 | 38.36 | 15,528 | 11,111 | 1.4 |
| | 500 | 5/5 | 37.65 | 15,346 | 10,146 | 1.5 |
| | 1,000 | 5/5 | 36.17 | 18,549 | 10,785 | 1.7 |
| *Shigella* | 100 | 5/5 | 35.79 | 4,915 | 770 | 6.4 |
| | 150 | 5/5 | 35.06 | 5,604 | 764 | 7.3 |
| | 300 | 5/5 | 34.34 | 6,791 | 782 | 8.7 |
| | 500 | 5/5 | 33.49 | 7,568 | 771 | 9.8 |
| | 1,000 | 5/5 | 32.58 | 8,775 | 820 | 10.7 |
| STEC-*Stx1* | 100 | 5/5 | 38.31 | 6,971 | 4,952 | 1.4 |
| | 150 | 5/5 | 38.82 | 6,287 | 4,380 | 1.4 |
| | 300 | 5/5 | 36.77 | 7,905 | 4,696 | 1.7 |
| | 500 | 5/5 | 35.82 | 8,906 | 4,718 | 1.9 |
| | 1,000 | 5/5 | 35.00 | 8,199 | 4,150 | 2.0 |
| STEC-*Stx2* | 100 | 5/5 | 38.69 | 6,202 | 4,331 | 1.4 |
| | 150 | 5/5 | 38.39 | 6,590 | 4,446 | 1.5 |
| | 300 | 5/5 | 36.94 | 7,152 | 4,351 | 1.6 |
| | 500 | 5/5 | 36.00 | 7,503 | 4,273 | 1.8 |
| | 1,000 | 5/5 | 34.79 | 8,400 | 4,528 | 1.9 |

**Table 3. Confirmatory LoD Testing Results**

| Target | Conc (CFU/mL) | Stool Matrix | Reactivity | Avg Ct | Avg Total RFU | Avg Background RFU | Signal-to-Noise |
|---|---|---|---|---|---|---|---|
| *Salmonella* | 100 | Raw | 18/20 | 36.93 | 16,100 | 8,410 | 1.9 |
| | | Cary-Blair | 19/20 | 36.12 | 18,638 | 8,839 | 2.1 |
| | 150 | Raw | 19/20 | 36.64 | 13,089 | 9,338 | 1.4 |
| | | Cary-Blair | 20/20 | 35.35 | 21,523 | 8,380 | 2.6 |
| *C. jejuni* | 75 | Raw | 20/20 | 37.78 | 13,089 | 9,338 | 1.4 |
| | | Cary-Blair | 20/20 | 36.30 | 20,001 | 10,962 | 1.8 |
| *C. coli* | 150 | Raw | 20/20 | 40.57 | 12,987 | 10,285 | 1.3 |
| | | Cary-Blair | 20/20 | 39.41 | 14,161 | 10,529 | 1.3 |
| | 300 | Raw | 20/20 | 38.77 | 14,178 | 10,311 | 1.4 |
| | | Cary-Blair | 20/20 | 37.63 | 16,193 | 10,553 | 1.5 |
| *Shigella* | 100 | Raw | 20/20 | 35.97 | 5,049 | 838 | 6.0 |
| | | Cary-Blair | 20/20 | 35.43 | 5,618 | 867 | 6.5 |
| STEC-*Stx1* | 100 | Raw | 20/20 | 38.83 | 6,513 | 4,667 | 1.4 |
| | | Cary-Blair | 20/20 | 38.72 | 7,065 | 4,874 | 1.4 |
| STEC-*Stx2* | 100 | Raw | 20/20 | 38.99 | 6,465 | 4,561 | 1.4 |
| | | Cary-Blair | 20/20 | 38.32 | 7,153 | 4,890 | 1.5 |

Data from this experiment showed 100% positivity (5/5) at concentrations at least as low as 100 CFU/mL for *Salmonella typhimurium*, *Shigella sonnei*, STEC stxl and STEC stx2. *Campylobacter coli* was 100% positive at 300 CFU/mL and 80% positive (4/5) at 100 CFU/mL, and *Campylobacter jejuni* was 100% positive at 75 CFU/mL. Confirmatory testing showed 100% positivity (20/20) for each of *Campylobacter coli*, *Campylobacter jejuni*, *Shigella sonne*i, STEC stxl and STEC stx2 in two different stool matrices. *Salmonella typhimurium* was 90% positive in the raw stool matrix and 95% positive in the Cary-Blair stool matrix. These data show that the multiplex primer/probe combination has a sensitivity between 75 to 150 CFU/mL.

### Example 3 - Primer-Probe Combinations for Amplification and Detection of Salmonella

Several primer and probe combinations were prepared and tested for amplification and detection of *Salmonella enterica.* Combinations are described in **Table 4** below. These primer-probe combinations were tested against three concentrations of a serially diluted stock concentration of *Salmonella enterica.* Dilutions were made into a sample transport reagent and the 500 CFU/mL, 150 CFU/mL, and 50 CFU/mL concentrations were tested. Negative reaction wells were sample transport media. Reaction conditions were set up in triplicate and real-time amplification and detection reactions were performed. RFU and Ct results are shown in **Table 5.**

**Table 4. Primer and Probe Combinations**

| Primer-Probe Combo No. | SEQ ID NO: | Oligo Type | Modifications & Labels* |
|---|---|---|---|
| Combo 1 | SEQ ID NO:21 | Forward Primer | 5mC at residues 13, 17, and 24 |
| | | | Inosine at residues 14, and 23 |
| | SEQ ID NO:47 | Reverse Primer | 5mC at residues 11, 16, and 17 |
| | | | Inosine at residue 4 |
| | SEQ ID NO:45 | Taqman Probe | 5mC at residues 3, 6, 8, and 17 |
| | | | Inosine at residues 10, 15, and 19 FAM/BHQ1 |
| Combo 2 | SEQ ID NO:38 | Forward Primer | 5mC at residues 4, 6, 7, and 8 |
| | | | Inosine at residue 22 |
| | SEQ ID NO:36 | Reverse Primer | 5mC at residues 2, 3, 4, 16, 18, 19, and 22 |
| | | | Inosine at residue 7 |
| | SEQ ID NO:44 | Taqman Probe | 5mC at residues 7, 9, 13, and 15 |
| | | | Inosine at residues 5, and 8FAM/BHQ1 |
| Combo 3 | SEQ ID NO:38 | Forward Primer | 5mC at residues 4, 6, 7, and 8 |
| | | | Inosine at residue 22 |
| | SEQ ID NO:36 | Reverse Primer | 5mC at residues 2, 3, 4, 16, 18, 19, and 22 |
| | | | Inosine at residues 5, and 8 |
| | SEQ ID NO:26 | Taqman Probe | 5mC at residues 9, 18, and 23 |
| | | | Inosine at residue 6 |
| | | | FAM/BHQ1 |
| Combo 4 | SEQ ID NO:35 | Forward Primer | |
| | SEQ ID NO:40 | Reverse Primer | 5mC at residues 4, 13, 18, and 22 |
| | | | Inosine at residues 16, and 19 |
| | SEQ ID NO:30 | Taqman Probe | 5mC at residues 2, 4, 18, 19, and 20 |
| | | | Inosine at residues 14, and 23 FAM/BHQ1 |
| Combo 5 | SEQ ID NO:12 | Forward Primer | 5mC at residues 4, 11, 12, and 16 |
| | SEQ ID NO:28 | Reverse Primer | 5mC at residues 2, 4, 12, 14, 15, and 17, |
| | | | Inosine at residue 18 |
| | SEQ ID NO:13 | Taqman Probe | FAM/BHQ1 |
| Combo 6 | SEQ ID NO:42 | Forward Primer | Inosine at 10 and 16 |
| | SEQ ID NO:31 | Reverse Primer | 5mC at residues 6, 7, 8, 13, 23, 24, and 26 |
| | | | Inosine at residue 20 |
| | SEQ ID NO:23 | Taqman Probe | 5mC at residues 2, 5, 7, and 14 |
| | | | Inosine at residue 15 |
| | | | FAM/BHQ1 |
| Combo 7 | SEQ ID NO:41 | Forward Primer | |
| | SEQ ID NO:27 | Reverse Primer | 5mC at residues at 6, 10, and 18 |
| | SEQ ID NO:43 | Taqman Probe | 5mC at residues 2, 9, 12, 16, 17, 22, 23, and 26 |
| | | | Inosine at residue 15 |
| | | | FAM/BHQ1 |

| | | | |
|---|---|---|---|
| * 5mC refers to a residue that comprises 5-methylcytosine. BHQ refers to Black Hole Quencher. FAM refers to fluorescein. | | | |

**Table 5. Results**

| Primer-Probe Combo No. | Condition | Avg RFU | Avg Ct |
|---|---|---|---|
| Combo 3 | STM only | - | - |
| | *S. enterica* 50 CFU/mL | 17,608 | 38.31 |
| | *S. enterica* 150 CFU/mL | 20,326 | 37.19 |
| | *S. enterica* 500 CFU/mL | 24,900 | 36.12 |
| Combo 4 | STM only | 271 | - |
| | *S. enterica* 50 CFU/mL | 23,236 | 37.14 |
| | *S. enterica* 150 CFU/mL | 25,402 | 36.30 |
| | *S. enterica* 500 CFU/mL | 28,034 | 35.29 |
| Combo 5 | STM only | - | - |
| | *S. enterica* 50 CFU/mL | 20,353 | 38.97 |
| | *S. enterica* 150 CFU/mL | 22,824 | 37.54 |
| | *S. enterica* 500 CFU/mL | 26,103 | 35.14 |
| Combo 6 | STM only | 28 | - |
| | *S. enterica* 50 CFU/mL | 5,605 | 41.32 |
| | *S. enterica* | 19,624 | 38.48 |
| | 150 CFU/mL | | |
| | *S. enterica* 500 CFU/mL | 23,292 | 36.93 |
| Combo 7 | STM only | - | - |
| | *S. enterica* 50 CFU/mL | 13,119 | 38.98 |
| | *S. enterica* 150 CFU/mL | 16,774 | 38.80 |
| | *S. enterica* 500 CFU/mL | 21,322 | 35.90 |

Primer and probe combinations 1 and 2 showed only negative results in this experiment, and thus the results are not presented in **Table 5.** Each of combinations 3 to 7 showed robust RFU over background. Combinations 4 and 5 showed the most robust RFUs and fastest Cts in this experiment.

### Example 4 - Analytical Specificity and Interference

Analytical specificity for an exemplary multiplex assay for detecting *Salmonella, Shigella*, *Campylobacter* (*C. jejuni* and *C*. *coli*, undifferentiated), and STEC (stx-1 and stx-2 undifferentiated) was tested. The assay was run as a real-time, multiplex PCR reaction utilizing the primer and probe combination of **Table 1** and cycling parameters.

Analytical specificity of the PCR assay was determined using a total of 81 organisms divided into 17 panels as listed in **Table 6** and **Table 6.1.** These 81 organisms are commonly found in stool samples (organisms available from American Type Tissue Culture, Manassas, VA). The 17 panels of organisms were spiked into negative Cary-Blair stool matrices. The concentrations of the organisms were 1E6 CFU/mL in each test, except for *Bifidobacterium adolescentis*, *Egglerthella lenta*, and *Peptostreptococcus micros* with concentration of 1E6 rRNA copies/mL, and *Entamoeba histolytica* with a concentration of 1E4 cells/mL. Positive control was *Salmonella enterica* (strain V1796), *Campylobacter coli* (strain RO 268), *Shigella flexneri* (strain 24570), and STEC (strain CDC 1999-3302) spiked at 3X LoD into Cary-Blair stool matrices. Negative control was Cary-Blair stool matrix only. Reactions were run in triplicate. Reactivity or positivity was defined as an amplification curve crossing the Ct threshold for each channel. Any curve below this threshold was considered as negative or not reactive. Ct thresholds used were: FAM, 600; HEX, 1000; ROX, 500; Q670, 600; and Q705, 1000. Results are presented in **Table 7, Table 7.1, Table 8,** and **Table 8.1.**

**Table 6. Analytical Specificity Panel**

| Panel No. | Organism |
|---|---|
| 1 | *Abiotrophia defectivia* |
| | *Acinetobacter baumannii* |
| | *Acinetobacter lwoffii* |
| | *Aeromonas hydrophila* |
| | *Alcaligenes faecalis* |
| | *Anaerococcus tetradius* |
| 2 | *Anaerococcus vaginalis* |
| | *Acrcobacter butzleri* |
| | *Bacillus cereus* |
| | *Bacteriodes fragilis* |
| | *Bacteroides thetaiotaomicron* |
| | *Bacteroides vulgatus* |
| 3 | *Bifiodobacterium adolescentis* |
| | *Bifiodobacterium longum* |
| | *Campylobacter fetus* |
| | *Campylobacter hyointestinalis* |
| | *Campylobacter rectus* |
| | *Campylobacter sputorum* |
| 4 | *Candida albicans* |
| | *Citrobacter freundii* |
| | *Citrobacter koseri* |
| | *Clostridium difficile* |
| | *Clostridium perfringens* |
| | *Clostridium ramosum* |
| 5 | *Clostridium sordelli* |
| | *Clostridium tertium* |
| | *Collinsella aerofaciens* |
| | *Corynebacterium genitalium* |
| | *Cronobacter sakazakii* |
| | *Edwardsiella tarda* |
| 6 | *Egglerthella lenta* |
| | *Entamoeba histolytica* |
| | *Entercoccus faecalis* |
| | *Enterobacter aerogenes* |
| | *Enterobacter cloacae* |
| | *Escherichia coli* |
| 7 | *Escherichia coli* |
| | *Escherichia fergusonii* |
| | *Escherichia hermanii* |
| | *Escherichia vulneris* |
| | *Gardnerella vaginalis* |
| | *Helicobacter pylori* |
| 8 | *Klebsiella oxytoca* |
| | *Klebsiella ozaenae* |
| | *Klebsiella pneumoniae* |
| | *Lactobacillus acidophilus* |
| | *Lactobacillus crispatus* |
| | *Lactococcus lactis* |
| 9 | *Leptotrichia buccalis* |
| | *Listeria grayi* |
| | *Listeria monocytogenes* |
| | *Megaspbaeara elsdenii* |
| | *Morganella morganii* |
| | *Peptostreptococcus anaerobius* |
| 10 | *Peptostreptococcus micros* |
| | *Photobacterium damselae* |
| | *Plesiomonas shigelloides* |
| | *Prevotella bivia* |
| | *Prevotella melaninogenica* |
| | *Proteus mirabilis* |
| 11 | *Proteus penneri* |
| | *Proteus vulgaris* |
| | *Providencia alcalifaciens* |
| | *Providencia rettgeri* |
| | *Providencia stuartii* |
| | *Pseudomonas aeruginosa* |
| 12 | *Pseudomonas fluorescens* |
| | *Serratia liquefaciens* |
| | *Serratia marcescens* |
| | *Staphylococcus aureus* |
| | *Staphylococcus epidermidis* |
| | *Stenotrophomonas maltophilia* |
| 13 | *Streptococcus anginosus* |
| | *Streptococcus dysgalactiae* |
| | *Yersinia bercovieri* |
| | *Yersinia pseudotuberculosis* |
| | *Yersinia rohdei* |
| 14 | *Campylobacter lari* |
| 15 | *Campylobacter upsaliensis* |

**Table 6.1. Analytical Specificity Panel (EIEC)**

| Panel No. | Organism |
|---|---|
| A | *Escherichia coli O124* (EIEC O124) |
| B | *Escherichia coli O29* (EIEC O29) |

**Table 7. Specificity Data**

| Panel | Reactivity | | | |
|---|---|---|---|---|
| | *Salmonella* | *Campylobacter* | *Shigella* | STEC |
| Control | 0/3 | 0/3 | 0/3 | 0/3 |
| 1 | 0/3 | 0/3 | 0/3 | 0/3 |
| 2 | 0/3 | 0/3 | 0/3 | 0/3 |
| 3 | 0/3 | 0/3 | 0/3 | 0/3 |
| 4 | 0/3 | 0/3 | 0/3 | 0/3 |
| 5 | 0/3 | 0/3 | 0/3 | 0/3 |
| 6 | 0/3 | 0/3 | 0/3 | 0/3 |
| 7 | 0/3 | 0/3 | 0/3 | 0/3 |
| 8 | 0/3 | 0/3 | 0/3 | 0/3 |
| 9 | 0/3 | 0/3 | 0/3 | 0/3 |
| 10 | 0/3 | 0/3 | 0/3 | 0/3 |
| 11 | 0/3 | 0/3 | 0/3 | 0/3 |
| 12 | 0/3 | 0/3 | 0/3 | 0/3 |
| 13 | 0/3 | 0/3 | 0/3 | 0/3 |
| 14 | 0/3 | 0/3 | 0/3 | 0/3 |
| 15 | 0/3 | 0/3 | 0/3 | 0/3 |

**Table 7.1. Specificity Data (EIEC)**

| Panel | Reactivity | | | |
|---|---|---|---|---|
| | *Salmonella* | *Campylobacter* | *Shigella* | STEC |
| Control | 0/3 | 0/3 | 0/3 | 0/3 |
| A | 0/3 | 0/3 | 3/3 | 0/3 |
| B | 0/3 | 0/3 | 3/3 | 0/3 |

**Table 8. Interference Data**

| Panel | *Salmonella* | | *Campylobacter* | | *Shigella* | | STEC | |
|---|---|---|---|---|---|---|---|---|
| | Reactivity | Avg Ct | Reactivity | Avg Ct | Reactivity | Avg Ct | Reactivity | Avg Ct |
| Control | 3/3 | 33.6 | 3/3 | 35.9 | 3/3 | 31.3 | 3/3 | 36.5 |
| 1 | 3/3 | 34.1 | 3/3 | 36.2 | 3/3 | 31.8 | 3/3 | 37.3 |
| 2 | 3/3 | 33.7 | 3/3 | 35.9 | 3/3 | 31.6 | 3/3 | 37.4 |
| 3 | 3/3 | 33.8 | 3/3 | 35.7 | 3/3 | 31.4 | 3/3 | 36.4 |
| 4 | 3/3 | 33.9 | 3/3 | 35.9 | 3/3 | 31.9 | 3/3 | 37.1 |
| 5 | 3/3 | 33.9 | 3/3 | 36.2 | 3/3 | 31.5 | 3/3 | 36.7 |
| 6 | 3/3 | 33.4 | 3/3 | 36.0 | 3/3 | 31.4 | 3/3 | 36.4 |
| 7 | 3/3 | 33.8 | 3/3 | 35.7 | 3/3 | 31.7 | 3/3 | 36.5 |
| 8 | 3/3 | 33.2 | 3/3 | 34.9 | 3/3 | 31.8 | 3/3 | 36.3 |
| 9 | 3/3 | 34.2 | 3/3 | 36.3 | 3/3 | 32.1 | 3/3 | 36.7 |
| 10 | 3/3 | 34.1 | 3/3 | 36.1 | 3/3 | 31.9 | 3/3 | 36.6 |
| 11 | 3/3 | 33.9 | 3/3 | 36.0 | 3/3 | 31.7 | 3/3 | 36.8 |
| 12 | 3/3 | 34.1 | 3/3 | 36.3 | 3/3 | 31.9 | 3/3 | 36.9 |
| 13 | 3/3 | 33.8 | 3/3 | 36.1 | 3/3 | 31.7 | 3/3 | 37.1 |
| 14 | 3/3 | 34.2 | 3/3 | 36.3 | 3/3 | 31.3 | 3/3 | 36.8 |
| 15 | 3/3 | 33.7 | 3/3 | 35.7 | 3/3 | 30.9 | 3/3 | 36.5 |

**Table 8.1. Interference Data (EIEC)**

| Panel | *Salmonella* | | *Campylobacter* | | *Shigella* | | STEC | |
|---|---|---|---|---|---|---|---|---|
| | Reactivity | Avg Ct | Reactivity | Avg Ct | Reactivity | Avg Ct | Reactivity | Avg Ct |
| Control | 3/3 | 33.6 | 3/3 | 35.9 | 3/3 | 31.3 | 3/3 | 36.5 |
| A | 3/3 | 33.7 | 3/3 | 36.0 | 3/3 | 20.7 | 3/3 | 37.3 |
| B | 3/3 | 33.7 | 3/3 | 35.9 | 3/3 | 20.4 | 3/3 | 38.0 |

The primers and probes did not react with any of the challenge organisms listed in **Table 6,** as shown in **Table 7.** Moreover, the primers and probes showed 100% positivity when target bacteria were present in a reaction well, as shown in **Table 8.** Thus, these primers and probes demonstrate no cross-reactivity with organisms that are commonly found in stool or cause similar disease states as the target organisms. The primers and probes demonstrated high specificity, even while in the presence of commonly found non-target organisms.

Additionally, since *Shigella* and EIEC have substantially identical ipaH genes, the *Shigella* primers and probes reacted with the organisms in **Table 6.1,** as shown in **Table 7.1.** And the primers and probes also showed 100% positivity when target bacteria were present in a reaction well, as shown in **Table 8.1.** As such, the *Shigella* primers and probes may be used to detect the presence of EIEC in a sample.

### Example 5 - Shigella Primer and Probe Screening

Several primer and probe combinations were prepared and tested for amplification and detection of *Shigella* sonnei. Combinations are described in **Table 9** below. These primer-probe combinations were tested against three concentrations of a serially diluted stock concentration of *Shigella sonnei.* Dilutions were made into a sample transport reagent and the 500 CFU/mL, 150 CFU/mL, and 50 CFU/mL concentrations were tested. Negative reaction wells were sample transport media. Reaction conditions were set up in triplicate and real-time amplification and detection reactions were performed. RFU and Ct results were analyzed and are presented below.

**Table 9. Primer & Probe Combinations**

| Primer-Probe Combo No. | SEQ ID NO: | Oligo Type | Modifications & Labels* |
|---|---|---|---|
| Combo 1.1 | SEQ ID NO:34 | Forward Primer | |
| | SEQ ID NO:22 | Reverse Primer | 5mC at residues 5 and 16 |
| | SEQ ID NO:46 | Taqman Probe | 5mC at residues 5, 11, and 12 ROX/BHQ2 |
| Combo 1.2 | SEQ ID NO:34 | Forward Primer | |
| | SEQ ID NO:22 | Reverse Primer | 5mC at residues 5 and 16 |
| | SEQ ID NO:29 | Taqman Probe | ROX/BHQ2 |
| Combo 2 | SEQ ID NO:15 | Forward Primer | 5mC at residues 7 and 15 |
| | | | Inosine at residue 3 |
| | SEQ ID NO:17 | Reverse Primer | |
| | SEQ ID NO:16 | Taqman Probe | ROX/BHQ2 |
| | | | Inosine at residue 16 |
| Combo 3 | SEQ ID NO:32 | Forward Primer | 5mC at residues 5, 11, 12 and 15 |
| | | | Inosine at residue 9 |
| | SEQ ID NO:33 | Reverse Primer | |
| | SEQ ID NO:24 | Taqman Probe | ROX/BHQ2 |

| | | | |
|---|---|---|---|
| * 5mC refers to a residue that comprises 5-methylcytosine. BHQ refers to Black Hole Quencher. FAM refers to fluorescein. ROX refers to carboxyrhodamine. | | | |

Results showed 100% positivity for each primer-probe combination (Ct threshold set at 1,000 RFU). Primer-probe combination 2 showed the highest RFU (approximately 12,000 RFU at 500 cfu/mL), though at 50 cfu/mL one of the replicates fell to about 2,000 RFU while the other two replicates were at about 8,000 RFU. Primer-probe combination 1.2 showed good overall results with consistent RFU and Ct values for the tested replicate conditions. RFU and Ct results are shown in **Table 10.**

**Table 10. Combination Screening**

| Primer-Probe Combo No. | Condition | Individual RFU measurements | Average RFU | Average Ct |
|---|---|---|---|---|
| Combo 1.1 | STM only | 128.47 | 137 | - |
| | | 138.43 | | |
| | | 144.71 | | |
| | *S. sonnei* 50 CFU/mL | 2938.8 | 2,279 | 40.87 |
| | | 2528.24 | | |
| | | 1370.21 | | |
| | *S. sonnei* 150 CFU/mL | 3348.32 | 3,546 | 38.88 |
| | | 3961.5 | | |
| | | 3327.68 | | |
| | *S. sonnei* 500 CFU/mL | 4401.85 | 4,346 | 37.81 |
| | | 5090.91 | | |
| | | 3544.61 | | |
| Combo 1.2 | STM only | 79.32 | 88 | - |
| | | 81.6 | | |
| | | 103 | | |
| | *S. sonnei* 50 CFU/mL | 4144.9 | 4,856 | 38.98 |
| | | 6076.92 | | |
| | | 4345 | | |
| | *S. sonnei* 150 CFU/mL | 5442.56 | 6,492 | 37.18 |
| | | 7344 | | |
| | | 6690.76 | | |
| | *S. sonnei* 500 CFU/mL | 5658.92 | 6,759 | 36.51 |
| | | 7793.4 | | |
| | | 6826.04 | | |
| Combo 2 | STM only | 150.8 | 122 | - |
| | | 115.7 | | |
| | | 100.28 | | |
| | *S. sonnei* 50 CFU/mL | 2127.4 | 6,090 | 38.88 |
| | | 8369.19 | | |
| | | 7773.1 | | |
| | *S. sonnei* 150 CFU/mL | 9423 | 9,633 | 37.15 |
| | | 9812.83 | | |
| | | 9663.4 | | |
| | *S. sonnei* 500 CFU/mL | 11020.7 | 11,469 | 35.31 |
| | | 11591.6 | | |
| | | 11794.2 | | |
| Combo 3 | STM only | 111 | 109 | - |
| | | 116.4 | | |
| | | 99.28 | | |
| | *S. sonnei* 50 CFU/mL | 3604 | 5,108 | 39.91 |
| | | 5927.2 | | |
| | | 5792.7 | | |
| | *S. sonnei* 150 CFU/mL | 7257.6 | 7,436 | 37.66 |
| | | 6773 | | |
| | | 8276 | | |
| | *S. sonnei* 500 CFU/mL | 6957.8 | 7,304 | 36.87 |
| | | 7275.8 | | |
| | | 7680.6 | | |

### Example 6 - Co-Infection/Competitive Interference

The performance of detecting two or more target organisms in a single sample, using the primer-probe combination shown in **Table 1** in a multiplex PCR assay, was measured. To create the co-infected sample, a negative Cary-Blair stool matrix was spiked with a combination of a high concentration (1E6 CFU/mL) and a low concentration (3xLoD) of target organisms, as illustrated in **Table 11.** The primer-probe combination was tested against a target organism at the low concentration in the absence of a co-infectant, and was further tested with a combination of a target organism at the low concentration and a separate target organism at the high concentration (see **Table 12**).

**Table 11. Target Bacteria and Representative Strain Used.**

| Target Organism | Representative Strain |
|---|---|
| *Salmonella enterica* | CDC 6516-60 |
| *Campylobacter coli* | CIP 7080 |
| *Shigella sonnei* | AMC 43-GG9 |
| STEC-O157 | O157:H7 |

**Table 12. Results of GI-Bac1 Co-Infection**

| Low Conc. 3X LoD | High Conc. 1E6 CFU/mL | *Salmonella* | | *Campylobacter* | | *Shigella* | | STEC | |
|---|---|---|---|---|---|---|---|---|---|
| | | React. | Mean Ct (SD) | React. | Mean Ct (SD) | React. | Mean Ct (SD) | React. | Mean Ct (SD) |
| *Salmonella* | None | 3/3 | 33.93 (0.7) | 0/3 | - | 0/3 | - | 0/3 | - |
| | *C. coli* | 3/3 | 33.17 (0.2) | 3/3 | 24.05 (0.4) | 0/3 | - | 0/3 | - |
| | *Shigella* | 3/3 | 33.29 (0.1) | 0/3 | - | 3/3 | 20.47 (0.2) | 0/3 | - |
| | STEC | 3/3 | 33.23 (0.2) | 0/3 | - | 0/3 | - | 3/3 | 23.40 (0.1) |
| *C. coli* | None | 0/3 | - | 3/3 | 35.37 (0.2) | 0/3 | - | 0/3 | - |
| | *Salmonella* | 3/3 | 22.09 (0.06) | 3/3 | 35.58 (0.2) | 0/3 | - | 0/3 | - |
| | *Shigella* | 0/3 | - | 3/3 | 35.56 (0.2) | 3/3 | 20.50 (0.2) | 0/3 | - |
| | STEC | 0/3 | - | 3/3 | 35.06 (0.2) | 0/3 | - | 3/3 | 23.19 (0.1) |
| *Shigella* | None | 0/3 | - | 0/3 | - | 3/3 | 32.16 (0.0) | 0/3 | - |
| | *Salmonella* | 3/3 | 22.38 (0.2) | 0/3 | - | 3/3 | 32.09 (0.2) | 0/3 | - |
| | *C. coli* | 0/3 | - | 3/3 | 24.54 (0.2) | 3/3 | 32.57 (0.3) | 0/3 | - |
| | STEC | 0/3 | - | 0/3 | - | 3/3 | 31.69 (0.2) | 3/3 | 23.32 (0.2) |
| STEC-0157 | None | 0/3 | - | 0/3 | - | 0/3 | - | 3/3 | 35.80 (0.3) |
| | *Salmonella* | 3/3 | 22.51 (0.2) | 0/3 | - | 0/3 | - | 3/3 | 36.04 (0.3) |
| | *C. coli* | 0/3 | - | 3/3 | 24.22 (0.3) | 0/3 | - | 3/3 | 36.21 (0.6) |
| | *Shigella* | 0/3 | - | 0/3 | - | 3/3 | 20.52 (0.06) | 3/3 | 36.57 (0.2) |

Detection of both the high and low concentrations of all target bacteria were 100%. There were no false positives, and no competitive interference was observed. The data show that the primer-probe combination can accurately detect multiple target bacteria present in a single sample using multiplex PCR.

### Example 7 - Mitigating Inhibition of Amplication Reactions with α-Cyclodextrin and/or Polysorbate 20

Detergents that are present in, e.g., wash buffers, are known to inhibit or diminish nucleic acid amplification reactions. To investigate the ability of α-cyclodextrin and polysorbate 20 to mitigate such negative/inhibitory effects, several PCR reaction mixtures were prepared for testing with samples containing a detergent.

An master mix was prepared to contain DNA polymerase at 0.46 U/µL, reverse transcriptase at 0.5 U/µL, RNase inhibitor at 0.2 U/µL, dNTPs at 0.25 mM each, dUTP at 0.05 mM, inorganic salts including KCl and MgCl₂ at 81 and 5.1 mM, EDTA at 0.1 mM, and primers and probes for amplification and detection of a target nucleic acid. The master mix was separated into conditions (A) through (F), wherein conditions (B) and (D) further contained polysorbate 20 at 0.025% (v/v), conditions (B), (C) and (F) further contained 12.5 mg/mL α-cyclodextrin, condition (C) further contained polysorbate 20 at 0.13% (v/v), and condition (E) further contained 50 mg/mL α-cyclodextrin. Samples were prepared by spiking the target nucleic acid into a suitable media and were incubated in a buffered reagent containing poly-T coated magnetic microparticles and a target capture oligomer to bind the target nucleic acids to a magnetic solid support. The magnetic microparticles and bound nucleic acid were separated out, and following additional separation and washing, an eluate containing nucleic acid was recovered. The eluate was divided into separate containers, and one of the eluate portions was further spiked with 30% (v/v) of wash buffer to produce eluates containing 100 mg/mL of sodium dodecyl sulfate to simulate carry over of wash buffer from an upstream sample processing step. Each of the above master mix conditions (A) to (F) were combined with an aliquot of the wash-buffer-spiked or non-spiked eluate (20 µL master mix, 10 µL eluate, 30 µL total reaction volume per replicate). Thus, the final concentration of polysorbate 20 and/or α-cyclodextrin in each reaction mixture was 2/3 of the concentration listed above for the master mix. Real-time amplification and detection reactions were set up for the replicates, and the reactions were performed using a thermal cycling instrument (Panther Fusion Instrument, Hologic, Inc., San Diego, CA). Results are presented in **Table 13** below.

**Table 13. Amplification Results**

| **Master Mix Condition** | **Wash Buffer Spike** | **N Positives** | **Average Ct** | **SD Ct** | **Average RFU** | **SD RFU** |
|---|---|---|---|---|---|---|
| (A) | No | 20/20 | 33.2 | 0.8 | 22225 | 3134 |
| (A) | Yes | 1/20 | 38.2* | n/a | 791 | n/a |
| (B) | No | 20/20 | 32.2 | 0.5 | 25043 | 2460 |
| (B) | Yes | 20/20 | 32.5 | 0.9 | 24276 | 2879 |
| (C) | No | 20/20 | 32.4 | 0.5 | 22664 | 3118 |
| (C) | Yes | 20/20 | 32.3 | 0.5 | 23200 | 2561 |
| (D) | Yes | 20/20 | 34.2 | 0.6 | 15690 | 1785 |
| (E) | Yes | 19/19 | 34.2 | 0.8 | 19965 | 2826 |
| (F) | Yes | 20/20 | 35.2 | 1.2 | 19346 | 3674 |

| | | | | | | |
|---|---|---|---|---|---|---|
| *Ct value is for a single replicate, not an average | | | | | | |

The addition of α-cyclodextrin and polysorbate 20 into the reaction mixture mitigated the negative/inhibitory effects that detergents have on the nucleic acid amplification reactions. The Ct and RFU values further indicate that the combination of these two reagents had a synergistic mitigating effect on the detergent-induced inhibition relative to the mitigating effects observed using either α-cyclodextrin or polysorbate 20 alone.

**Table of Sequences.**

| **SEQ ID NO:** | **Description** | **Oligo ID** | **Sequence (N = inosine)** |
|---|---|---|---|
| 1 | *E. coli* Set1-stx1-Probe2 | 349336 | CTATACCACGTTACAGCGTGTTGCAG |
| 2 | *E. coli* Set1-stx1-Probe3 | 349337 | TTATCTGCATCCCCGTACGACTGATC |
| 3 | *E. coli* Set1-stx1-RP2 | 349794 | GAAGTAGTCAACGAATGGCG |
| 4 | *E. coli* Set1-stx2-FP2 | 349795 | CCATGACAACGGACAGCAGTTATA |
| 5 | *E. coli* Set1-stx2-Probe4 | 349340 | ACTCTGCAACGTGTCGCAGCGCT |
| 6 | *E. coli* Set1-stx2-Probe7 | 349723 | AACGTTCCGGAATGCAAATCAGTCGTC |
| 7 | *E. coli* Set1-stx2-RP | 348780 | CGCCAGATATGATGAAACCAGTGAG |
| 8 | *C. coli* Set1-cadF-FP | 348752 | GAGCGTGGCTTATCTTGACC |
| 9 | *C. coli* Set1-cadF-Probe7 | 349690 | ACTGTCTGGATACGACTTTTATCTACACCG |
| 10 | *C. coli* Set1-cadF-RP | 348754 | CAGAAAGACGCGCTAACAGCGT |
| 11 | *C. jejuni* Set1-glyA-FP3 | 349910 | GCTAATGTTCAGCCTAATTCAGGT |
| 12 | *Salmonella* Set1-InvA-FP | 348725 | GAACGTGTTTCCGTGCGTAAT |
| 13 | *Salmonella* Set1-InvA-Probe6 | 349688 | ATGGAAGCGCTCGCATTGTGGGC |
| | *Salmonella* Set1-InvA-Probe | 348726 | |
| 14 | *Salmonella* Set1-InvA-RP3 | 349577 | ATATAACGCGCCATTGCYCCACGAATATG |
| 15 | *Shigella* Set2-ipaH-FP | 348762 | AGNGTACTCATTCTCCAGCATCTC |
| 16 | *Shigella* Set2-ipaH-Probe | 349863 | TTCTGCTCTTCTGCCNGCGCCCA |
| | *Shigella* Set2-ipaH-Probe | 348764 | TTCTGCTCTTCTGCCNGCGCCCA |
| 17 | *Shigella* Set2-ipaH-RP | 348765 | TACGGACTGGTTCTCCCTCT |
| 18 | *C.jejuni* Set1-glyA-Probe9 | 349913 | CTCAAGCYAATCAAGGTGTTTATGCGGCT |
| | *C. jejuni* Set1-glyA-Probe9 | 350333 | |
| 19 | *C. jejuni* Set1-glyA-RP2 | 349793 | CCATGACTTAAATCCATTCCTANAA |
| 20 | *E. coli* Set1-stx1-FP2 | 349626 | GGTTACATTGTCTGGTGACAGTAG |
| 21 | *Salmonella* Set1-OrgC-FP | 348715 | AAGAGATGAATGCNTTCAAAGANCC |
| 22 | *Shigella* Set1-ipaH-RP | 348761 | AATGCGTTTCTATGGCGTGTCGG |
| 23 | *Salmonella* Set2-InvA-Probe | 348729 | ACTGCTCGTAATTCNCCGCCATTGGC |
| 24 | *Shigella* Set3-ipaH-Probe | 348767 | AGGGAGAGCAGAGCGCCGGTATCAT |
| 25 | *Salmonella* Set2-OrgC-Probe3 | 349092 | TGAANGCNCAGTCACAAATG |
| 26 | *Salmonella* Set2-OrgC-Probe2 | 348721 | CAGGANGTCATTTGTGACTGTGCG |
| 27 | *Salmonella* Set3-InvA-RP | 348733 | CAGTGCGATCAGGAAATCAACCAGA |
| 28 | *Salmonella* Set1-InvA-RP | 348727 | CCACGAATATGCTCCACNAGGTTAATG |
| 29 | *Shigella* Set1-ipaH-Probe2 | 348760 | CCAGAGGGAGAACCAGTCCG |
| 30 | *Salmonella* Set3-OrgC-Probe | 348723 | CCTCATATAAATGNGTGCCCGGNTC |
| 31 | *Salmonella* Set2-InvA-RP | 348730 | CGGATCCCTTTGCGAATAANATCCTCA |
| 32 | *Shigella* Set3-ipaH-FP | 348766 | CGGGCAATNTCCTCCAGAATTT |
| 33 | *Shigella* Set3-ipaH-RP | 348768 | CTCCTGGTCCATCAGGCATC |
| 34 | *Shigella* Set1-ipaH-FP | 348758 | CTTCCGTACGCTTCAGTACAG |
| 35 | *Salmonella* Set3-OrgC-FP | 348722 | GCAGCAAACGGCTATGCTACAA |
| 36 | *Salmonella* Set2-OrgC-RP | 348719 | GCCCGANAAAATAATCTCCTGCATC |
| 37 | *C. coli* Set1-cadF-Probe | 348753 | CGGTGTAGATANAAGTCGTATCCAGACAG |
| | *C. coli* Set1-cadF-Probe3 | 349433 | |
| | *C. coli* Set1-cadF-Probe5 | 349435 | |
| 38 | *Salmonella* Set2-OrgC-FP | 348718 | GGGCACCCATTTATATGAGGCNTTA |
| 39 | *C. coli* Set1-cadF-Probe2 | 349432 | TCTGGATACGACTTNTATCTACACCG |
| 40 | *Salmonella* Set3-OrgC-RP | 348724 | GGTCATTTGTGACTGNGCNTTCA |
| 41 | *Salmonella* Set3-InvA-FP | 348731 | GTGCTCGTTTACGACCTGAATTACTG |
| 42 | *Salmonella* Set2-InvA-FP | 348728 | TCGTGGAGCNATGGCNCGTTATAT |
| 43 | *Salmonella* Set3-InvA-Probe | 348732 | TCTATGTTCGTCATNCCATTACCTACC |
| 44 | *Salmonella* Set2-OrgC-Probe1 | 348720 | TGAANGCNCAGTCACAAATGAC |
| 45 | *Salmonella* Set1-OrgC-Probe | 348716 | TGCTGCTCANAAGANACANAAGC |
| 46 | *Shigella* Set1-ipaH-Probe1 | 348759 | TGGTCAGAAGCCGTGAAGAGAATG |
| 47 | *Salmonella* Set1-OrgC-RP | 348717 | TGTNTTGTAGCATAGCCGTT |
| 48 | *E. coli* Set1-stx1-Probe | 348770 | ACAGCGTGTTGCAGGGATCAGTCGT |
| 49 | *E. coli* Set1-stx1-FP | 348769 | CGGTTACATTGTCTGGTGACAGTAG |

The further aspects of the present disclosure are set forth in the following numbered paragraphs:
1. A set of oligonucleotides for determining the presence or absence of at least one enteric pathogen, said set of oligonucleotides comprising at least one of (a)-(e):
   (a) a *Salmonella-*specific amplification oligomer set comprising first and second oligomers respectively comprising target-hybridizing sequences substantially corresponding to the nucleotide sequences of: (i) SEQ ID NO:12 and SEQ ID NO:14; (ii) SEQ ID NO:21 and SEQ ID NO:47; (iii) SEQ ID NO:38 and SEQ ID NO:36; (iv) SEQ ID NO:35 and SEQ ID NO:40; (v) SEQ ID NO:12 and SEQ ID NO:28; (vi) SEQ ID NO:42 and SEQ ID NO:31; or (vii) SEQ ID NO:41 and SEQ ID NO:27;
   (b) a *C. jejuni*-specific amplification oligomer set comprising first and second oligomers respectively comprising target-hybridizing sequences substantially corresponding to the nucleotide sequences of: (i) SEQ ID NO:11 and SEQ ID NO:19;
   (c) a *C*. *coli-*specific amplification oligomer set comprising first and second oligomers respectively comprising target-hybridizing sequences substantially corresponding to the nucleotide sequences of: (i) SEQ ID NO:8 and SEQ ID NO:10;
   (d) a *Shigella*-specific amplification oligomer set comprising first and second oligomers respectively comprising target-hybridizing sequences substantially corresponding to the nucleotide sequences of: (i) SEQ ID NO:15 and SEQ ID NO:17; (ii) SEQ ID NO:34 and SEQ ID NO:22; or (iii) SEQ ID NO:32 and SEQ ID NO:33; and
   (e) a Shigatoxigenic *E*. *coli* (STEC)-specific amplification oligomer set comprising first and second oligomers respectively comprising target-hybridizing sequences substantially corresponding to the nucleotide sequences of: (i) SEQ ID NO:20 and SEQ ID NO:3; (ii) SEQ ID NO:49 and SEQ ID NO:3; or (iii) SEQ ID NO:4 and SEQ ID NO:7.
2. The set of oligonucleotides of paragraph 1, comprising the *Salmonella-*specific amplification oligomer set.
3. The set of oligonucleotides of any one of the preceding paragraphs, comprising the *C. jejuni*-specific amplification oligomer set.
4. The set of oligonucleotides of any one of the preceding paragraphs, comprising the *C*. *coli-*specific amplification oligomer set.
5. The set of oligonucleotides of any one of the preceding paragraphs, comprising the *Shigella-*specific amplification oligomer set.
6. The set of oligonucleotides of any one of the preceding paragraphs, comprising the STEC-specific amplification oligomer set.
7. The set of oligonucleotides of any one of the preceding paragraphs, comprising at least two of the *Salmonella-*specific amplification oligomer set, the *C. jejuni-*specific amplification oligomer set, the *C*. *coli-*specific amplification oligomer set, the *Shigella*-specific amplification oligomer set, and the STEC-specific amplification oligomer set.
8. The set of oligonucleotides of any one of the preceding paragraphs, comprising at least three of the *Salmonella-specific* amplification oligomer set, the *C. jejuni-*specific amplification oligomer set, the *C*. *coli-*specific amplification oligomer set, the *Shigella*-specific amplification oligomer set, and the STEC-specific amplification oligomer set.
9. The set of oligonucleotides of any one of the preceding paragraphs, comprising at least four of the *Salmonella-*specific amplification oligomer set, the *C. jejuni-*specific amplification oligomer set, the *C*. *coli-*specific amplification oligomer set, the *Shigella*-specific amplification oligomer set, and the STEC-specific amplification oligomer set.
10. The set of oligonucleotides of any one of the preceding paragraphs, comprising the *Salmonella-*specific amplification oligomer set, the *C. jejuni*-specific amplification oligomer set, the *C*. *coli-*specific amplification oligomer set, the *Shigella-*specific amplification oligomer set, and the STEC-specific amplification oligomer set.
11. The set of oligonucleotides of the immediately preceding paragraph, wherein
   the *Salmonella-*specific amplification oligomer set comprises first and second oligomers respectively comprising target-hybridizing sequences substantially corresponding to the nucleotide sequences of SEQ ID NO:12 and SEQ ID NO:14;
   the *C. jejuni*-specific amplification oligomer set comprises first and second oligomers respectively comprising target-hybridizing sequences substantially corresponding to the nucleotide sequences of SEQ ID NO:11 and SEQ ID NO:19;
   the *C*. *coli-*specific amplification oligomer set comprises first and second oligomers respectively comprising target-hybridizing sequences substantially corresponding to the nucleotide sequences of SEQ ID NO:8 and SEQ ID NO:10;
   the *Shigella*-specific amplification oligomer set comprises first and second oligomers respectively comprising target-hybridizing sequences substantially corresponding to the nucleotide sequences of SEQ ID NO:15 and SEQ ID NO:17; and
   the STEC-specific amplification oligomer set comprises first and second oligomers respectively comprising target-hybridizing sequences substantially corresponding to the nucleotide sequences of (i) SEQ ID NO:20 and SEQ ID NO:3; and (ii) SEQ ID NO:4 and SEQ ID NO:7.
12. The set of oligonucleotides of paragraph 11, further comprising:
   a *Salmonella* detection probe, comprising a target-hybridizing sequence substantially corresponding to the nucleotide sequence of:
   SEQ ID NO:13 if the *Salmonella-*specific amplification oligomer set comprises first and second oligomers respectively comprising target-hybridizing sequences substantially corresponding to the nucleotide sequences of SEQ ID NO:12 and SEQ ID NO:14;
   SEQ ID NO:13 if the *Salmonella-*specific amplification oligomer set comprises first and second oligomers respectively comprising target-hybridizing sequences substantially corresponding to the nucleotide sequences of SEQ ID NO:12 and SEQ ID NO:28;
   SEQ ID NO:45 if the *Salmonella-*specific amplification oligomer set comprises first and second oligomers respectively comprising target-hybridizing sequences substantially corresponding to the nucleotide sequences of SEQ ID NO:21 and SEQ ID NO:47;
   SEQ ID NO:44, SEQ ID NO:26, or SEQ ID NO:25 if the *Salmonella-*specific amplification oligomer set comprises first and second oligomers respectively comprising target-hybridizing sequences substantially corresponding to the nucleotide sequences of SEQ ID NO:38 and SEQ ID NO:36;
   SEQ ID NO:30 if the *Salmonella-*specific amplification oligomer set comprises first and second oligomers respectively comprising target-hybridizing sequences substantially corresponding to the nucleotide sequences of SEQ ID NO:35 and SEQ ID NO:40;
   SEQ ID NO:23 if the *Salmonella-*specific amplification oligomer set comprises first and second oligomers respectively comprising target-hybridizing sequences substantially corresponding to the nucleotide sequences of SEQ ID NO:42 and SEQ ID NO:31; or
   SEQ ID NO:43 if the *Salmonella-*specific amplification oligomer set comprises first and second oligomers respectively comprising target-hybridizing sequences substantially corresponding to the nucleotide sequences of SEQ ID NO:41 and SEQ ID NO:27.
13. The set of oligonucleotides of paragraph 11 or 12, further comprising:
   a *Shigella* detection probe, the *Shigella* detection probe comprising a target-hybridizing sequence substantially corresponding to the nucleotide sequence of
   SEQ ID NO:16 if the *Shigella*-specific amplification oligomer set comprises first and second oligomers respectively comprising target-hybridizing sequences substantially corresponding to the nucleotide sequences of SEQ ID NO:15 and SEQ ID NO:17;
   SEQ ID NO:46 or SEQ ID NO:29 if the *Shigella*-specific amplification oligomer set comprises first and second oligomers respectively comprising target-hybridizing sequences substantially corresponding to the nucleotide sequences of SEQ ID NO:34 and SEQ ID NO:22; or
   SEQ ID NO:24 if the *Shigella-*specific amplification oligomer set comprises first and second oligomers respectively comprising target-hybridizing sequences substantially corresponding to the nucleotide sequences of SEQ ID NO:32 and SEQ ID NO:33.
14. The set of oligonucleotides of any one of paragraphs 11-13, further comprising:
   a STEC detection probe, the STEC detection probe comprising a target-hybridizing sequence substantially corresponding to the nucleotide sequence of
   SEQ ID NO:1, SEQ ID NO:2, or SEQ ID NO:48 if the STEC-specific amplification oligomer set comprises first and second oligomers respectively comprising target-hybridizing sequences substantially corresponding to the nucleotide sequences of SEQ ID NO:20 and SEQ ID NO:3;
   SEQ ID NO:1, SEQ ID NO:2, or SEQ ID NO:48 if the STEC-specific amplification oligomer set comprises first and second oligomers respectively comprising target-hybridizing sequences substantially corresponding to the nucleotide sequences of SEQ ID NO:49 and SEQ ID NO:3; or
   SEQ ID NO:5 or SEQ ID NO:6 if the STEC-specific amplification oligomer set comprises first and second oligomers respectively comprising target-hybridizing sequences substantially corresponding to the nucleotide sequences of SEQ ID NO:4 and SEQ ID NO:7.
15. The set of oligonucleotides of any one of paragraphs 3-14, further comprising a *C. jejuni* detection probe, the *C. jejuni* detection probe comprising a target-hybridizing sequence substantially corresponding to the nucleotide sequence of SEQ ID NO:18.
16. The set of oligonucleotides of any one of paragraphs 4-15, further comprising a *C*. *coli* detection probe, the *C*. *coli* detection probe comprising a target-hybridizing sequence substantially corresponding to the nucleotide sequence of SEQ ID NO:9, SEQ ID NO:37, or SEQ ID NO:39.
17. The set of oligonucleotides of any one of paragraphs 12-16, wherein one or more, or each, of the detection probes comprises a fluorescent dye compound.
18. The set of oligonucleotides of the immediately preceding paragraph, wherein each of the detection probes further comprises a non-fluorescent quenching dye compound.
19. An oligonucleotide for determining the presence or absence of at least one enteric pathogen, wherein the oligonucleotide comprises the sequence of any one of:
   SEQ ID NO:15, wherein each of nucleotides 7 and 15 comprises 5-methylcytosine;
   SEQ ID NO:16;
   SEQ ID NO:19, wherein each of nucleotides 1, 2, 7, 14, 15, 19, and 20 comprises 5-methylcytosine;
   SEQ ID NO:21, wherein each of nucleotides 13, 17, and 24 comprises 5-methylcytosine;
   SEQ ID NO:23, wherein each of nucleotides 2, 5, 7, and 14 comprises 5-methylcytosine;
   SEQ ID NO:25, wherein each of nucleotides 7, 9, 13, and 15 comprises 5-methylcytosine;
   SEQ ID NO:26, wherein each of nucleotides 9, 18, and 23 comprises 5-methylcytosine;
   SEQ ID NO:27, wherein each of nucleotides 6, 10, and 18 comprises 5-methylcytosine;
   SEQ ID NO:28, wherein each of nucleotides 2, 4, 12, 14, 15, and 17 comprises 5-methylcytosine;
   SEQ ID NO:30, wherein each of nucleotides 2, 4, 18, 19, and 20 comprises 5-methylcytosine;
   SEQ ID NO:31, wherein each of nucleotides 6, 7, 8, 13, 23, 24, and 26 comprises 5-methylcytosine;
   SEQ ID NO:32, wherein each of nucleotides 5, 11, 12, and 15 comprises 5-methylcytosine;
   SEQ ID NO:36, wherein each of nucleotides 2, 3, 4, 16, 18, 19, and 22 comprises 5-methylcytosine;
   SEQ ID NO:38, wherein each of nucleotides 4, 6, 7, and 8 comprises 5-methylcytosine;
   SEQ ID NO:39, wherein each of nucleotides 2, 9, 12, 19, 22, 24, and 25 comprises 5-methylcytosine;
   SEQ ID NO:40, wherein each of nucleotides 4, 13, 18, and 22 comprises 5-methylcytosine;
   SEQ ID NO:42;
   SEQ ID NO:43, wherein each of nucleotides 2, 9, 12, 16, 17, 22, 23, and 26 comprises 5-methylcytosine;
   SEQ ID NO:44, wherein each of nucleotides 7, 9, 13, and 15 comprises 5-methylcytosine;
   SEQ ID NO:45, wherein each of nucleotides 3, 6, 8, and 17 comprises 5-methylcytosine;
   SEQ ID NO:47, wherein each of nucleotides 11, 16, and 17 comprises 5-methylcytosine;
   SEQ ID NO:1, wherein each of nucleotides 6, 7, 9, 14, 17, and 24 comprises 5-methylcytosine;
   SEQ ID NO:2, wherein each of nucleotides 5, 8, 11, 13, 18, and 21 comprises 5-methylcytosine;
   SEQ ID NO:6, wherein each of nucleotides 3, 7, 8, 15, 20, 24, and 27 comprises 5-methylcytosine;
   SEQ ID NO:7, wherein each of nucleotides 3, 4, 18, and 19 comprises 5-methylcytosine;
   SEQ ID NO:9, wherein each of nucleotides 2, 6, 13, 16, 23, 26, 28, and 29 comprises 5-methylcytosine;
   SEQ ID NO:12, wherein each of nucleotides 4, 11, 12, and 16 comprises 5-methylcytosine;
   SEQ ID NO:18, wherein each of nucleotides 7 and 12 or each of nucleotides 7, 12, and 25 comprises 5-methylcytosine;
   SEQ ID NO:22, wherein each of nucleotides 5 and 16 comprises 5-methylcytosine;
   SEQ ID NO:37, wherein each of nucleotides 17, 22, 23, and 27 comprises 5-methylcytosine; and
   SEQ ID NO:46, wherein each of nucleotides 5, 11, and 12 comprises 5-methylcytosine.
20. A kit comprising the set of oligonucleotides of any one of paragraphs 1-18 or the oligonucleotide of paragraph 19.
21. A reaction mixture comprising the set of oligonucleotides of any one of paragraphs 1-18 or the oligonucleotide of paragraph 19.
22. The reaction mixture of the immediately preceding paragraph, further comprising α-cyclodextrin or polysorbate 20.
23. The reaction mixture of paragraph 21 or 22, further comprising a detergent.
24. The reaction mixture of the immediately preceding paragraph, wherein the detergent comprises sodium dodecyl sulfate.
25. A multiplex method, comprising:
   (1) contacting a sample, said sample suspected of containing the at least one enteric pathogen, with the set of oligonucleotides of any one of paragraphs 1-56;
   (2) performing an *in vitro* nucleic acid amplification reaction, wherein any *Salmonella, C. jejuni*, *C. coli, Shigella,* and STEC target nucleic acid, if present in said sample, is used as a template for generating one or more amplification products corresponding to the *Salmonella, C. jejuni*, *C. coli, Shigella,* and STEC target regions; and
   (3) detecting the presence or absence of the one or more amplification products,
      thereby determining the presence or absence of at least one of *Salmonella*, *C. jejuni*, *C. coli*, *Shigella*, and STEC in said sample.
26. The multiplex method of the immediately preceding paragraph, wherein (3) comprises:
   contacting the sample with at least one of the *Salmonella* detection probe, the *C*. *jejuni* detection probe, the *C*. *coli* detection probe, the *Shigella* detection probe, and the STEC detection probe;
   performing electrophoresis on the sample; or
   determining the sequence of the one or more amplification product, if present.
27. A method for synthesizing an oligonucleotide, comprising the steps of:
   (a) obtaining a solid support comprising at least one nucleobase residue, wherein the at least one nucleobase residue is covalently bound at a 3' position to the solid support;
   (b) coupling a 5' position of the nucleobase residue furtherest from the solid support to a 3' position of another nucleobase residue;
   (c) repeating step (b) at least 13 additional times, thereby generating at least 15 contiguous nucleobase residues coupled to the solid support; and
   (d) cleaving the at least 15 contiguous nucleobase residues generated in step (c), thereby obtaining the oligonucleotide,
   wherein the oligonucleotide comprises the sequence of any one of:
   SEQ ID NO:15, wherein each of nucleotides 7 and 15 comprises 5-methylcytosine;
   SEQ ID NO:16;
   SEQ ID NO:19, wherein each of nucleotides 1, 2, 7, 14, 15, 19, and 20 comprises 5-methylcytosine;
   SEQ ID NO:21, wherein each of nucleotides 13, 17, and 24 comprises 5-methylcytosine;
   SEQ ID NO:23, wherein each of nucleotides 2, 5, 7, and 14 comprises 5-methylcytosine;
   SEQ ID NO:25, wherein each of nucleotides 7, 9, 13, and 15 comprises 5-methylcytosine;
   SEQ ID NO:26, wherein each of nucleotides 9, 18, and 23 comprises 5-methylcytosine;
   SEQ ID NO:27, wherein each of nucleotides 6, 10, and 18 comprises 5-methylcytosine;
   SEQ ID NO:28, wherein each of nucleotides 2, 4, 12, 14, 15, and 17 comprises 5-methylcytosine;
   SEQ ID NO:30, wherein each of nucleotides 2, 4, 18, 19, and 20 comprises 5-methylcytosine;
   SEQ ID NO:31, wherein each of nucleotides 6, 7, 8, 13, 23, 24, and 26 comprises 5-methylcytosine;
   SEQ ID NO:32, wherein each of nucleotides 5, 11, 12, and 15 comprises 5-methylcytosine;
   SEQ ID NO:36, wherein each of nucleotides 2, 3, 4, 16, 18, 19, and 22 comprises 5-methylcytosine;
   SEQ ID NO:38, wherein each of nucleotides 4, 6, 7, and 8 comprises 5-methylcytosine;
   SEQ ID NO:39, wherein each of nucleotides 2, 9, 12, 19, 22, 24, and 25 comprises 5-methylcytosine;
   SEQ ID NO:40, wherein each of nucleotides 4, 13, 18, and 22 comprises 5-methylcytosine;
   SEQ ID NO:42;
   SEQ ID NO:43, wherein each of nucleotides 2, 9, 12, 16, 17, 22, 23, and 26 comprises 5-methylcytosine;
   SEQ ID NO:44, wherein each of nucleotides 7, 9, 13, and 15 comprises 5-methylcytosine;
   SEQ ID NO:45, wherein each of nucleotides 3, 6, 8, and 17 comprises 5-methylcytosine;
   SEQ ID NO:47, wherein each of nucleotides 11, 16, and 17 comprises 5-methylcytosine;
   SEQ ID NO:1, wherein each of nucleotides 6, 7, 9, 14, 17, and 24 comprises 5-methylcytosine;
   SEQ ID NO:2, wherein each of nucleotides 5, 8, 11, 13, 18, and 21 comprises 5-methylcytosine;
   SEQ ID NO:6, wherein each of nucleotides 3, 7, 8, 15, 20, 24, and 27 comprises 5-methylcytosine;
   SEQ ID NO:7, wherein each of nucleotides 3, 4, 18, and 19 comprises 5-methylcytosine;
   SEQ ID NO:9, wherein each of nucleotides 2, 6, 13, 16, 23, 26, 28, and 29 comprises 5-methylcytosine;
   SEQ ID NO:12, wherein each of nucleotides 4, 11, 12, and 16 comprises 5-methylcytosine;
   SEQ ID NO:18, wherein each of nucleotides 7 and 12 or each of nucleotides 7, 12, and 25 comprises 5-methylcytosine;
   SEQ ID NO:22, wherein each of nucleotides 5 and 16 comprises 5-methylcytosine;
   SEQ ID NO:37, wherein each of nucleotides 17, 22, 23, and 27 comprises 5-methylcytosine; and
   SEQ ID NO:46, wherein each of nucleotides 5, 11, and 12 comprises 5-methylcytosine.
28. The method of paragraph 27, wherein the oligonucleotide has a length of from 18 to 32 contiguous nucleobase residues, or from 20 to 30 contiguous nucleobase residues.
29. A method for synthesizing a pair of oligonucleotides, comprising synthesizing a first oligonucleotide and synthesizing a second oligonucleotide,
   wherein each of the synthesizing the first oligonucleotide and the synthesizing the second oligonucleotide comprises the steps of:
      (a) obtaining a solid support comprising at least one nucleobase residue, wherein the at least one nucleobase residue is covalently bound at a 3' position to the solid support;
      (b) coupling a 5' position of the nucleobase residue furtherest from the solid support to a 3' position of another nucleobase residue;
      (c) repeating step (b) at least 13 additional times, thereby generating at least 15 contiguous nucleobase residues coupled to the solid support; and
      (d) cleaving the at least 15 contiguous nucleobase residues generated in step (c), thereby obtaining the oligonucleotide, and
   wherein the first oligonucleotide and the the second oligonucleotide respectively comprise the sequence of any one of:
      SEQ ID NO:12 and SEQ ID NO:14;
      SEQ ID NO:21 and SEQ ID NO:47;
      SEQ ID NO:38 and SEQ ID NO:36;
      SEQ ID NO:35 and SEQ ID NO:40;
      SEQ ID NO:12 and SEQ ID NO:28;
      SEQ ID NO:42 and SEQ ID NO:31;
      SEQ ID NO:41 and SEQ ID NO:27;
      SEQ ID NO:11 and SEQ ID NO:19;
      SEQ ID NO:8 and SEQ ID NO:10;
      SEQ ID NO:15 and SEQ ID NO:17;
      SEQ ID NO:34 and SEQ ID NO:22;
      SEQ ID NO:32 and SEQ ID NO:33;
      SEQ ID NO:49 and SEQ ID NO:3;
      SEQ ID NO:20 and SEQ ID NO:3; and
      SEQ ID NO:4 and SEQ ID NO:7.
30. The method of paragraph 29, wherein each of the first oligonucleotide and the second oligonucleotide have a length of from 18 to 32 contiguous nucleobase residues, or from 20 to 30 contiguous nucleobase residues.

## Claims

1. A set of oligonucleotides for determining the presence or absence of at least two enteric pathogens, said set of oligonucleotides comprising (a) and at least one of (b)-(e):
(a) a *C*. *coli*-specific amplification oligomer set comprising first and second oligomers respectively comprising target-hybridizing sequences consisting of the nucleotide sequences of: (i) SEQ ID NO:8 and SEQ ID NO: 10;
(b) a *Salmonella-*specific amplification oligomer set comprising first and second oligomers respectively comprising target-hybridizing sequences consisting of the nucleotide sequences of: (i) SEQ ID NO: 12 and SEQ ID NO: 14; (ii) SEQ ID NO:21 and SEQ ID NO:47; (iii) SEQ ID NO:38 and SEQ ID NO:36; (iv) SEQ ID NO:35 and SEQ ID NO:40; (v) SEQ ID NO: 12 and SEQ ID NO:28; (vi) SEQ ID NO:42 and SEQ ID NO:31; or (vii) SEQ ID NO:41 and SEQ ID NO:27;
(c) a *C*. *jejuni-*specific amplification oligomer set comprising first and second oligomers respectively comprising target-hybridizing sequences consisting of the nucleotide sequences of: SEQ ID NO: 11 and SEQ ID NO: 19;
(d) a *Shigella-*specific amplification oligomer set comprising first and second oligomers respectively comprising target-hybridizing sequences consisting of the nucleotide sequences of: (i) SEQ ID NO: 15 and SEQ ID NO: 17; (ii) SEQ ID NO:34 and SEQ ID NO:22; or (iii) SEQ ID NO:32 and SEQ ID NO:33; and
(e) a Shigatoxigenic *E. coli* (STEC)-specific amplification oligomer set comprising first and second oligomers respectively comprising target-hybridizing sequences consisting of the nucleotide sequences of: (i) SEQ ID NO:20 and SEQ ID NO:3; (ii) SEQ ID NO:49 and SEQ ID NO:3; or (iii) SEQ ID NO:4 and SEQ ID NO:7.

2. The set of oligonucleotides of claim 1, comprising the *C*. *coli*-specific amplification oligomer set and at least two of the *Salmonella-*specific amplification oligomer set, the *C*. *jejuni-*specific amplification oligomer set, the *Shigella-*specific amplification oligomer set, and the STEC-specific amplification oligomer set.

3. The set of oligonucleotides of claim 1, comprising the *C*. *coli*-specific amplification oligomer set and at least three of the *Salmonella-*specific amplification oligomer set, the *C*. *jejuni-*specific amplification oligomer set, the *Shigella-*specific amplification oligomer set, and the STEC-specific amplification oligomer set.

4. The set of oligonucleotides of claim 1, comprising the *C*. *coli*-specific amplification oligomer set, the *Salmonella-*specific amplification oligomer set, the *C*. *jejuni-*specific amplification oligomer set, the *Shigella-*specific amplification oligomer set, and the STEC-specific amplification oligomer set,
wherein the *Salmonella-*specific amplification oligomer set comprises the first and second oligomers of any one of (b)(i) and (b)(iii)-(b)(vii).

5. The set of oligonucleotides of claim 4, wherein
the *Salmonella-*specific amplification oligomer set comprises first and second oligomers respectively comprising target-hybridizing sequences consisting of the nucleotide sequences of SEQ ID NO: 12 and SEQ ID NO: 14;
the *Shigella-*specific amplification oligomer set comprises first and second oligomers respectively comprising target-hybridizing sequences consisting of the nucleotide sequences of SEQ ID NO: 15 and SEQ ID NO: 17; and/or
the STEC-specific amplification oligomer set comprises first and second oligomers respectively comprising target-hybridizing sequences consisting of the nucleotide sequences of (i) SEQ ID NO:20 and SEQ ID NO:3; and (ii) SEQ ID NO:4 and SEQ ID NO:7.

6. The set of oligonucleotides of claim 4, further comprising:
(a) a *C*. *coli* detection probe, comprising a target-hybridizing sequence consisting of the nucleotide sequence of SEQ ID NO:9, SEQ ID NO:37, or SEQ ID NO:39;
(b) a *Salmonella* detection probe, comprising a target-hybridizing sequence consisting of the nucleotide sequence of:
SEQ ID NO: 13 if the *Salmonella-*specific amplification oligomer set comprises first and second oligomers respectively comprising target-hybridizing sequences consisting of the nucleotide sequences of SEQ ID NO: 12 and SEQ ID NO: 14 or the nucleotide sequences of SEQ ID NO: 12 and SEQ ID NO:28;
SEQ ID NO:44, SEQ ID NO:26, or SEQ ID NO:25 if the *Salmonella-*specific amplification oligomer set comprises first and second oligomers respectively comprising target-hybridizing sequences consisting of the nucleotide sequences of SEQ ID NO:38 and SEQ ID NO:36;
SEQ ID NO:30 if the *Salmonella-*specific amplification oligomer set comprises first and second oligomers respectively comprising target-hybridizing sequences consisting of the nucleotide sequences of SEQ ID NO:35 and SEQ ID NO:40;
SEQ ID NO:23 if the *Salmonella-*specific amplification oligomer set comprises first and second oligomers respectively comprising target-hybridizing sequences consisting of the nucleotide sequences of SEQ ID NO:42 and SEQ ID NO:31; or
SEQ ID NO:43 if the *Salmonella-*specific amplification oligomer set comprises first and second oligomers respectively comprising target-hybridizing sequences consisting of the nucleotide sequences of SEQ ID NO:41 and SEQ ID NO:27;
(c) a *C*. *jejuni* detection probe, comprising a target-hybridizing sequence consisting of the nucleotide sequence of SEQ ID NO: 18;
(d) a *Shigella* detection probe, comprising a target-hybridizing sequence consisting of the nucleotide sequence of
SEQ ID NO: 16 if the *Shigella-*specific amplification oligomer set comprises first and second oligomers respectively comprising target-hybridizing sequences consisting of the nucleotide sequences of SEQ ID NO: 15 and SEQ ID NO: 17;
SEQ ID NO:46 or SEQ ID NO:29 if the *Shigella-*specific amplification oligomer set comprises first and second oligomers respectively comprising target-hybridizing sequences consisting of the nucleotide sequences of SEQ ID NO:34 and SEQ ID NO:22; or
SEQ ID NO:24 if the *Shigella-*specific amplification oligomer set comprises first and second oligomers respectively comprising target-hybridizing sequences consisting of the nucleotide sequences of SEQ ID NO:32 and SEQ ID NO:33; and/or
(e) a STEC detection probe, comprising a target-hybridizing sequence consisting of the nucleotide sequence of
SEQ ID NO:1, SEQ ID NO:2, or SEQ ID NO:48 if the STEC-specific amplification oligomer set comprises first and second oligomers respectively comprising target-hybridizing sequences consisting of the nucleotide sequences of SEQ ID NO:20 and SEQ ID NO:3 or the nucleotide sequence of SEQ ID NO:49 and SEQ ID NO:3; or
SEQ ID NO:5 or SEQ ID NO:6 if the STEC-specific amplification oligomer set comprises first and second oligomers respectively comprising target-hybridizing sequences consisting of the nucleotide sequences of SEQ ID NO:4 and SEQ ID NO:7.

7. The set of oligonucleotides of claim 6, wherein one or more, or each, of the detection probes comprises a fluorescent dye compound.

8. The set of oligonucleotides of claim 7, wherein each of the detection probes comprising the fluorescent dye compound further comprises a non-fluorescent quenching dye compound.

9. An oligonucleotide for determining the presence or absence of *C*. *coli,* wherein the oligonucleotide comprises the sequence of any one of:
SEQ ID NO:9, wherein each of nucleotides 2, 6, 13, 16, 23, 26, 28, and 29 comprises 5-methylcytosine;
SEQ ID NO:37, wherein each of nucleotides 17, 22, 23, and 27 comprises 5-methylcytosine; and
SEQ ID NO:39, wherein each of nucleotides 2, 9, 12, 19, 22, 24, and 25 comprises 5-methylcytosine.

10. A kit comprising the set of oligonucleotides of any one of claims 1 to 8 or the oligonucleotide of claim 9.

11. A reaction mixture comprising the set of oligonucleotides of any one of claims 1 to 8 or the oligonucleotide of claim 9.

12. A multiplex method for determining the presence or absence of *C*. *coli* and at least one of *Salmonella, C. jejuni, Shigella,* and Shigatoxigenic *E. coli* (STEC) in a sample, the method comprising:
(1) contacting a sample, said sample suspected of containing *C*. *coli* and at least one of *Salmonella, C. jejuni, Shigella,* and STEC, with the set of oligonucleotides of any one of claims 1 to 5;
(2) performing an *in vitro* nucleic acid amplification reaction, wherein any *C*. *coli, Salmonella, C. jejuni, Shigella,* and STEC target nucleic acid, if present in said sample, is used as a template for generating one or more amplification products corresponding to the *C*. *coli, Salmonella, C. jejuni, Shigella,* and STEC target regions; and
(3) detecting the presence or absence of the one or more amplification products,
thereby determining the presence or absence of *C*. *coli* and at least one of *Salmonella, C. jejuni*, *Shigella*, and STEC in said sample.

13. The multiplex method of claim 12, wherein (3) comprises:
contacting the sample with at least one of a *C*. *coli* detection probe, a *Salmonella* detection probe, a *C*. *jejuni* detection probe, a *Shigella* detection probe, and a STEC detection probe, wherein each of said detection probes is as defined in any one of claims 6 to 8;
performing electrophoresis on the sample; or
determining the sequence of the one or more amplification products, if present.

14. A method for synthesizing an oligonucleotide, comprising the steps of:
(a) obtaining a solid support comprising at least one nucleobase residue, wherein the at least one nucleobase residue is covalently bound at a 3' position to the solid support;
(b) coupling a 5' position of the nucleobase residue furthest from the solid support to a 3' position of another nucleobase residue;
(c) repeating step (b) at least 13 additional times, thereby generating at least 15 contiguous nucleobase residues coupled to the solid support; and
(d) cleaving the at least 15 contiguous nucleobase residues generated in step (c), thereby obtaining the oligonucleotide,
wherein the oligonucleotide comprises the sequence of any one of:
SEQ ID NO:9, wherein each of nucleotides 2, 6, 13, 16, 23, 26, 28, and 29 comprises 5-methylcytosine;
SEQ ID NO:37, wherein each of nucleotides 17, 22, 23, and 27 comprises 5-methylcytosine; and
SEQ ID NO:39, wherein each of nucleotides 2, 9, 12, 19, 22, 24, and 25 comprises 5-methylcytosine.

15. A method for synthesizing a pair of oligonucleotides, comprising synthesizing a first oligonucleotide and synthesizing a second oligonucleotide,
wherein each of the synthesizing the first oligonucleotide and the synthesizing the second oligonucleotide comprises the steps of:
(a) obtaining a solid support comprising at least one nucleobase residue, wherein the at least one nucleobase residue is covalently bound at a 3' position to the solid support;
(b) coupling a 5' position of the nucleobase residue furthest from the solid support to a 3' position of another nucleobase residue;
(c) repeating step (b) at least 13 additional times, thereby generating at least 15 contiguous nucleobase residues coupled to the solid support; and
(d) cleaving the at least 15 contiguous nucleobase residues generated in step (c), thereby obtaining the oligonucleotide, and
wherein the first oligonucleotide and the second oligonucleotide respectively comprise the sequence of SEQ ID NO:8 and SEQ ID NO:10.
